# EUROPEAN PATENT SPECIFICATION

(11) **EP 2 506 864 B1**
(45) Date of publication and mention of the grant of the patent: **27.07.2016**
(21) Application number: 10833678.5
(22) Date of filing: 30.11.2010
(51) Int. Cl.: A61K 38/02, A61P 35/00, A61K 39/395, A61P 37/04

(54) **TREATMENT AND PREVENTION OF CANCERS ASSOCIATED WITH INTRACELLULAR ONCOPROTEINS BY ANTIBODY THERAPY OR VACCINATION**
BEHANDLUNG UND PROPHYLAXE VON MIT INTRAZELLULÄREN ONKOPROTEINEN ASSOZIIERTEN KREBSERKRANKUNGEN DURCH ANTIKÖRPERTHERAPIE ODER IMPFUNG
TRAITEMENT ET PRÉVENTION DE CANCERS ASSOCIÉS À DES ONCOPROTÉINES INTRACELLULAIRES PAR UNE THÉRAPIE AUX ANTICORPS OU UNE VACCINATION

(30) Priority: 30.11.2009 US 264892 P; 17.05.2010 US 345219 P
(43) Date of publication of application: 10.10.2012
(73) Proprietor: Agency for Science, Technology and Research, Singapore 138632 (SG)
(72) Inventor: ZENG, Qi, Singapore 138673 (SG)
(74) Representative: Clegg, Richard Ian
(86) International application number: PCT/SG2010/000449
(87) International publication number: WO 2011/065923

(56) References cited:
- WO-A1-2008/136774
- WO-A1-2008/136774
- WO-A2-2009/022988
- BRIGHT R K ET AL: "Immunization of BALB/c mice with recombinant simian virus 40 large tumor antigen induces antibody-dependent cell-mediated cytotoxicity against simian virus 40-transformed cells. An antibody-based mechanism for tumour immunity", THE JOURNAL OF IMMUNOLOGY, THE AMERICAN ASSOCIATION OF IMMUNOLOGISTS, US, vol. 153, no. 5, 1 September 1994 (1994-09-01), pages 2064-2071, XP008157143, ISSN: 0022-1767
- SHEN X G ET AL: "Prokaryotic Expression and Polyclonal Antibody Preparation of PRL3", CHEMICAL RESEARCH IN CHINESE UNIVERSITIES, BEIJING, CN, vol. 23, no. 6, 1 November 2007 (2007-11-01), pages 697-700, XP022856189, ISSN: 1005-9040, DOI: 10.1016/S1005-9040(07)60151-2 [retrieved on 2007-11-01]
- GUO, K. ET AL.: 'Monoclonal antibodies target intracellular PRL phosphatises to inhibit cancer metastases in mice' CANCER BIOLOGY AND THERAPY vol. 7, no. 3, May 2008, pages 750 - 757, XP008157134
- ARAFAT, W. ET AL.: 'Antineoplastic effect of anti-erbB-2 intrabody is not correlated with scFv affinity for its target' CANCER GENE THERAPY vol. 7, no. 9, June 2000, pages 1250 - 1256, XP008157139
- LOWE, D.B. ET AL.: 'Tumor Immunity against a Simian Virus 40 Oncoprotein Requires CD8+ T Lymphocytes in the Effector Immune Phase' JOURNAL OF VIROLOGY vol. 84, no. 2, 04 November 2009, pages 883 - 893, XP008157132
- BRIGHT, R.K. ET AL.: 'Immunization of BALB/c mice with recombinant simian virus 40 large tumor antigen induces antibody-dependent cell-mediated cytotoxicity against simian virus 40-transformed cells. An antibody-based mechanism for tumour immunity' JOURNAL OF IMMUNOLOGY vol. 153, no. 5, 01 September 1994, pages 2064 - 2071, XP008157143
- DING, Y. ET AL.: 'Coadministration of the fungal immunomodulatory protein FIP-Fve and a tumour-associated antigen enhanced antitumour immunity.' IMMUNOLOGY vol. 128, September 2009, pages E881 - E894., XP008157135
- KENTER, G.G. ET AL.: 'Vaccination against HPV-16 Oncoproteins for Vulvar Intraepithelial Neoplasia' THE NEW ENGLAND JOURNAL OF MEDICINE vol. 361, no. 19, 05 November 2009, pages 1838 - 1847, XP008157144

## Description

### FIELD

The present invention relates to the fields of cell biology, molecular biology and biochemistry. This invention also relates to the field of medicine. In particular, it relates to prevention and treatment of diseases, in particular cancer, as well as compositions for such use.

### BACKGROUND

Cancer has been the subject of intense investigation in the past two decades. However, the underlying causes responsible for cancer metastasis are poorly understood and most types of cancer preventions are still limited. Effective ways to prevent cancer metastasis are urgently needed.

Antibody based therapy has proven to be effective for cancer treatment; however, this approach has been traditionally limited to extracellular or secreted proteins expressed by cancer cells^{1,2}.

Thus, a number of potential cancer or tumour markers and cancer antigens have been identified in the literature and antibody therapies have been developed against some of them.

For example, the well-known cancer therapy Herceptin (Trastuzumab) is a monoclonal antibody that can kill HER2-positive cancer cells. Herceptin binds to the HER2 (human epidermal growth factor receptor 2) antigen on the cancer cell. Likewise, Bevacizumab (Avastin™) is a monoclonal antibody targeted against vascular endothelial growth factor (VEGF), one of the growth factors implicated in the formation of new blood vessels. By inhibiting angiogenesis, Bevacizumab prevents tumour cells from receiving a constant supply of blood to receive the oxygen and nutrients the tumour needs to survive.

However, the applicability of antibody therapeutics for different cancers is not universal. One of the limitations that has prevented the general use of antibody therapeutics is the large size of antibody molecules and their consequent inability to cross the plasma or cell membrane. In the absence of modification, antibodies (including monoclonal antibodies) are only generally suitable for targeting cancer antigens located at the surface or exterior of host cells¹⁴⁻¹⁵. In the examples above, HER2 receptor is located on the cell surface and is hence accessible for antibody binding by Herceptin. Likewise, VEGF is secreted into the bloodstream and is able to be bound by Bevacizumab.

Most oncogenic proteins are intracellular proteins (such as intracellular phosphatases, intracellular kinases, transcription factors, etc), and have remained under-explored by the approach of antibody therapies. The long held view that antibodies are too large to penetrate cell membrane has hampered the technology of antibody therapy used in targeting intracellular proteins.

Shen et al 2007 (Chem. Res. Chinese U. 23(6) 697-700) presented the results of cloning, prokaryotic expression, purification, and polyclonal antibody preparation of PRL3. To obtain a specific polyclonal antibody against PRL3 they prepared GST-PRL3 to immunize rabbits and purify an anti-PRL3 polyclonal antibody by negative selection affinity columns.

There is therefore an urgent need for effective ways of treating and preventing cancer metastasis. Antibodies have not hitherto been used for targeting intracellular antigens or cancer markers because of the inability of the antibodies to cross the cell membrane and the consequent inaccessibility of the antigen.

### SUMMARY

We demonstrate in the Examples that intracellular oncoproteins can be targeted with either antibody therapy or vaccination to prevent cancer metastasis in mice.

Firstly, wild-type C57BL6 mice intravenously (i.v.) injected either with B16F0 or B16F10 melanoma cells, followed by i.v. injection with PRL-3 monoclonal antibody (mAb) showed that PRL-3 mAb can reduce tumors formed by B16F0 melanoma cells that express endogenous PRL-3 intracellular phosphatase,but not those by B16F10 melanoma cells that do not express PRL-3.

Secondly, C57BL6 mice i.v. injected with either EGFP-B16F0 or EGFP-B16F10 cells in which both cell lines were engineered to overexpress EGFP, followed by i.v. injection with EGFP monoclonal antibody showed that regardless of PRL-3 expression status, EGFP mAb could eradicate tumors formed by both EGFP-B16F0 and EGFP-B 16F 10 melanoma cell lines.

Thirdly, polyomavirus middle T (Py-mT)-transgenic young females i.v. injected with mT antibody showed that the mT antibody could effectively reduce the formation of mT-expressing breast tumors.

We further show that immunisation with VHZ protein can protect against cancer development.

We therefore provide for methods of treating cancers and metastases thereof with antibodies against intracellular oncoproteins.

According to this disclosure, we describe use of an antibody therapy for targeting intracellular oncoproteins or proteins to prevent cancer metastasis. The antibody therapy may be an intracellular antibody therapy.

There is described use of an vaccine
therapy comprising an oncoprotein protein for vaccination to stimulate a host to produce its own antibodies to prevent tumor formation.

The vaccine therapy may be an intracellular vaccine therapy.

We describe a method of treatment of
cancer, such as metastatic cancer, in an individual suffering or suspected to be suffering from cancer, the method comprising administering a therapeutically effective amount of an antibody capable of binding to an intracellular oncoprotein to the individual.

The antibody may be capable of binding to an intracellular oncoprotein, preferably by being capable of crossing the plasma membrane of a cell. The antibody may comprise a monoclonal antibody or a humanised antibody. The antibody may be capable of binding to and inhibiting a biological activity of the intracellular oncoprotein, such as protein tyrosine phosphatase (PTP) activity.

Logically, by using antigen to stimulate host immune system for own antibody production, one would expect to have similar antibody therapy impact in controlling tumor progression.

C57BL6 mice vaccinated with PRL-3 or EGFP antigens showed significantly reduction in the formation of metastatic tumors expressing either PRL-3 or EGFP protein respectively, as compared with un-immunized mice. Furthermore, Py-mT mice vaccinated with mT antigen significantly prevented tumor formation in mammary glands. The results obtained from 194 wild type mice demonstrate that cancer metastasis caused by intracellular oncoproteins can be successfully blocked with antibody therapy and vaccination.

We provide methods of preventing metastatic cancer by vaccination using PRL3, as set out in the claims.

As disclosed herein there is a method of prevention of
cancer, such as metastatic cancer, in an individual suffering or suspected to be suffering from cancer, the method comprising administering a prophylactically effective amount of an intracellular oncoprotein, or a variant, homologue, derivative or fragment thereof to the individual.

The intracellular oncoprotein may be such that it does not comprise an extracellular domain or a transmembrane domain. The intracellular oncoprotein may comprise a cytoplasmic or nuclear oncoprotein. The intracellular oncoprotein may comprise PRL-3 protein or polyomavirus middle T (Py-mT) protein.

The cancer may be associated with or caused by over-expression of the intracellular oncoprotein. The cancer may be selected from the group consisting of: ovarian cancer, breast cancer, liver cancer, pancreatic cancer, prostate cancer, gastric cancer, lung cancer, penis cancer, cervical cancer, brain cancer, esophageal cancer, bladder carcinoma, kidney renal cell carcinoma, ovary lymphoma and skin melanoma.

The cancer may comprise a metastatic cancer. The method may be such that the number of metastatic tumours in a treated individual is reduced by at least 50%, at least 60%, at least 70%, at least 80% or at least 90%, compared to an untreated individual.

We describe an antibody capable of
binding to an intracellular oncoprotein for use in a method of treatment of cancer, such as metastatic cancer, the method comprising administering a therapeutically effective amount of the antibody capable of binding to an intracellular oncoprotein to an individual suffering or suspected to be suffering from cancer.

Also described is an intracellular oncoprotein, or a
variant, homologue, derivative or fragment thereof, for use in a method of prevention of cancer, such as metastatic cancer, the method comprising administering a prophylactically effective amount of an intracellular oncoprotein, variant, homologue, derivative or fragment thereof, to an individual suffering or suspected to be suffering from cancer.

We provide, according to the present invention, a pharmaceutical composition comprising PRL3 together with a pharmaceutically acceptable excipient, diluent or carrier.

The practice of the present invention will employ, unless otherwise indicated, conventional techniques of chemistry, molecular biology, microbiology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; Using Antibodies: A Laboratory Manual: Portable Protocol NO. I by Edward Harlow, David Lane, Ed Harlow (1999, Cold Spring Harbor Laboratory Press, ISBN 0-87969-544-7); Antibodies: A Laboratory Manual by Ed Harlow (Editor), David Lane (Editor) (1988, Cold Spring Harbor Laboratory Press, ISBN 0-87969-314-2), 1855. Handbook of Drug Screening, edited by Ramakrishna Seethala, Prabhavathi B. Fernandes (2001, New York, NY, Marcel Dekker, ISBN 0-8247-0562-9); and Lab Ref: A Handbook of Recipes, Reagents, and Other Reference Tools for Use at the Bench, Edited Jane Roskams and Linda Rodgers, 2002, Cold Spring Harbor Laboratory, ISBN 0-87969-630-3.

### BRIEF DESCRIPTION OF THE DRAWINGS

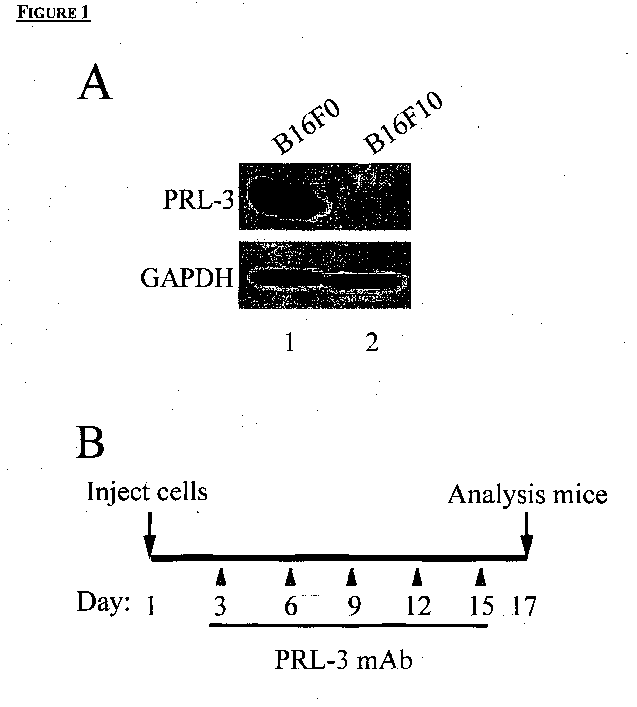

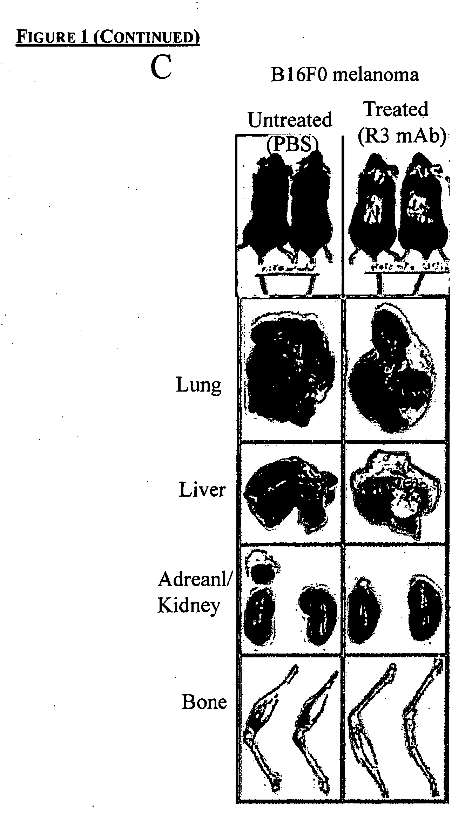

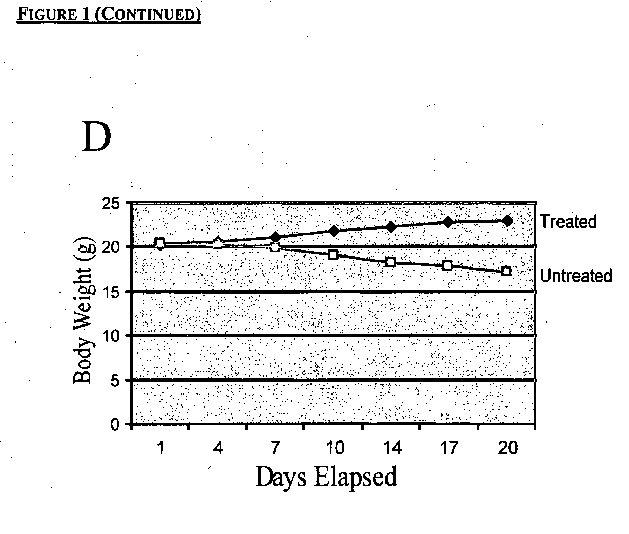

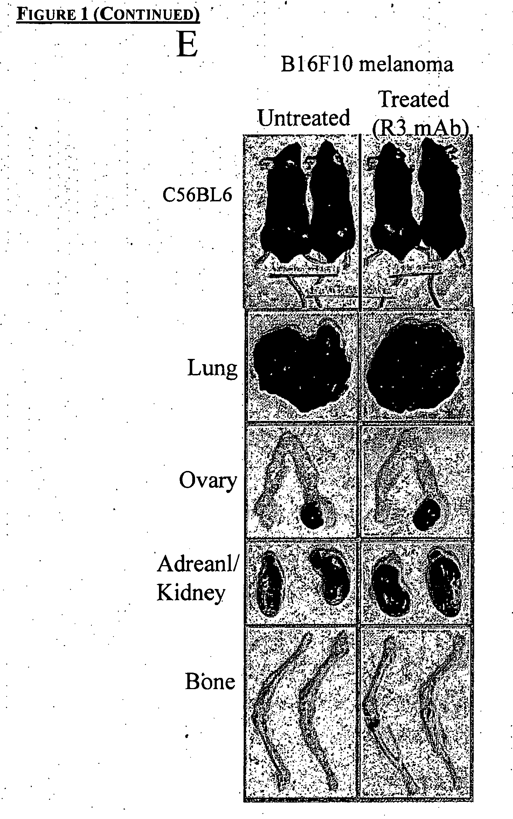

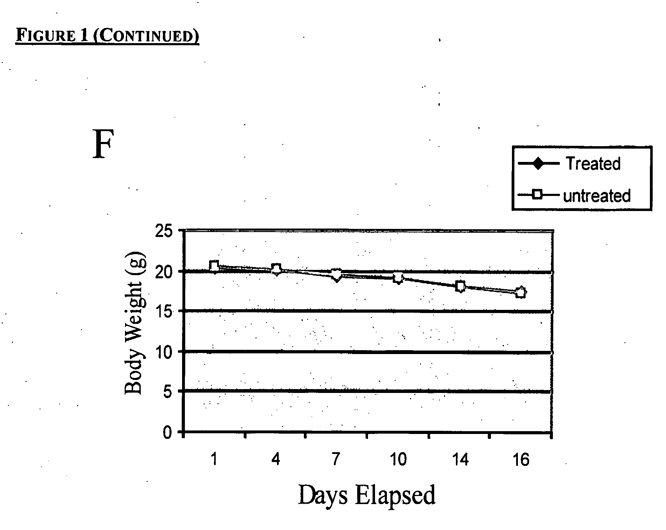

Figure 1 is a diagram showing that PRL-3 mAb effectively inhibits the formation of metastatic tumors formed by PRL-3 expressing cancer cells. A, Total cell lysates were prepared from B16F0 and B16F10 melanoma cancer cells and endogenous PRL-3 protein expression was determined using immunoblot. GAPDH was used as a loading control. B, C57BL6 mice were injected with 1x10⁶ B16F0 cells (on day 1) followed by PRL-3 mouse antibody (mAb, clone #318) treatment with therapeutic plan. C. On day 17-20, tumors were frequently found in the lung, liver, adrenal gland, kidney, and bone in untreated mice (n=5), while the PRL-3 mAb eliminates the formation of tumors in most tissues of treated mice (n=5). D, The body weight of PRL-3 mAb 'treated' B16F0 recipient mice constantly increased throughout the duration of the experiment. E, Parallel experiments were performed with non-PRL-3 expressing B16F10 melanoma cancer cell line and no therapeutic outcome was obtained with PRL-3 mAb therapy. F, The body weights of both treated and untreated B16F10 receipt mice constantly decreased over the duration of the experiment.

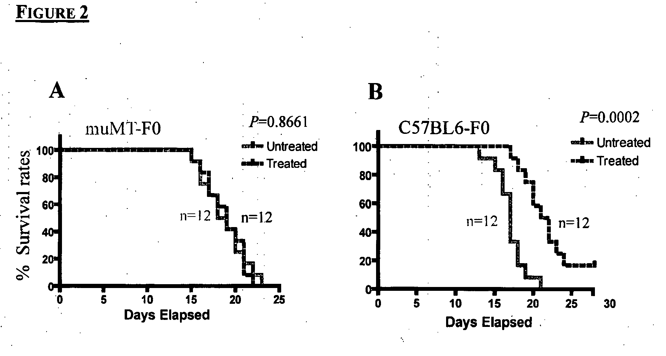

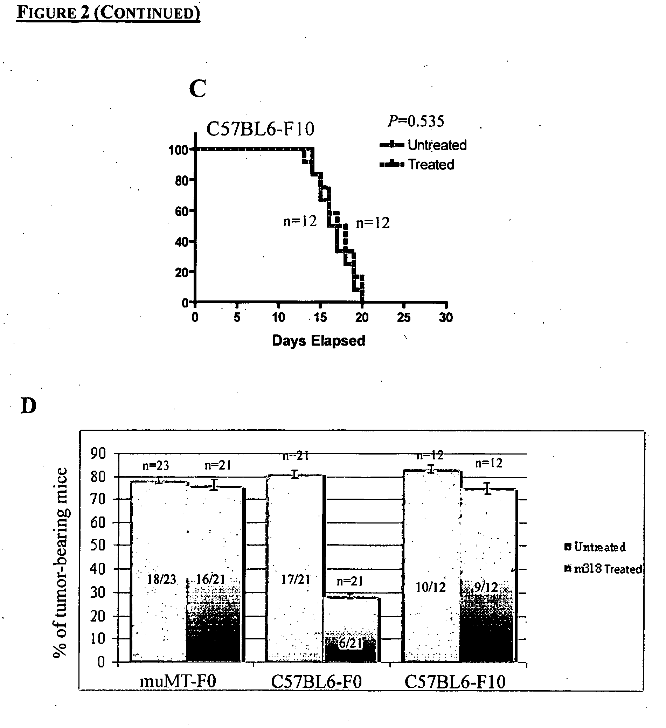

Figure 2 is a diagram showing that B-cells are important in mediating the antibody therapeutic event. A. Kaplan-Meier survival curves were used to compare the 'treated' and 'untreated' groups of B-cell deficient muMT mice injected with B16F0 cells. No difference in median survival rate for untreated (18.5 days) and treated (19 days) groups was evident. B. Kaplan-Meier analysis of wild-type C57BL6 mice injected with B16F0 had a statistically significant difference (*P*=0.0002) in median survival between untreated (17 days) and treated (21.5 days) groups. C. Kaplan-Meier analysis of wild-type C57BL6 mice C57BL6 mice injected with B16F10 had no statistically significant difference between untreated (16.5 days) and treated (17.5 days). D. The results between treated and untreated mice were summarized. The numbers of ineffective (or tumor-bearing) mice were placed at the numerator and the total numbers of mice were placed at the denominator. Fraction indicated at the Y-axis represents percentage of tumor-bearing mice verse total mice (n) in each group. Column represents type of mice indicated at the X-axis.

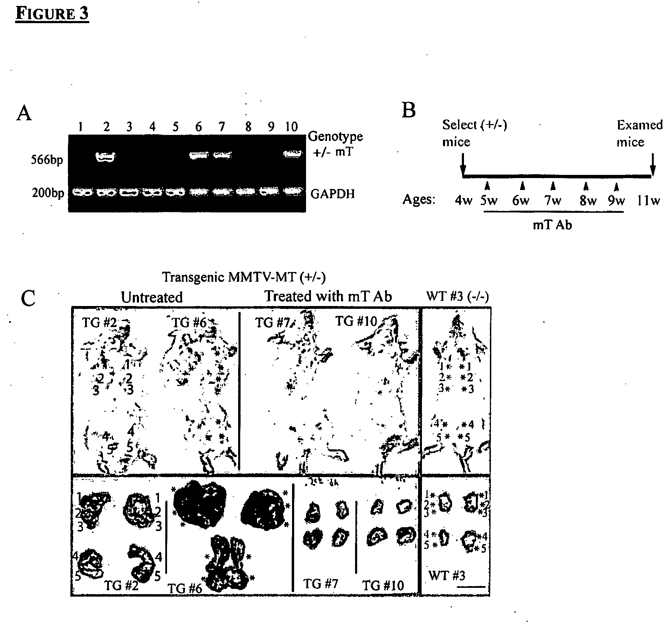

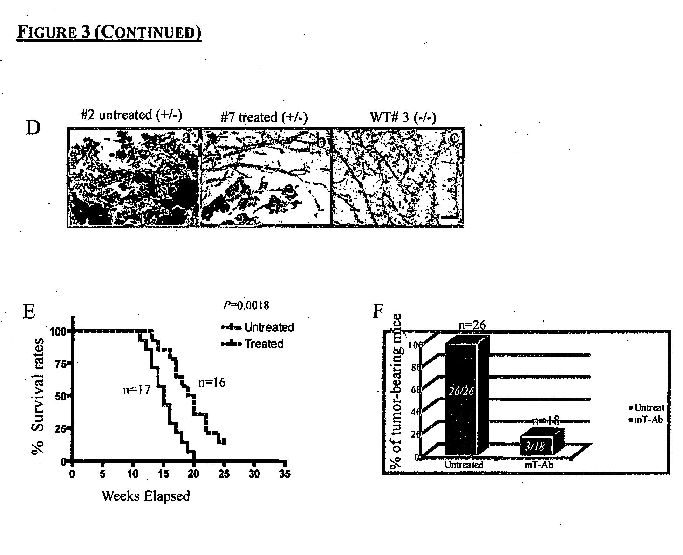

Figure 3 is a diagram showing that mT Ab effectively inhibits the formation of breast tumors expressing mT oncoprotein. A, Genotyping was used to identify transgenic MMTV-PymT females. Mice #2, #6, #7, and #10 are heterozygote transgenic females (+/-). B, #2 and #6 transgenic mice remained untreated as controls to monitor the progression of mammary gland tumor progression. #7 and #10 transgenic mice received mT antibody i.v. injection. At the end of the experiment (3-month), all mice were sacrificed. The sizes and weights of the breast tissues (tumors) were measured. The 5 pairs of mammary glands were indicated and showed as 4 groups in respective lower panel. Bar: 1.5 cm. C, The morphology of the whole mount breast tissues was examined in breasts from untreated (a), treated (b), and wild type (c). E. Kaplan-Meier survival curves were used to compare 'treated' and 'untreated' group of MMTV-PymT heterozygous transgenic females. F. Fraction indicated at the Y-axis represents percentage of tumor-bearing mice verse total mice (n) in each group. Column represents type of mice indicated at the X-axis.

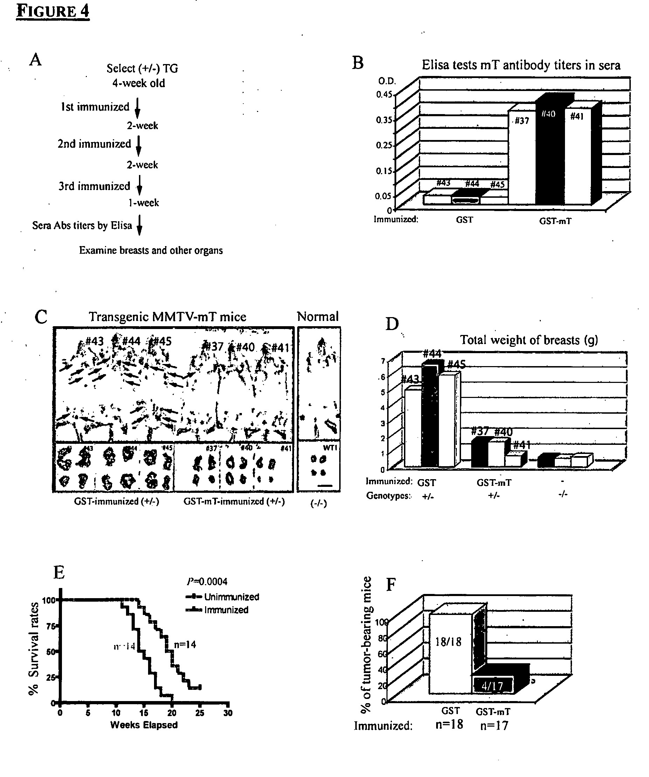

Figure 4 is a diagram showing that immunization of MMTV-PyMT mice with mT antigen could prevent the formation of breast tumors that express mT intracellular protein. A, a therapeutic plan of vaccination with mT antigen. B-D. MMTV-PymT heterozygous transgenic mice (TG) were used for the experiment. TG females (#43, #44, #45) were immunized with GST protein. By ELISA analysis, these mice had no mT antibody in their sera and showed dramatic progression of mammary gland tumors. In contrast, TG females (#37, #40, #41) were immunized with GST-mT fusion protein; these mice had high levels of mT antibodies in their sera and showed significantly repression of mammary gland tumors. The 10 mammary glands were shown as 4 groups at the bottom of each panel. Top two groups of breasts consist of the top three pairs of mammary glands, while bottom two groups represent the bottom two pairs of mammary glands. Weights of total 10 mammary glands were measured. Wild type mice (n=3) were used as controls. Bar: 1.5cm. E, Kaplan-Meier survival curves were compared between 'immunized' and 'unimmunized' MMTV-PymT mice, with median survival time (p=0.0004) clearly longer in mT-immunized group (19.5-week) versus unimmunized group (14.5-weeks). F. Summary of results between immunized and unimmunized mice. N stands for number of mice in each group; fraction in each column represents proportion of tumor-bearing MMTV-PymT mice verse total TG females used.

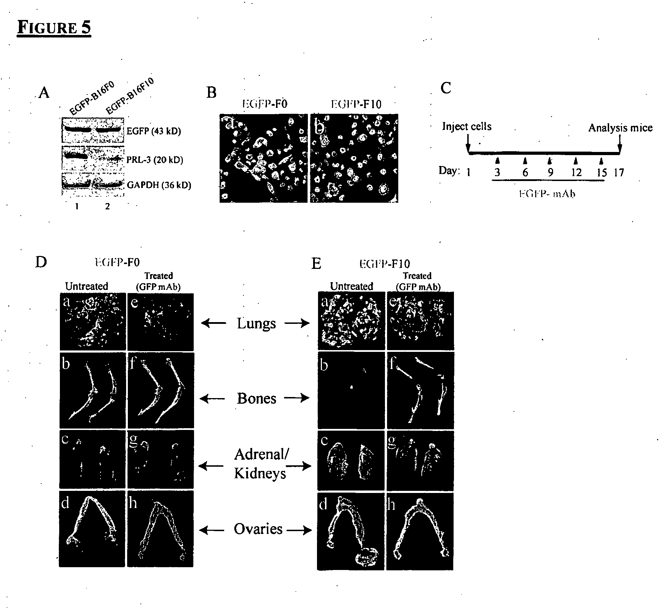

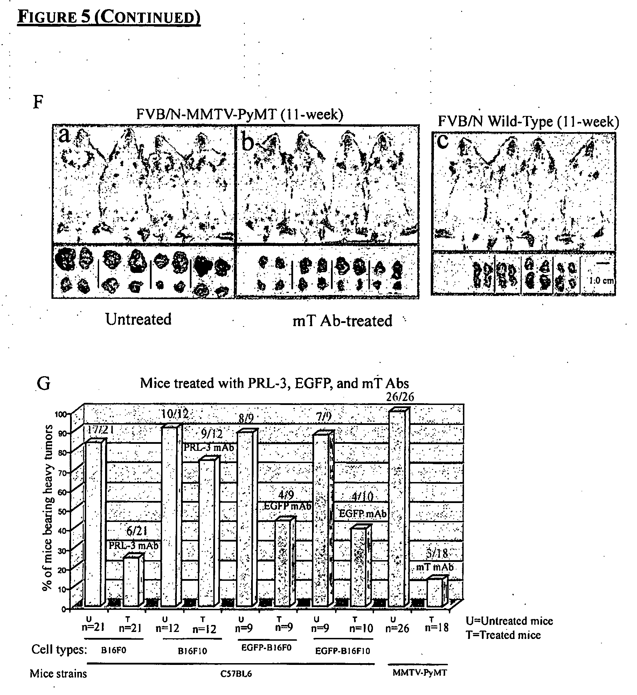

Figure 5 is a diagram showing that PRL-3-, EGFP- or mT-Abs effectively inhibit the formation of PRL-3, EGFP, and mT expressing tumors. A, Total cell lysates were prepared from EGFP-F0 and EGFP-F10 cancer cells. Exogenous EGFP protein was detected in both cell lines. PRL-3 was only expressed in EGFP-F0 (but low in EGFP-F10). GAPDH was used as the loading control. B, EGFP-F0 and EGFP-F10 stable populations of pooled cells were showed to be heterogeneous expressing of EGFP. C. a therapeutic plan showed C57BL6 mice which were injected with 10⁶ cancer cells via tail veins (on day 1). Treated mice were i.v. injected with EGFP mAb. Untreated mice were i.v. injected with PBS. D-E, Organs were harvested, examined and imaged on 17-20 day. Tumors were found in lung, bone, adrenal, and ovary in the untreated group, but greatly reduced in treated mice. Note EGFP-Ab specifically inhibits EGFP-tumors, but not non-EGFP-tumors in lungs (panels, e). F. a. Untreated FVB/N-MMTV-PymT, b. mT mAb effectively inhibits the formation of breast tumors in transgenic MMTV-PymT mice c. non-transgenic mice show normal sizes of breast tissues as controls. G. Summary of results between untreated and treated mice with antibodies against intracellular targets. The numbers of ineffective (or tumor-bearing) mice were placed at the numerator and the total numbers of mice were placed at the denominator. 146 mice were tested by three different antibody therapies against their respective intracellular targets. N stands for number of mice in each group; fraction in each column represents proportion of mice bearing heavy tumors.

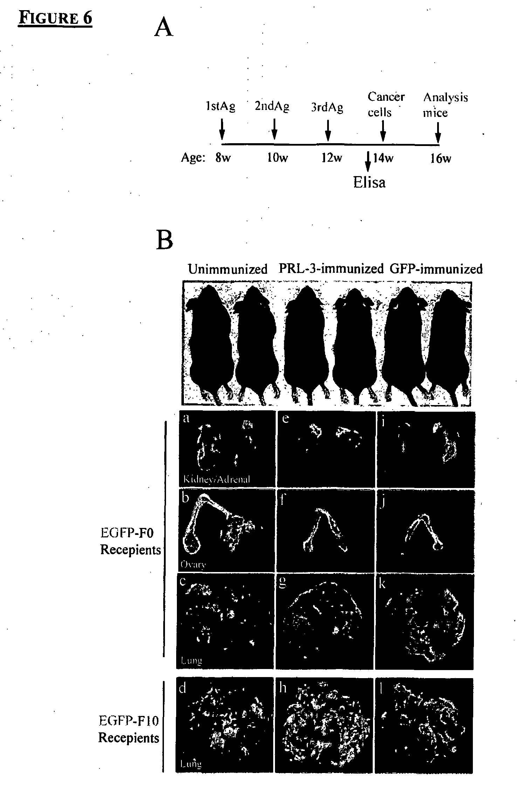

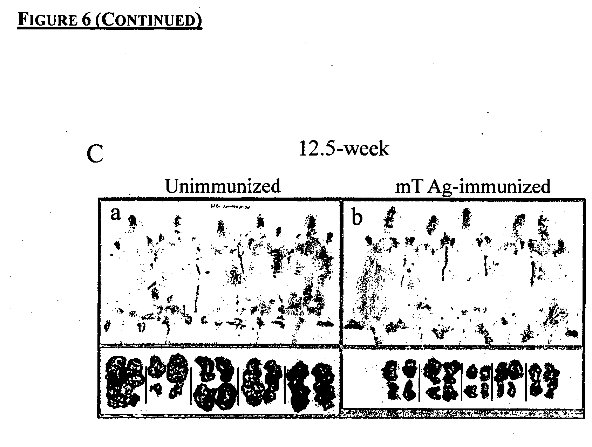

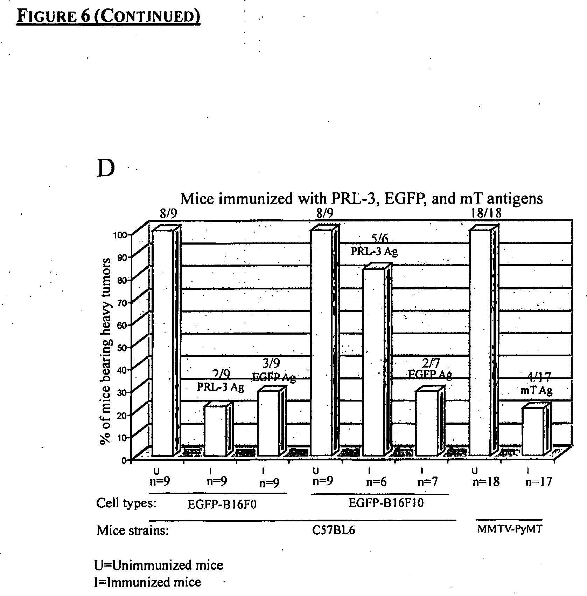

Figure 6 is a diagram showing that mice immunized with PRL-3, EGFP, or mT intracellular antigens could prevent PRL-3, EGFP and mT expressing tumors. A, a therapeutic plan of vaccination with respective antigen. B, Un-immunized, PRL-3-immunized or GFP-immunized mice were then challenged with either 1 million EGFP-F0 or EGFP-F10 cancer cells via tail veins. All the organs were examined 3 weeks post-cancer cell injection and photographed with fluorescent microscopy to show morphologies of metastatic tumors. Black tumors represent non-EGFP expressing melanoma cells, whereas green tumors represent those expressing EGFP. C, An overview of MMTV-PymT heterozygous transgenic femates: a. Unimmunized MMTV-PymT mice b. mT-immunized mice showed reduction breast tumors. D, Summary of results between unimmunized and immunized mice with intracellular antigens. 84 mice were used in vaccination experiment. N stands for number of mice in each group; fraction in each column represents proportion of mice bearing heavy tumors.

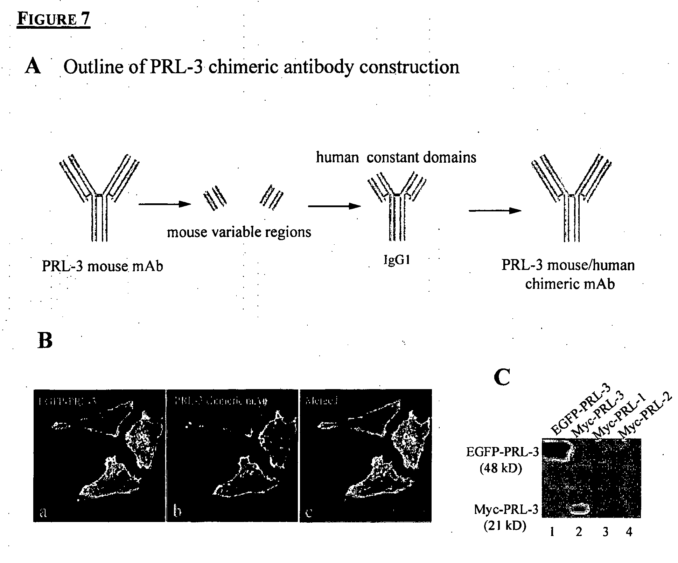

Figure 7 is a diagram showing that generation of PRL-3 chimeric mAb specifically reacts with PRL-3 antigen. A. A schematic diagram outlining the major steps of chimeric mAb construction. B. PRL-3 chimeric mAb recognizes EGFP-PRL-3 overexpressed in DLD-1 human colorectal cancer cells by indirect immunofluorescence: a, distribution of EGFP-PRL-3 in fixed DLD-1 cells (green); b, PRL-3 chimeric antibody and anti-human Texas-Red to reveal the binding to PRL-3 protein; c, merged image. Bar: 20 µm. C. Cell lysate derived from DLD-1 cells overexpressing EGFP-PRL-3, and lysates derived from CHO stable cell lines overexpressing myc-PRL-3, myc-PRL-1, and myc-PRL-2 were analyzed by western blot. PRL-3 chimeric antibody specifically recognized EGFP-PRL-3 (48 kDa) and myc-PRL-3 (20kDa), but does not react with myc-PRL-1 and myc-PRL-2.

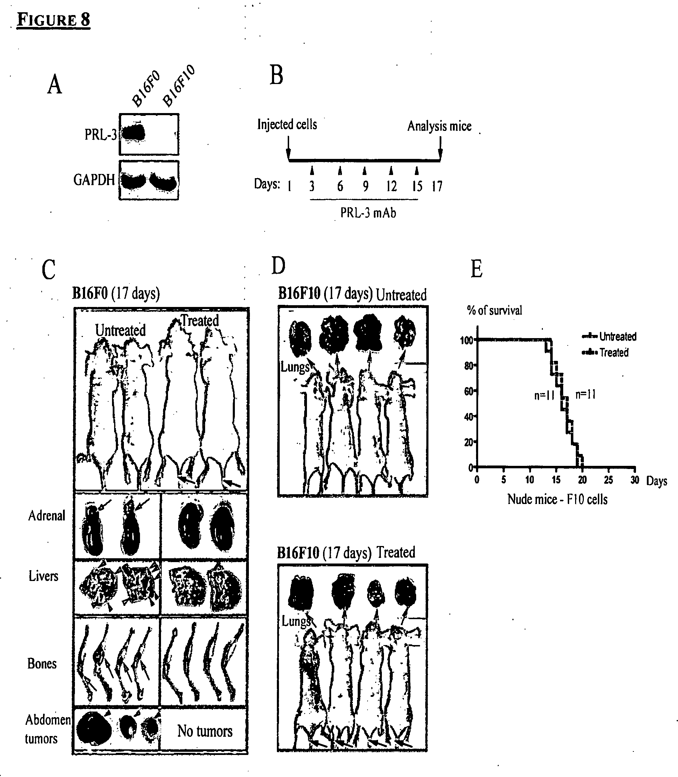

Figure 8 is a diagram showing that PRL-3 chimeric antibody effectively inhibits the formation of metastatic tumors formed by B16F0 cells that express endogenous PRL-3 A. Total cell lysates were prepared from F0 and F10 melanoma cells and analyzed by immunoblot. Abundant endogenous PRL-3 protein was detected in F0 but was almost undetectable in F10 cells. B. On day 1, *nude* mice (n=27) were injected with 1x10⁶ F0 cells via tail vein, followed by two intravenous administrations of the PRL-3 chimeric mAb per week (day 3, 6, 9, 12, 15). C. At the end of the experiment (day 17), mice were photographed and tissues were dissected. Metastatic tumors were found in the adrenal gland, liver, bone, and abdomen in untreated mice (left), but not in treated mice (right). D. *nude* mice (n=22) were injected with 1x10⁶ F10 cells for the experiment. At the end of the experiment (day 17), dozens of lung metastatic tumors were found both in untreated (top panel) and treated mice (bottom panel). E. Kaplan-Meier survival curves for 'treated' and 'untreated' F10 recipients were shown.

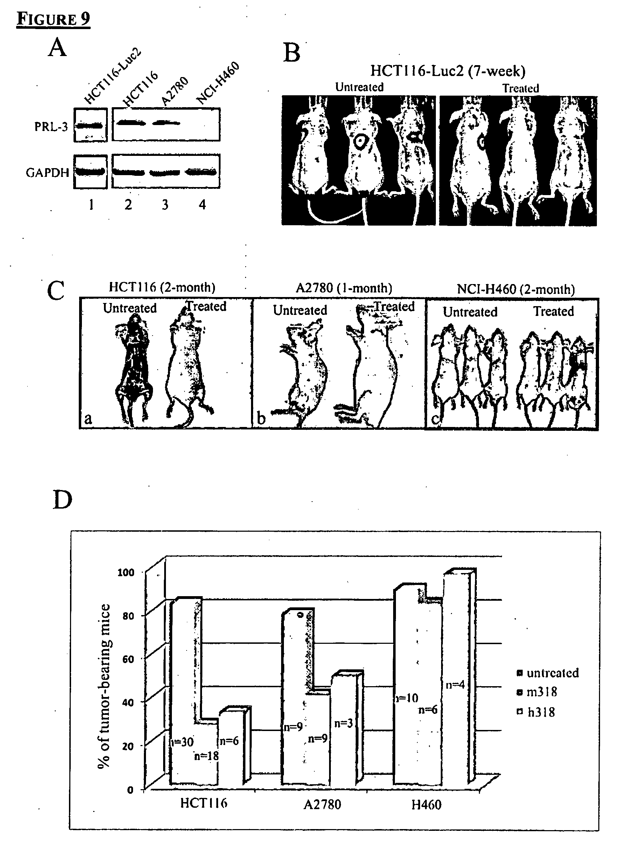

Figure 9 is a diagram showing that PRL-3 chimeric mAb inhibits the formation of metastatic tumors formed by A2780 cells and HCT-116 cells that express endogenous PRL-3. A. Total cell lysates were prepared from HCT116-*luc2*, HCT-116, A2780, and NCI-H460 cancer cell lines. Endogenous PRL-3 protein was detected in HCT116-*luc2*, HCT-116, and A2780 cells, but not in H460. B: On day 1, *nude* mice (n=6) were injected with 1x10⁶ HCT-116-*luc2* cancer cells and subsequently administered with PRL-3 chimeric mAb (n=3, treated) or PBS (n=3, untreated) on day 3, followed by two intravenous administrations of the PRL-3 chimeric mAb for 7-week. Both cancer cells and antibodies were injected via tail vein. IVIS® Imaging System was used to track and monitor tumor development *in vivo* at week 7. C. On day 1, *nude* mice were injected with 1x10⁶ cancer cells and treated as described in B. Paired experiments (untreated/treated) were terminated when mice appeared very sick. Experiment durations are indicated on the top of each panel. D. Summary of therapeutic results from mouse PRL-3 (R3-mAb) or chimeric PRL-3 (R3-hAb) antibody therapies in *nude* mice injected with three human cancer cell lines. Percentages of tumor-bearing mice were averaged from each group (n=numbers of mice) and indicated at the Y-axis. 101 mice in total were used in this experiment.

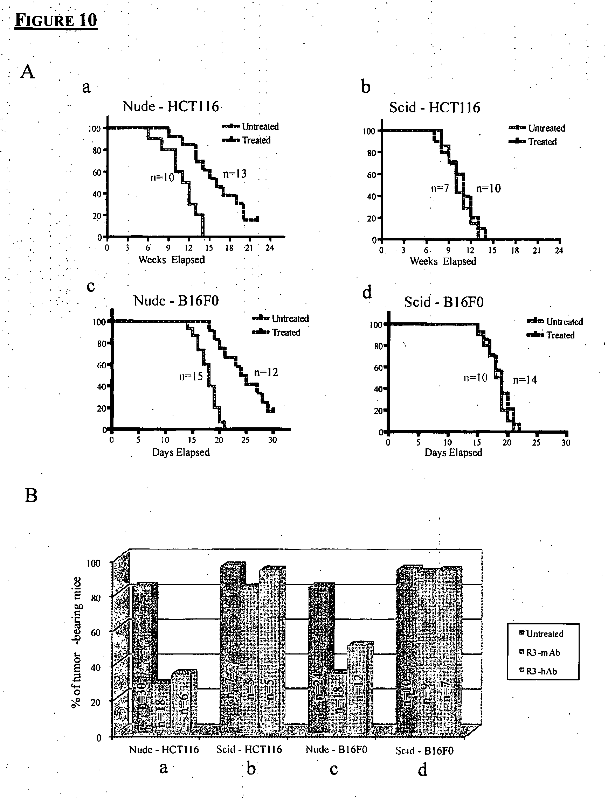

Figure 10 is a diagram showing that B-cells are important in mediating therapeutic efficacy of PRL-3 chimeric antibody. A. Kaplan-Meier survival curves of 'treated' and 'untreated' HCT-116-injected *nude* and *scid* mice (a, b). Kaplan-Meier survival curves of 'treated' and 'untreated' B16F0-injected *nude* and *scid* mice (c, d). B. Summary of results for therapeutic experiments in *nude* and *scid* mice (a-d). Percentages of tumor-bearing mice were averaged from each group (n=numbers of mice) and indicated at the Y-axis. 151 mice in total were used in this experiment.

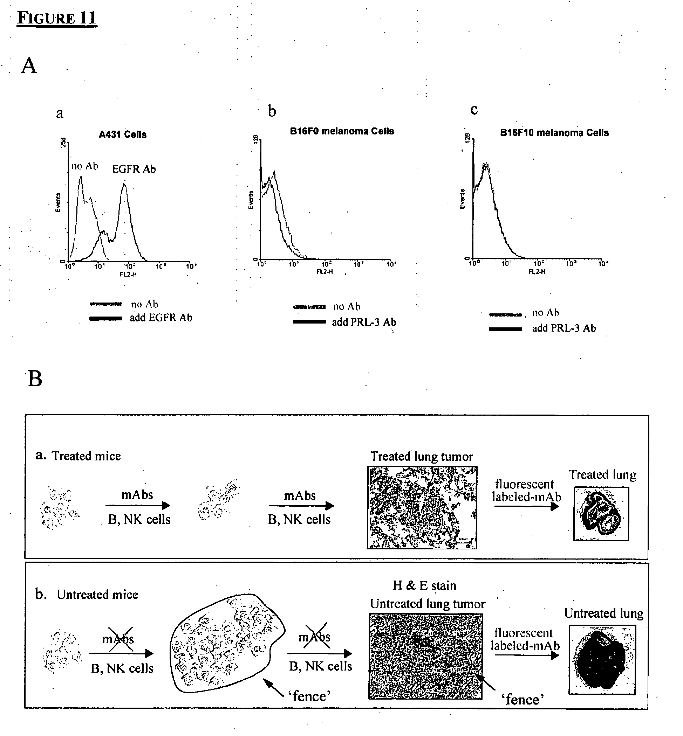

Figure 11 is a diagram showing that A. PRL-3 is not detectable at the cell surface. a. Peak-shift was observed between A431 cells incubated with EGF-receptor antibody (black line) compared to A431 cells without incubation of EGF-receptor antibody (red line). b. Peak-shift was not observed between F0 cells incubated with PRL-3 mAb (black line) and without Ab (red line). c: Peak-shift was not observed between F10 cells incubated with PRL-3 mAb (black line) and without Ab (red line). B. Labeled antibodies were intravenously injected 1 hr before IVIS live imaging based working models: a. Treated mice. Early delivery of the antibody will persistently attack cancer cells to prevent them from further progression, resulting in micro-metastases in open stages, as such, fluorescent labeled PRL-3 antibody can access and bind to metastatic lung tumors in 'treated mice'. We observed strong fluorescent labeled lung in treated mice. b, Untreated mice. The uncontrolled cancer cells rapidly multiplied. They freely developed into macro-metastases and established defense territory, some kind of tumor microenvironment, which makes less accessible to antibody and immune system, as consequences, fluorescent labeled PRL-3 antibodies were not able to label metastatic lung tumors in 'untreated mice'. We therefore are unable to observe fluorescent labeled metastatic lungs in untreated mice. In H&E stain section, black arrows showed a clear boundary ('fence') of the metastatic lung tumor from untreated B16F0 recipients. Scale bar: 200 µm. Red arrows indicated blood vessels.

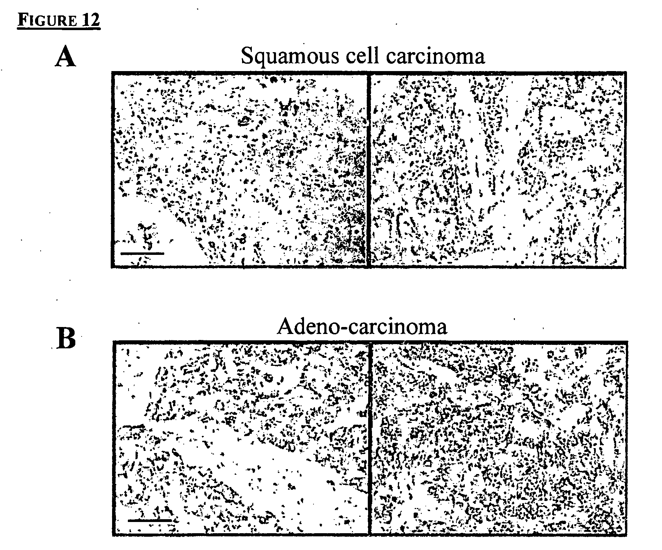

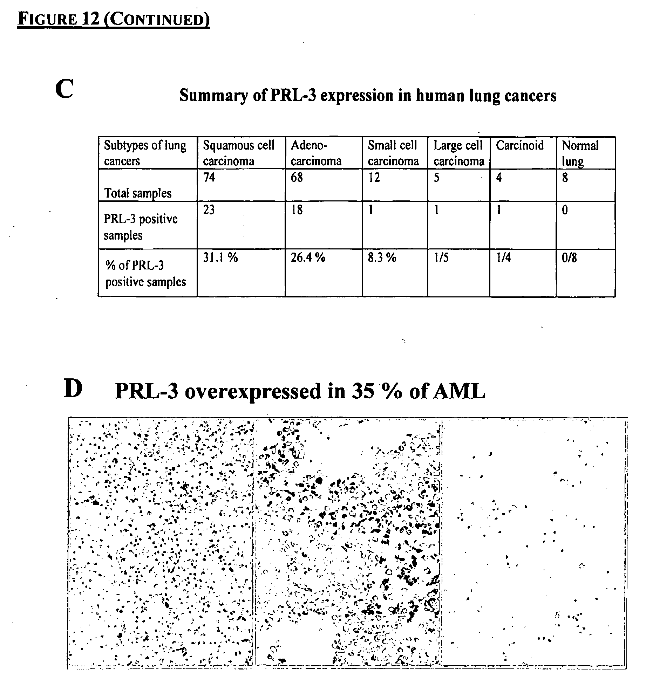

Figure 12 is a diagram showing that PRL-3 protein is upregulated in lung cancers and AML. A. Representative IHC staining of PRL-3 expression in lung squamous cell carcinoma. B. lung adenocarcinoma. Scale bar: 100 µm. C. The percentages of PRL-3-positive lung cancers as detected with immunohistochemistry (IHC) are summarized, grouped according to cancer subtypes. D. AML bone marrow samples were examined by IHC, 24 out of 69 (35%) showed PRL-3 expression. Three selected images were shown.

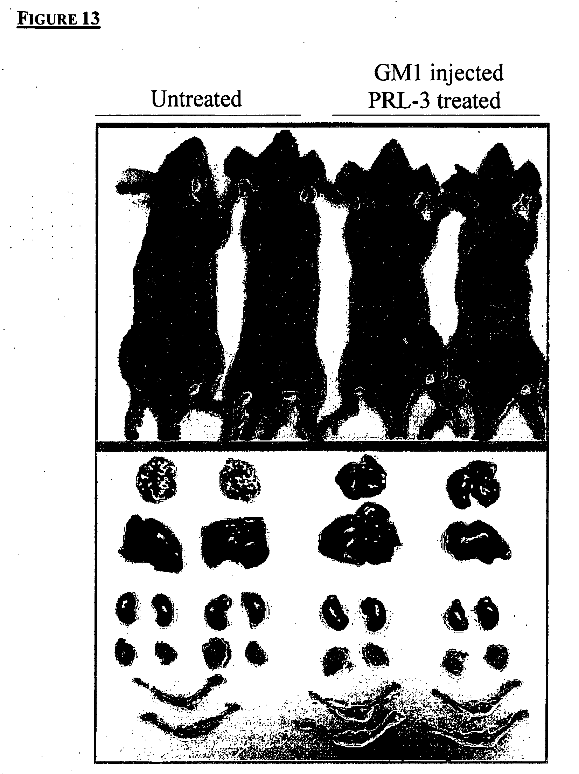

Figure 13 is a diagram showing that NK cells in Innate Immune system are involved in the therapy. *Nude* mice were injected (n=2) and un-injected (n=2) with GM1 antibody 24 hrs before the experiment. On day 1, all mice were injected with 1x10⁶ F0 cells via tail vein, followed by two intravenous administrations of the PRL-3 chimeric mAb per week (day 3, 6, 9, 12, 15) in GM1 injected mice. On day 18, the therapeutic efficacy was examined. GM1 injected *nude* mice showed more severe tumors (in black)-bearing burden in lung, liver, adrenal, testis, and bone than GM1 un-injected mice, regardless PRL-3 mAb treated or untreated.

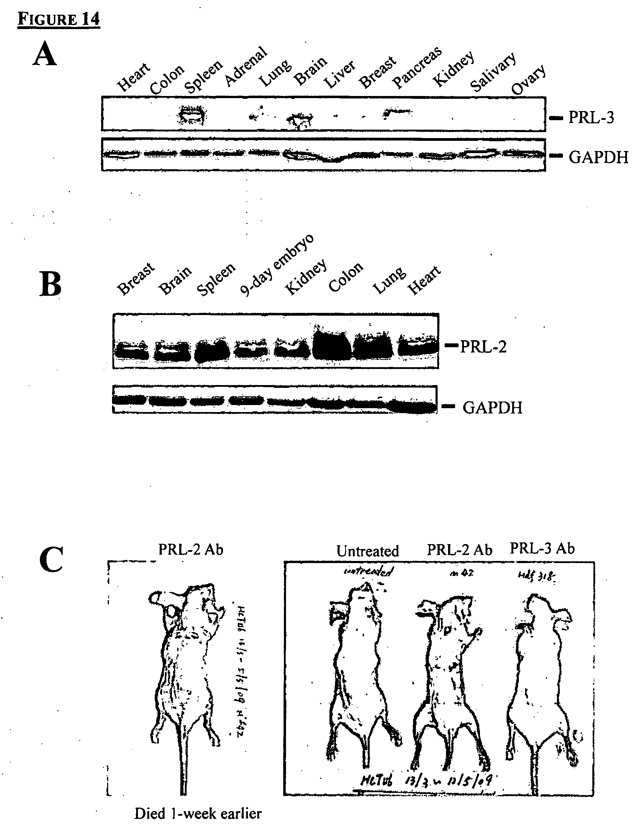

Figure 14 is a diagram showing that the outcome of the treatment is highly associated with tissue expression patterns of the targets. A, B. The protein expression patterns of PRL-3 and PRL-2 in normal mouse tissues by western blot, GAPDH was used as a loading control. C. HCT-116 (PRL-2 positive cell line) recipients fail to respond to PRL-2 antibody (the antibody also cross-reacts with PRL-1) therapy, most likely due to the fact that PRL-2 is ubiquitously expressed in most of mouse tissues that we have examined.

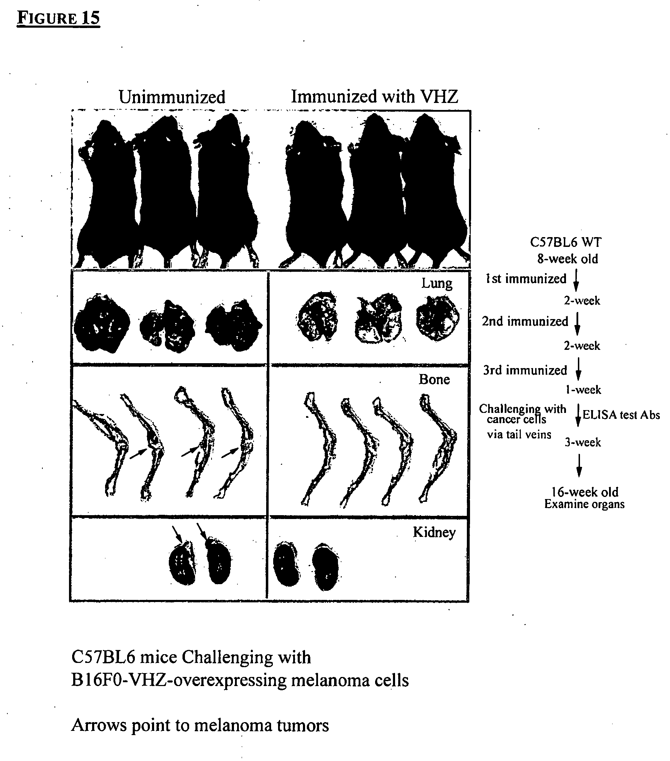

Figure 15 shows the results of immunization of C57BL6 mice with VHZ and monitoring tumor development. 8-week old C57BL6 mice were immunized by intraperitoneal injection with a total volume of 200 ml Freund's Adjuvant: 20 mg of VHZ antigen in 100 ml saline mixed with 100 ml of complete adjuvant (Cat#77140, Pierce). The next two immunizations were injected with a total volume of 200 ml adjuvant: 20 mg of VHZ antigen in 100 ml saline mixed with 100 ml of incomplete adjuvant (Cat#77145, Pierce). The second and third injections were administrated every 2 weeks, 100-200 ml of tail bleed was collected in a heparin-coated capillary tube. Plasma was prepared from blood sample and antibody titer was measured by ELISA. The detailed steps of ELISA were described previously. Mice with high titers of VHZ antibodies in their sera were selected and lateral tail vein injected with 1x106 VHZ-expressing cancer cells. Unvaccinated mice serve as a negative control in this study.

### DETAILED DESCRIPTION

To explore the possibility of antibodies targeting intracellular proteins, in this study, we have chosen to focus on three representative intracellular targets for anticancer therapies in animal models.

Firstly, we select a cancer associated-PRL-3 intracellular phosphatase as a target for the PRL-3 antibody therapy. PRL-3 is one of the three members (PRL-1, -2, and -3) in the PRL (phosphatase of regenerating liver) family which was identified in 1994 and 1998^{5,6}. The three PRLs form a subgroup of the protein tyrosine phosphatase (PTP) family⁷.

PRL-3 was first linked to colorectal cancer metastasis in 2001⁸. Subsequently, up-regulation of individual PRLs-PTPs was reported to be correlated with numerous types of advanced human metastatic cancers when compared with their normal counterparts⁹.

The PRL phosphatases represent an intriguing group of proteins being validated as biomarkers and therapeutic targets in human cancers¹⁰. PRLs are intracellular C-terminally prenylated phosphatases, while mutant forms of PRLs that lack the prenylation signal are often localized in nuclei^{11,12}.

The localization of PRL-1 and PRL-3 to the inner leaflet of the plasma membrane and early endosomes has been revealed by EM immunogold labeling¹³. Targeting PRLs by exogenous reagents to ablate PRLs-cancer cells requires their penetration into cells and is a challenging task.

Secondly, we chose the cytosolic enhanced green fluorescent protein (EGFP), a popular reporter protein originally isolated from the jellyfish *Aequorea victoria* that fluoresces green when exposed to blue light. EGFP is an intracellular protein that sometimes enters the nucleus. As a neutral exogenous protein, EGFP serves as an artificial 'cancer cell specific intracellular protein' in EGFP-B16F0 and EGFP-B16F10 melanoma cells.

These EGFP-overexpressing melanoma cells will then be targeted by EGFP mAb. We expect that EGFP antibody should have less undesired side effect in the animal model as EGFP is not expressed in the host tissues.

Last but not least, we selected a well known oncoprotein, the polyomavirus middle T (mT) intracellular kinase, as the third target¹⁴. We used the mammary cancer model of Py-mT transgenic heterozygous females (+/-) carrying the mT oncogene under the transcriptional control of the mouse mammary tumor virus promoter/enhancer. The transgenic mice have been widely used as excellent mouse tumor models for decades in the cancer research community to assess the relative contribution of the metastatic mammary tumor phenotype^{15,16}.

To obtain a better understanding and extend our previous promising pre-clinical data¹⁷ to future clinical applications in human, herein, we performed the entire antibody therapy study in wild type animal models to reveal a hitherto unrecognized concept that antibody therapy can be used for targeting intracellular-oncoproteins.

The Examples demonstrate that each of the above intracellular proteins can be targeted by antibodies against the cognate antigens. Accordingly, we provide for the treatment of cancers associated with intracellular oncoproteins by antibodies against such intracellular oncoproteins.

If an antibody can recognize its intracellular antigen, it may be that an intracellular antigen could potentially be used for vaccination to stimulate host immune system and produce antibodies against it. This prompted us to explore vaccination with intracellular oncoproteins against tumors which express specific intracellular antigens.

Vaccination is inexpensive yet effective in stimulating host immunity. Compared with extracellular antigens, intracellular proteins used in vaccination receive much less attention because of their intracellular location. The main objective of this vaccination is to artificially activate the immune system against a particular intracellular protein to prime the antibody in some individuals against certain malignant cells expressing that intracellular protein thus preventing tumor formation in the future.

Next, we used the same three intracellular antigens (PRL-3, EGFP, and mT) to perform vaccination in C57BL6 wild type mice. We obtained interesting results, as shown in the Examples, that mice immunized with an intracellular tumor antigen are able to eradicate these cancer cells expressed that specific tumor antigen to reduce the formations of tumors. More importantly, the data suggest the possibility of vaccination to prevent disease progression for individuals who are genetically at high risk in developing certain cancers caused by known intracellular oncoproteins. Such specific active immunotherapy of cancer, if successful and effective have clear advantages over passive immunotherapy.

Accordingly, we provide for the prevention of cancers associated with intracellular oncoproteins by vaccination using such intracellular oncoproteins.

In this study, we used 194 mice (Figure 6) for both antibody therapies and vaccinations with intracellular proteins for anticancer. Cancer research is rapidly moving towards individualized cancer therapy, as oncologists begin to use tailoring treatment strategies to individual patients. Antibody therapy and Vaccination may fulfil the promise of personalized treatment and could be the future of individualized medicine because we all have the ability to make antibodies for our own recovery.

Currently, vaccines are mainly used to prevent bacteria and viral infections and to target a few extracellular proteins (receptors) on cancer cells. Our data indicate that strategies targeting intracellular oncoproteins hold enormous promise for cancer prevention in the future.

### TREATMENT OF CANCER WITH ANTIBODIES AGAINST INTRACELLULAR ONCOPROTEINS

As shown in the Examples, we demonstrate that antibodies against PRL-3, EGFP and polyomavirus middle T (Py-mT) as well as other intracellular antigens, can surprisingly bind to their intracellular targets.

According to the expectation in the literature, targeting intracellular proteins and such other intracellular oncoproteins with antibodies to ablate cancer cells and cancer metastasis has never been previously thought to be possible because of their intracellular location. We have shown that this is not the case.

We therefore provide generally for antibodies against cancer causing oncoproteins, which oncoproteins are intracellular in nature, as cancer therapies, particularly therapies for cancer metastasis. We provide for use of an antibody against a cancer causing oncoprotein, which oncoprotein is intracellular in nature, in a method of treatment of cancer such as cancer metastasis.

We provide for methods of treating cancers and cancer metastases associated with cancer causing oncoproteins, which oncoproteins are intracellular in nature, by administration of a therapeutically effective amount of an antibody against such an intracellular oncoprotein to a patient in need thereof.

An antibody capable of binding to an intracellular oncogenic polypeptide or oncoprotein, or a variant, homologue, derivative or fragment thereof, may be used to treat cancer. The antibody may be capable of crossing the plasma membrane of a cell.

The antibody may be capable of binding to and inhibiting a biological activity of the oncoprotein. For example, where the oncoprotein comprises PRL-1 or PRL-3, the biological activity which may be inhibited may comprise protein tyrosine phosphatase (PTP) activity.

The antibody may be capable of preventing metastasis of a cancer, preferably colorectal cancer, ovarian cancer, breast cancer, liver cancer, pancreatic cancer, prostate cancer, gastric cancer, lung cancer, penis cancer, cervical cancer, brain cancer, esophageal cancer, bladder carcinoma, kidney renal cell carcinoma, ovary lymphoma and skin melanoma.

The antibody may comprise a monoclonal antibody or a humanised monoclonal antibody.

The antibody may be comprised in a combination with another entity or substance, such as a cancer drug.

The antibody capable of binding to an intracellular oncoprotein may be comprised in a pharmaceutical composition comprising such an antibody or combination, together with a pharmaceutically acceptable excipient, diluent or carrier.

The antibody or a pharmaceutical composition may be used in a method of treatment or prevention of cancer or metastasis thereof.

The method may comprise exposing a cancer cell to the antibody. The method may comprise administering a therapeutically effective amount of the antibody, combination or composition to an individual suffering or suspected of suffering from cancer. The cancer may comprise a metastatic cancer.

The cancer may comprise colorectal cancer, ovarian cancer, breast cancer, liver cancer, pancreatic cancer, prostate cancer, gastric cancer, lung cancer, penis cancer, cervical cancer, brain cancer, esophageal cancer, bladder carcinoma, kidney renal cell carcinoma, ovary lymphoma and skin melanoma.

The number of metastatic tumours in a treated individual may be reduced by at least 50% compared to an untreated individual. It may be reduced by at least 60%. It may be reduced by at least 70%. It may be reduced by at least 80%. The number of metastatic tumours in a treated individual may be reduced by at least 90% compared to an untreated individual.

We further describe a method of treatment or prevention of cancer, such as metastatic cancer, in an individual suffering or suspected to be suffering from cancer, the method comprising administering a therapeutically effective amount of an antibody as described, a combination as described or a composition as described, to the individual.

The method may comprise a feature as set out in any of the above paragraphs.

We further provide a method of treatment or prevention of cancer, such as metastatic cancer, in an individual suffering or suspected to be suffering from cancer, the method comprising diagnosing cancer in the individual and treating the individual by a method as described.

We describe a method comprising the steps of providing an antibody as described and allowing the antibody to bind to an oncogenic polypeptide.

The antibody may be allowed to bind to a cell expressing a such an oncogenic polypeptide. The oncogenic polypeptide may comprise an intracellular oncogenic polypeptide.

### PREVENTION OF CANCER BY VACCINATION WITH INTRACELLULAR ONCOPROTEINS

According to the expectation in the literature, targeting intracellular PRLs and such other intracellular oncoproteins with antibodies to ablate cancer cells and cancer metastasis has never been previously thought to be possible because of their intracellular location.

Accordingly, vaccination with intracellular oncoproteins to prevent cancer cell formation, cancer cell growth and cancer metastasis has never been previously thought to be possible. We have shown that this is not the case.

We therefore provide generally for cancer causing oncoproteins, which oncoproteins are intracellular in nature, as cancer vaccines, particularly vaccines against cancer metastasis.

We provide for use of an oncoprotein, which oncoprotein is intracellular in nature,, in a method of prevention of cancer including cancer metastases associated with cancer causing oncoproteins.

We provide for methods of preventing cancer including cancer metastases associated with cancer causing oncoproteins, which oncoproteins are intracellular in nature, by administration of a prophylactically effective amount of the intracellular oncoprotein to a patient in need thereof.

We provide a pharmaceutical composition comprising an antibody against an intracellular oncoprotein, together with a pharmaceutically acceptable excipient, diluent or carrier. We provide for the use of such a pharmaceutical composition for the treatment of cancer including cancer metastasis.

We provide a pharmaceutical composition comprising an intracellular oncoprotein, together with a pharmaceutically acceptable excipient, diluent or carrier. We provide for the use of such a pharmaceutical composition for the prevention of cancer including cancer metastasis.

### INTRACELLULAR ONCOPROTEINS

Intracellular oncoproteins and intracellular antigens may include any one or more of the following, or their variants, derivatives, homologues or fragments:

### PRL-3

The following text is adapted from OMIM entry 606449.

PRL-3 is also known as Protein-Tyrosine Phosphatase, Type 4A, 3; PTP4A3. The chromosomal location of PRL-3 is at gene map locus 8q24.3.

In the heart, protein kinases regulate contractility, ion transport, metabolism, and gene expression. Phosphatases, in addition to their role in dephosphorylation, are involved in cardiac hypertrophy and dysfunction.

By database searching and screening of a heart cDNA library, Matter et al. 2001, Biochem. Biophys. Res. Commun. 283: 1061-1068 identified a cDNA encoding PTP4A3, which they termed PRL3. The deduced PRL3 protein is 76% identical to PRL1 (PTP4A1; 601585) and 96% identical to mouse Prl3. Northern blot analysis revealed expression of an approximately 2.3-kb PRL3 transcript predominantly in heart and skeletal muscle, with lower expression in pancreas. This expression pattern is distinct from the wider expression of PRL1 and PRL2 (PTP4A2; 601584). In situ hybridization analysis localized PRL3 expression to cardiomyocytes. Tris glycine gel analysis showed that PRL3 is expressed as a 22-kD protein. Functional and mutation analyses indicated that phosphate cleavage is dependent on cys104 of PRL3. Overexpression of PRL3 resulted in increased cell growth. Western blot analysis showed dephosphorylation of p130cas (BCAR1; 602941) in response to angiotensin II (106150), suggesting a role for PRL3 in the modulation of intracellular calcium transients induced by angiotensin II.

To gain insights into the molecular basis for metastasis, Saha et al. 2001, Science 294: 1343-1346 compared the global gene expression profile of metastatic colorectal cancer with that of primary cancers, benign colorectal tumors, and normal colorectal epithelium. PRL3 was expressed at high levels in each of 18 cancer metastases studied but at lower levels in nonmetastatic tumors and normal colorectal epithelium. In 3 of 12 metastases examined, multiple copies of the PRL3 gene were found within a small amplicon located at chromosome 8q24.3. Saha et al. (2001) concluded that the PRL3 gene is important for colorectal cancer metastasis.

Using the Stanford G3 radiation hybrid panel and database sequence analysis, Saha et al. (2001) mapped the PRL3 gene to surrounding marker 145.20. The PRL3 gene is also tightly linked to marker S.HGC-22154, which is located at 8q24.3, approximately 3 Mb from the 8q telomere.

### [End of text adapted from OMIM]

Mouse and human PRL-3 proteins were described in detail in Li et al (2005), Clin Cancer Res;11:2195-204.

### PRL-3 Sequences

The methods and compositions described here make use of PRL-3 polypeptides, which are described in detail below. As used here, the term "PRL-3" is intended to refer to a sequence set out in Table D2 below.

| **Unigene** | **Description** |
|---|---|
| | Homo sapiens potentially prenylated protein tyrosine phosphatase |
| AF041434.1 | hPRL-3 mRNA, complete cds Homo sapiens protein tyrosine phosphatase type IVA, member 3 |
| BT007303.1 | mRNA, complete cds |
| AK128380.1 | Homo sapiens cDNA FLJ46523 fis, clone THYMU3034099 Homo sapiens protein tyrosine phosphatase type IVA, member 3 |
| NM_007079.2 | (PTP4A3), transcript variant 2, mRNA |
| AY819648.1 | Homo sapiens HCV p7-transregulated protein 2 mRNA, complete cds Homo sapiens protein tyrosine phosphatase type IVA, member 3, |
| BC003105.1 | mRNA (cDNA clone MGC:1950 IMAGE:3357244), complete cds Homo sapiens protein tyrosine phosphatase type IVA, member 3 |
| NM_032611.1 | (PTP4A3), transcript variant 1, mRNA |
| AK311257.1 | Homo sapiens cDNA, FLJ18299 Human protein tyrosine phosphatase homolog hPRL-R mRNA, partial |
| U87168.1 | cds Homo sapiens mRNA for protein tyrosine phosphatase hPRL-3, short |
| AJ276554.1 | form |
| BC066043.1 | Mus musculus protein tyrosine phosphatase 4a3, mRNA (cDNA clone |
| | MGC:90066 IMAGE:6415021), complete cds Mus musculus cDNA, clone:Y1G0102I03, strand:plus, reference:ENSEMBL:Mouse-Transcript- |
| AK190358.1 | ENST:ENSMUST00000053232, based on BLAT search Mus musculus full open reading frame cDNA clone RZPDo836H0950D for gene Ptp4a3, Protein tyrosine phosphatase 4a3; complete cds, incl. |
| CT010215.1 | stopcodon Mus musculus adult male brain UNDEFINED_CELL_LINE cDNA, RIKEN full-length enriched library, clone:M5C1053F14 |
| AK147489.1 | product:protein tyrosine phosphatase 4a3, full insert sequence Mus musculus activated spleen cDNA, RIKEN full-length enriched library, clone:F830102P03 product:protein tyrosine phosphatase 4a3, |
| AK172192.1 | full insert sequence Mus musculus 6 days neonate spleen cDNA, RIKEN full-length enriched library, clone:F430011C20 product:protein tyrosine |
| AK143702.1 | phosphatase 4a3, full insert sequence Mus musculus potentially prenylated protein tyrosine phosphatase |
| AF035645.1 | mPRL-3 (Prl3) mRNA, complete cds |
| NM_008975.2 | Mus musculus protein tyrosine phosphatase 4a3 (Ptp4a3), mRNA Mus musculus 0 day neonate skin cDNA, RIKEN full-length enriched library, clone:4632430E19 product:protein tyrosine phosphatase 4a3, |
| AK014601.1 | full insert sequence Mus musculus adult male lung cDNA, RIKEN full-length enriched library, clone:1200003F10 product:protein tyrosine phosphatase 4a3, |
| AK004562.1 | full insert sequence Mus musculus 18-day embryo whole body cDNA, RIKEN full-length |
| AK003954.1 | enriched library, clone:1110029E17 product:protein tyrosine phosphatase 4a3, full insert sequence |
| BC027445.1 | Mus musculus protein tyrosine phosphatase 4a3, mRNA (cDNA clone MGC:36146 IMAGE:4482106), complete cds |

A "PRL-3 polypeptide" may comprise or consist of a human PRL-3 polypeptide, such as the sequence having Unigene accession number AF041434.1.

Homologues variants and derivatives thereof of any, some or all of these polypeptides are also included. For example, PRL-3 may include Unigene Accession Number BC066043.1.

### VHZ

The methods and compositions described here make use of VHZ, which is described in detail below.

VHZ is also known as DUSP23, MOSP, LDP-3, DUSP25, FLJ20442 and RP11-190A12.1

As used here, the term "VHZ" may refer to a polypeptide sequence having GenBank Accession number NP_060293.2, NP_081001.1, XP_341157.1, XP_001170819.1, XP_001170835.1, XP_545747.2, NP_001076078.1, NP_001011371.1, NP_783859.1, NP_001034709.1, XP_001480730.1, XP_001117253.1 or XP_001117256.1.

A "VHZ polypeptide" may comprise or consist of a human VHZ polypeptide, such as the sequence having accession number NP_060293.

With regard to nucleic acid sequences, the terms "VHZ polynucleotide", "VHZ nucleotide" and "VHZ nucleic acid" may be used interchangeably, and should be understood to specifically include both cDNA and genomic VHZ sequences. These terms are also intended to include a nucleic acid sequence capable of encoding a VHZ polypeptide and/or a fragment, derivative, homologue or variant of this.

Where reference is made to a VHZ nucleic acid, this should be taken as a reference to any member of the VHZ family of nucleic acids. Of particular interest are VHZ nucleic acids selected from the group consisting of: NM_017823.3, NM_026725.2, XM_341156.3, XM_001170819.1, XM_001170835.1, XM_545747.2, NM_001082609.1, NM_001011371.1, NM_75732.1, NM_001039620.1, XM_001480680.1, XM_001117253.1 or XM_001117256.1.

Also included are any one or more of the nucleic acid sequences set out as "Other VHZ nucleic acid sequences" below.

For example, the VHZ nucleic acid may comprise a human VHZ sequence having GenBank Accession Number NM_017823.3.

### ANTI-INTRACELLULAR ONCOPROTEIN ANTIBODIES

We describe the generation and production of antibodies against intracellular oncogenic proteins, i.e., anti-intracellular oncoprotein antibodies. Such anti-intracellular oncoprotein antibodies may be capable of binding oncoprotein, preferably intracellular oncoprotein.

Both monoclonal antibodies and humanised monoclonal antibodies and their properties are provided in this document. Humanised versions of each of these antibodies are also disclosed.

For the avoidance of doubt, where a specific antibody designation is referred to in this document, this should be taken to include a reference to both the mouse monoclonal antibody (as secreted by a hybridoma), as well as to the humanised version of it, unless the context dictates otherwise. Thus, for example, where antibody NNN is referred to, this includes both the monoclonal antibody NNN (i.e., the mouse hybridoma secreted antibody designated NNN), as well as a humanised monoclonal antibody NNN.

The antibodies described in this document may be produced by a person skilled in the art from the information disclosed in this document, and employing molecular biology techniques which we also describe in detail.

For this purpose, sequences of the variable regions of a monoclonal antibody against the intracellular oncoprotein may be employed. Variants, homologues, fragments and derivatives of these variable regions may be generated. Using such sequence information, a skilled person may produce antibodies comprising these variable regions or their variants, homologues, fragments and derivatives. Sequences of nucleic acid constructs for expressing monoclonal antibodies against a intracellular oncoprotein may also be used. Finally, sequences of constructs capable of expressing humanised monoclonal antibodies against intracellular oncoproteins may also be employed. We describe methods of expressing the antibodies of interest from cells transfected with the constructs, as well as variants, homologues, fragments and derivatives of these humanised constructs.

Using such sequences and the expression methods, the skilled person may readily transfect relevant host cells and cause them to express the whole monoclonal or humanised anti-intracellular oncoprotein antibodies, or variants, homologues, fragments and derivatives thereof.

We further provide for polypeptides in general having intracellular oncoprotein binding activity. Such polypeptides include anti-intracellular oncoprotein antibodies. The intracellular oncoprotein-binding polypeptides may comprise one or more of the same or similar properties as the monoclonal antibodies against the intracellular oncoprotein described above. The polypeptides will be referred to for convenience generally as "anti-intracellular oncoprotein antibodies".

It is within the skills of a reader to construct binding molecules which may not be (or may not be described as) antibodies or immunoglobulins but which comprise anti-intracellular oncoprotein binding activity as described here. Accordingly, and where the context allows the term "anti-intracellular oncoprotein antibodies" should be taken to include any molecule so long as it is capable of binding intracellular oncoprotein. Such molecules may include polypeptides, small molecules, as well as antibodies and immunoglobulins, and may be identified through various means known in the art, for example by screening a suitable library for intracellular oncoprotein binding activity.

The anti-intracellular oncoprotein antibodies (which include intracellular oncoprotein binding molecules) may comprise similar or identical properties may as the monoclonal antibodies against intracellular oncoprotein described above. Such similar or identical properties may in particular include binding properties. The anti-intracellular oncoprotein antibodies may in general be capable of binding to intracellular oncoprotein polypeptides.

Thus, the term "anti-intracellular oncoprotein antibody" will be taken to include monoclonal antibodies (as well as their humanised counterparts). Also included are polypeptides comprising the variable regions of antibodies against intracellular oncoproteins or variants, homologues, fragments and derivatives thereof. This term should also be taken to include reference to variants, homologues, fragments and derivatives of the anti-intracellular oncoprotein antibodies, as described below, where the context permits.

### INTRACELLULAR ONCOPROTEIN EPITOPES

The anti-intracellular oncoprotein antibodies may specifically bind to an epitope on the intracellular oncoprotein.

Methods are known in the art to determine an epitope that is bound by a particular antibody. Such epitope mapping methods are described for example in Hanson et al., (2006). Respiratory Research, 7:126. Furthermore, a skilled person will be able to generate antibodies and screen them for particular properties.

The anti-PRL antibodies may comprise the variable region of a monoclonal antibody against intracellular oncoprotein. They may comprise the same or different variable regions in a single antibody molecule. They may comprise one variable region, or more than one variable region. Accordingly, we provide the skilled person with the ability to produce any number of antibodies which comprise the same or similar binding reactivity as a monoclonal antibody against intracellular oncoprotein.

Such antibodies may comprise the full or substantially complete sequences of an antibody (i.e., heavy chain and light chain), or they may comprise a fragment of a whole antibody (such as Fv, F(ab') and F(ab')₂ fragments or single chain antibodies (scFv)). The antibodies may further comprise fusion proteins or synthetic proteins which comprise the antigen-binding site of the antibody, as described in detail below. It will also be evident that such antibodies may be engineered for desirable properties, such as lowered host reactivity, reduced rejection, etc.

The engineering could include "humanisation", by which term we mean the inclusion of (or substitution with) one or more human residues or sequences in an antibody sequence such as a mouse antibody sequence. "Humanisation" in the context of this document includes "chimeric" antibodies, in which the antibody comprises discrete sections of mouse and human sequences, e.g., where one or both of the variable regions comprise mouse sequences, and the remainder of the antibody molecule (such as the constant region) comprises human sequences. In such chimeric antibodies, the whole of the variable regions of, for example, a mouse or rat antibody may be expressed along with human constant regions. This provides such a chimeric antibody with human effector functions and also reduces immunogenicity (HAMA) caused by the murine Fc region.

Generally, a "chimeric antibody" may refer to an antibody having either a heavy and light chain encoded by a nucleotide sequence derived from a murine immunoglobulin gene and either a heavy and light chain encoded by a nucleotide sequence derived from a human immunoglobulin gene.

"Humanisation" also includes CDR grafted or reshaped antibodies. It thus includes engineering at a more discrete level, e.g., antibodies in which the mouse variable region has been mutated to include human residues to reduce immunogenicity. In such an antibody, only the complimentarity determining regions from the rodent antibody V-regions may be combined with framework regions from human V-regions. Such antibodies should be more human and less immunogenic than chimaeric antibodies.

The anti-intracellular oncoprotein antibody may generally be capable of binding to the oncoprotein polypeptide in a number of conditions.

In one embodiment, the binding environment comprises an intracellular condition. That is to say, the anti-intracellular oncoprotein antibody may be capable of binding to a oncoprotein polypeptide in an intact or unpermeabilised cell. Such an unpermeabilised cell may comprise a cell which has not been exposed, or not exposed substantially, to a permeabilisation agent such as a detergent (e.g., Triton X-100) or digitonin.

An anti-intracellular oncoprotein antibody as described here may be capable of binding to oncoprotein when it is inside the cell, within the cell membrane or encapsulated within the cell. Similarly, an intracellular oncoprotein polypeptide may be bound by an anti-intracellular oncoprotein antibody, as described generally in this document, in the context of an environment that comprises the interior of a cell. The intracellular oncoprotein polypeptide may be associated with one or a number of cellular structures, for example, the inner leaflet of the cell membrane, an organelle, a cytoskeletal structure, the nuclear membrane, etc. The intracellular oncoprotein polypeptide may be located within the nucleus. In each of these cases, the anti-intracellular oncoprotein antibody may be capable of binding to the oncoprotein polypeptide within the intracellular environment.

The anti-intracellular oncoprotein antibody may be capable of binding to a intracellular oncoprotein polypeptide in an intracellular environment in a number of ways. The anti-intracellular oncoprotein antibody may be capable of crossing the plasma membrane. It may be capable of otherwise gaining access to a binding region of the intracellular oncoprotein polypeptide, for example by cellular uptake. It may be internalised or translocated or otherwise delivered into the cell by any means.

In another embodiment, the binding condition comprises an extracellular condition. The anti-intracellular oncoprotein antibody may therefore be capable of binding to its cognate intracellular oncoprotein polypeptide in an extracellular environment.

The anti-intracellular oncoprotein antibody may therefore be capable of binding to an oncoprotein polypeptide extracellularly. In other words, an anti-intracellular oncoprotein antibody as described here may be capable of binding to the intracellular oncoprotein when it is outside the cell. Similarly, a intracellular oncoprotein polypeptide may be bound by an anti-intracellular oncoprotein antibody, as described generally in this document, in the context of an environment that is external to the interior of a cell. The anti-intracellular oncoprotein antibody may be capable of binding to a secreted intracellular oncoprotein polypeptide, as the case may be. The oncoprotein polypeptide may comprise a circulating oncoprotein polypeptide.

The anti-intracellular oncoprotein antibody may be capable of binding to bind to external or externalized oncoprotein polypeptides. They may bind to secreted oncoprotein polypeptides in blood circulation.

The binding between the anti-intracellular oncoprotein antibody and its target may be more or less strong or weak, transient, semi-permanent or permanent.

Binding of the anti-intracellular oncoprotein antibody to the intracellular oncoprotein polypeptide may take place within the cell. Such binding may inactivate, inhibit or lower an activity of the intracellular oncoprotein polypeptide. The binding may neutralise a intracellular oncoprotein activity. The activity may comprise any biological activity caused by or associated with the intracellular oncoprotein polypeptide. The activity may comprise binding to another protein, for example a downstream protein or factor. Binding of anti-intracellular oncoprotein antibody to intracellular oncoprotein polypeptide may inactivate, inhibit or lower an activity of a downstream protein or factor. The activity may comprise communication with other cells, for example cells such as metastatic cancer cells in circulation. Thus, the anti-intracellular oncoprotein antibodies may neutralise intracellular oncoprotein polypeptides in blood circulation to prevent intracellular oncoprotein from binding with down-stream factors or from their communicating with other cells in circulation.

The activity may comprise a biochemical activity or a pathogenic activity. The biochemical activity may comprise a catalytic activity. The catalytic activity may comprise phosphatase activity. The activity may comprise growth regulating activity, cancer activity, carcinogenic activity or metastatic activity.

The antibodies against intracellular oncoprotein may be used for treatment of disease in humans or other animals. Such anti-intracellular oncoprotein antibodies may have anti-cancer activity. The anti-intracellular oncoprotein antibodies are capable of preventing metastatic spread of cancer tumours.

Accordingly, we provide for the use of anti-intracellular oncoprotein antibodies in the treatment or prevention of disease, such as cancer. The cancer may comprise a metastatic cancer. The anti-intracellular oncoprotein antibodies may be used as drugs or therapies to treat metastasis of a cancer, such as an established tumour. They may be used to prevent cancer or metastasis thereof.

The cancer which is treatable or preventable may include one which is associated with expression or over-expression of a intracellular oncoprotein protein. The intracellular oncoprotein protein may be a relevant member of the family.

The cancer may include any of a number of cancers, such as colorectal cancer, ovarian cancer, breast cancer, liver cancer, pancreatic cancer, prostate cancer, gastric cancer, lung cancer, penis cancer, cervical cancer, brain cancer, esophageal cancer, bladder carcinoma, kidney renal cell carcinoma, ovary lymphoma and skin melanoma.

The treatment may comprise generally contacting a cancer cell, or a cell suspected of being a cancer cell, with an anti-intracellular oncoprotein antibody. The cell may be exposed to an anti-intracellular oncoprotein antibody. It may or in addition be exposed to a second compound such as a cancer drug, antibody against another protein, or an antibody against another oncoprotein. Where this is so, the cell may be exposed to both anti-intracellular oncoprotein antibody and the second compound together, or individually in sequence. The exposure may be repeated a number of times. Any combination of anti-intracellular oncoprotein antibody and the second compound in whatever amount or relative amount, in whatever timing of exposure, may be used.

We therefore provide for the use of combinations of anti-intracellular oncoprotein antibodies and other compounds, as described above, in the treatment of disease such as cancer.

The cell may be an individual cell, or it may be in a cell mass, such as a cancer or tumour cell mass. The cell may be inside the body of an organism. The organism may be one which is known to be suffering from cancer, or it could be one in which cancer is suspected. The treatment may comprise administering the antibody or antibodies to the organism. As above, a single antibody may be administered, or a combination of anti-intracellular oncoprotein antibody and a second compound may be administered. The administration may be simultaneous or sequential, as described above. Thus, the treatment may comprise administering an anti-intracellular oncoprotein antibody simultaneously or sequentially with a second compound to the individual.

The anti-intracellular oncoprotein antibody may generally comprise any immunoglobulin capable of binding to a intracellular oncoprotein molecule, as described in more detail below.

### INTRACELLULAR ONCOPROTEIN POLYPEPTIDES

Intracellular oncoprotein polypeptides may be used for a variety of means, for example, for production or screening of anti-intracellular oncoprotein agents such as specific intracellular oncoprotein binding agents, in particular, anti-intracellular oncoprotein antibodies.

Intracellular oncoprotein polypeptides may also be used as vaccines for vaccination of individuals suffering from cancer, or suspected to be suffering from cancer. Thus, the intracellular oncoprotein polypeptides may be administered to such individuals to raise an immune response against the intracellular oncoprotein.

These are described in further detail below. The expression of intracellular oncoprotein polypeptides may be detected for diagnosis or detection of cancer, in particular breast cancer.

A "polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids.

"Polypeptides" include amino acid sequences modified either by natural processes, such as post-translational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications.

Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-inking, cyclization, disulfide bond formation, demethylation, formation of covalent cross-inks, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, Proteins - Structure and Molecular Properties, 2nd Ed., T. E. Creighton, W. H. Freeman and Company, New York, 1993 and Wold, F., Posttranslational Protein Modifications: Perspectives and Prospects, pgs. 1-12 in Posttranslational Covalent Modification of Proteins, B. C. Johnson, Ed., Academic Press, New York, 1983; Seifter et al., "Analysis for protein modifications and nonprotein cofactors", Meth Enzymol (1990) 182:626-646 and Rattan et al, "Protein Synthesis: Posttranslational Modifications and Aging", Ann NY Acad Sci (1992) 663:48-62.

The term "polypeptide" includes the various synthetic peptide variations known in the art, such as a retroinverso D peptides. The peptide may be an antigenic determinant and/or a T-cell epitope. The peptide may be immunogenic *in vivo.* The peptide may be capable of inducing neutralising antibodies *in vivo.*

As applied to intracellular oncoproteins, the resultant amino acid sequence may have one or more activities, such as biological activities in common with a intracellular oncoprotein polypeptide, for example a human intracellular oncoprotein. For example, a intracellular oncoprotein homologue may have a increased expression level in cancer cells compared to normal breast cells. In particular, the term "homologue" covers identity with respect to structure and/or function providing the resultant amino acid sequence has intracellular oncoprotein activity. With respect to sequence identity (i.e. similarity), there may be at least 70%, such as at least 75%, such as at least 85%, such as at least 90% sequence identity. There may be at least 95%, such as at least 98%, sequence identity. These terms also encompass polypeptides derived from amino acids which are allelic variations of the intracellular oncoprotein nucleic acid sequence.

Where reference is made to the "activity" or "biological activity" of a polypeptide such as an intracellular oncoprotein, these terms are intended to refer to the metabolic or physiological function of the intracellular oncoprotein, including similar activities or improved activities or these activities with decreased undesirable side effects. Also included are antigenic and immunogenic activities of the intracellular oncoprotein. Examples of such activities, and methods of assaying and quantifying these activities, are known in the art, and are described in detail elsewhere in this document.

### ANTIBODIES

The terms "antibody" and "immunoglobulin", as used in this document, may be employed interchangeably where the context permits. These term include fragments of proteolytically-cleaved or recombinantly-prepared portions of an antibody molecule that are capable of selectively reacting with or recognising an intracellular oncoprotein or an epitope thereof.

Non limiting examples of such proteolytic and/or recombinant fragments include Fab, F (ab') 2, Fab', Fv fragments, and single chain antibodies(scFv) containing a VL and VH domain joined by a peptide linker. These Fvs may be covalently or non-covalently linked to form antibodies having two or more binding sites.

By "ScFv molecules" we mean molecules wherein the VH and VL partner domains are linked via a flexible oligopeptide. A general review of the techniques involved in the synthesis of antibody fragments which retain their specific binding sites is to be found in Winter & Milstein(1991) Nature 349, 293-299.

Whole antibodies, and F(ab') 2 fragments are "bivalent". By "bivalent" we mean that the said antibodies and F(ab') fragments have two antigen combining sites. In contrast, Fab, Fv, ScFv and dAb fragments are monovalent having only one antigen combining site.

The anti-intracellular oncoprotein antibody may comprise a high affinity antibody with an off rate from 10⁻²s⁻¹ to 10⁻⁴s⁻¹ The off rate may be about 2 x 10⁻⁴s⁻¹.

The term "off-rate" as used in this document refers to the dissociation rate (k_{off}) of an antibody such as an anti-intracellular oncoprotein antibody disclosed here. It may be measured using BIAevaluation software (Pharmacia). A low off rate is desirable as it reflects the affinity of an Fab fragment for an antigen.

The term "affinity" is defined in terms of the dissociation rate or off-rate (k_{off}) of a an antibody such as an anti-intracellular oncoprotein antibody. The lower the off-rate the higher the affinity that a an antibody such as an anti-intracellular oncoprotein antibody has for an antigen such as intracellular oncoprotein.

The anti-intracellular oncoprotein antibody may comprise a peptide *per se* or form part of a fusion protein.

The anti-intracellular oncoprotein antibodies described here include any antibody that comprise intracellular oncoprotein binding activity, such as binding ability to intracellular intracellular oncoprotein.

The anti-intracellular oncoprotein antibodies also include the entire or whole antibody, whether mouse, humanised or human, such antibody derivatives and biologically-active fragments. These may include antibody fragments with intracellular oncoprotein binding activity that have amino acid substitutions or have sugars or other molecules attached to amino acid functional groups, etc.

The anti-intracellular oncoprotein antibody may comprise isolated antibody or purified antibody. It may be obtainable from or produced by any suitable source, whether natural or not, or it may be a synthetic anti-intracellular oncoprotein antibody, a semi-synthetic anti-intracellular oncoprotein antibody, a derivatised anti-intracellular oncoprotein antibody or a recombinant anti-intracellular oncoprotein antibody.

Where the anti-intracellular oncoprotein antibody is a non-native anti-intracellular oncoprotein antibody, it may include at least a portion of which has been prepared by recombinant DNA techniques or an anti-intracellular oncoprotein antibody produced by chemical synthesis techniques or combinations thereof.

The term "derivative" as used in this document includes chemical modification of an anti-intracellular oncoprotein antibody. Illustrative of such modifications would be replacement of hydrogen by an alkyl, acyl, or amino group, for example. Thee sequence of the anti-intracellular oncoprotein antibody may be the same as that of the naturally occurring form or it may be a variant, homologue, fragment or derivative thereof.

### ANTIBODY VARIABLE REGIONS

The term "variable region", as used in this document, refers to the variable regions, or domains, of the light chains (VL) and heavy chains (VH) which contain the determinants for binding recognition specificity and for the overall affinity of the antibody against intracellular oncoprotein (or variant, homologue, fragment or derivative), as the case may be.

The variable domains of each pair of light (VL) and heavy chains (VH) are involved in antigen recognition and form the antigen binding site. The domains of the light and heavy chains have the same general structure and each domain has four framework (FR) regions, whose sequences are relatively conserved, connected by three complementarity determining regions (CDRs). The FR regions maintain the structural integrity of the variable domain. The CDRs are the polypeptide segments within the variable domain that mediate binding of the antigen.

The term "constant region", as used in this document, refers to the domains of the light (CL) and heavy (CH) chain of the antibody (or variant, homologue, fragment or derivative) which provide structural stability and other biological functions such as antibody chain association, secretion, transplacental mobility, and complement binding, but which are not involved with binding a intracellular oncoprotein or epitope. The amino acid sequence and corresponding exon sequences in the genes of the constant region will be dependent upon the species from which it is derived. However, variations in the amino acid sequence leading to allotypes are relatively limited for particular constant regions within a species. An "allotype" is an antigenic determinant (or epitope) that distinguishes allelic genes.

The variable region of each chain is joined to the constant region by a linking polypeptide sequence. The linkage sequence is coded by a "J" sequence in the light chain gene, and a combination of a "D" sequence and a "J" sequence in the heavy chain gene.

### PROPHYLACTIC AND THERAPEUTIC METHODS

We disclose methods of treating an abnormal conditions, such as cancer, related to excessive amounts of intracellular oncoprotein expression or activity. Methods of preventing cancer (i.e., prophylaxis) also suitably employ the same or similar approaches.

In general terms, our methods involve manipulation of cancer cells, by modulating (such as down-regulating) the expression, amount or activity of intracellular oncoprotein in the cell. The methods may involve destroying or eradicating cancer cells. The cancer cells may comprise intracellular oncoprotein expressing cancer cells. The cancer cells may be ones which overexpress intracellular oncoprotein, compared to non-cancerous cells. Our methods may comprise exposing a patient to an anti-intracellular oncoprotein antibody,. The anti-intracellular oncoprotein antibody may comprise a humanised anti-intracellular oncoprotein antibody.

The cancer cells may be from intracellular oncoprotein positive cancer patients. Thus, our methods may comprise eradicating intracellular oncoprotein -over-expressing cancer cells from intracellular oncoprotein positive cancer patients.

Our methods may therefore comprise eradicating intracellular oncoprotein over-expressing cells from intracellular oncoprotein-positive cancer patients using anti-intracellular oncoprotein humanised antibodies.

A step of detecting modulated intracellular oncoprotein expression, amount or activity in a cell may be conducted before or after the manipulation step. The detection step may detect upregulated or down-regulated intracellular oncoprotein expression, amount or activity. Any of the methods of modulating or down-regulating intracellular oncoprotein, as described in detail elsewhere in this document, may be used.

In particular, the method may comprise exposing the cell to an anti-intracellular oncoprotein antibody capable of specifically binding to intracellular oncoprotein. In the context of an individual suffering or suspected to be suffering from cancer, the method may comprise administering a therapeutically effective amount of anti-intracellular oncoprotein antibody to the individual. Anti-intracellular oncoprotein antibodies and methods of administering them are described in detail elsewhere in this document.

Furthermore, alternatively or in addition, the method may comprise vaccination with the intracellular oncoprotein, i.e., administering a prophylactically effective amount of intracellular oncoprotein to an individual suffering or suspected to be suffering from cancer.

According to our methods, a cancer cell becomes non-cancerous or the invasive or metastatic cancer cell becomes non-invasive or non-metastatic as a result of the manipulation. The cancer may in particular comprise a cancer such as an invasive or metastatic cancer selected from the group consisting of: colorectal cancer, ovarian cancer, breast cancer, liver cancer, pancreatic cancer, prostate cancer, gastric cancer, lung cancer, penis cancer, cervical cancer, brain cancer, esophageal cancer, bladder carcinoma, kidney renal cell carcinoma, ovary lymphoma and skin melanoma.

As intracellular oncoprotein is associated with aggressiveness and invasiveness of cancer, the level of intracellular oncoprotein may be detected in a cell of an individual with cancer, in a cancer or non-cancer cell, and the aggressiveness of the cancer assessed. A high level of intracellular oncoprotein amount, expression or activity compared with a normal cell indicates an aggressive or invasive cancer, and a stronger or harsher therapy may therefore be required and chosen. Similarly, a lower level may indicate a less aggressive or invasive therapy.

The approaches described here may be used for therapy of any intracellular oncoprotein related disease in general. Intracellular oncoprotein related diseases include proliferative diseases and in particular include cancer. For example, a intracellular oncoprotein related disease may include metastatic cancer, invasive cancer or aggressive cancer.

The methods and compositions described here suitably enable an improvement in a measurable criterion in an individual to whom the treatment is applied, compared to one who has not received the treatment.

For this purpose, a number of criteria may be designated, which reflect the progress of cancer or the well-being of the patient. Useful criteria may include tumour size, tumour dimension, largest dimension of tumour, tumour number, presence of tumour markers (such as alpha-feto protein), degree or number of metastates, etc.

Thus, as an example, a treated individual may show a decrease in tumour size or number as measured by an appropriate assay or test. A treated individual may for example show a 1%, 2%, 3%, 4%, 5%, 6%, 7%, 8%, 9%, 10%, 15%, 20%, 25%, 30%, 40%, 50%, 60%, 70%, 80%, 90%, 100% or more decrease in tumour size of a particular tumour, or decrease in tumour number, or both, compared to an individual who has not been treated.

For example, a intracellular oncoprotein related disease may be defined as being "treated" if a condition associated with the disease is significantly inhibited (*i.e.,* by 50% or more) relative to controls. The inhibition may be by at least 75% relative to controls, such as by 90%, by 95% or 100% relative to controls. The condition may comprise cell proliferation, or it may comprise cell cycle time, cell number, cell migration, cell invasiveness, tumour formation, tumour metastasis, tumour spread, etc. By the term "treatment" we mean to also include prophylaxis or alleviation of cancer.

The term proliferative disorder is used herein in a broad sense to include any disorder that requires control of the cell cycle. In particular, a proliferative disorder includes malignant and pre-neoplastic disorders. The methods and compositions described here are especially useful in relation to treatment or diagnosis of adenocarcinomas such as: small cell lung cancer, and cancer of the kidney, uterus, prostrate, bladder, ovary, colon and breast. For example, malignancies which may be treatable include acute and chronic leukemias, lymphomas, myelomas, sarcomas such as Fibrosarcoma, myxosarcoma, liposarcoma, lymphangioendotheliosarcoma, angiosarcoma, endotheliosarcoma, chondrosarcoma, osteogenic sarcoma, chordoma, lymphangiosarcoma, synovioma, mesothelioma, leimyosarcoma, rhabdomyosarcoma, colon carcinoma, ovarian cancer, prostate cancer, pancreatic cancer, breast cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, choriocarcinoma, renal cell carcinoma, hepatoma, bile duct carcinoma seminoma, embryonal carcinoma, cervical cancer, testicular tumour, lung carcinoma, small cell lung carcinoma, bladder carcinoma, epithelial carcinoma, glioma, astrocytoma, ependymoma, pinealoma, hemangioblastoma, acoustic neuoma, medulloblastoma, craniopharyngioma, oligodendroglioma, menangioma, melanoma, neutroblastoma and retinoblastoma.

The antibody approach to therapy involving use of anti-intracellular oncoprotein antibodies may be combined with other approaches for therapy of such disorders including expression of anti-sense constructs directed against intracellular oncoprotein polynucleotides as described here, and administering them to tumour cells, to inhibit gene function and prevent the tumour cell from growing or progressing.

Anti-sense constructs may be used to inhibit gene function to prevent growth or progression in a proliferative cell. Antisense constructs, *i.e.,* nucleic acid, such as RNA, constructs complementary to the sense nucleic acid or mRNA, are described in detail in US 6,100,090 (Monia et al.), and Neckers et al., 1992, Crit Rev Oncog 3(1-2):175-231, the teachings of which document are specifically incorporated by reference.

In a particular example, cancer may be treated or prevented by reducing the amount, expression or activity of intracellular oncoprotein in whole or in part, for example by siRNAs capable of binding to and destroying intracellular oncoprotein mRNA.

RNA interference (RNAi) is a method of post transcriptional gene silencing (PTGS) induced by the direct introduction of double-stranded RNA (dsRNA) and has emerged as a useful tool to knock out expression of specific genes in a variety of organisms. RNAi is described by Fire et al., Nature 391:806-811 (1998). Other methods of PTGS are known and include, for example, introduction of a transgene or virus. Generally, in PTGS, the transcript of the silenced gene is synthesised but does not accumulate because it is rapidly degraded. Methods for PTGS, including RNAi are described, for example, in the Ambion.com world wide web site, in the directory "/hottopics/", in the "rnai" file.

Suitable methods for RNAi *in vitro* are described herein. One such method involves the introduction of siRNA (small interfering RNA). Current models indicate that these 21-23 nucleotide dsRNAs can induce PTGS. Methods for designing effective siRNAs are described, for example, in the Ambion web site described above. RNA precursors such as Short Hairpin RNAs (shRNAs) can also be encoded by all or a part of the intracellular oncoprotein nucleic acid sequence.

Alternatively, double-stranded (ds) RNA is a powerful way of interfering with gene expression in a range of organisms that has recently been shown to be successful in mammals (Wianny and Zemicka-Goetz, 2000, Nat Cell Biol 2:70-75). Double stranded RNA corresponding to the sequence of a intracellular oncoprotein polynucleotide can be introduced into or expressed in oocytes and cells of a candidate organism to interfere with intracellular oncoprotein activity.

Other methods of modulating intracellular oncoprotein gene expression are known to those skilled in the art and include dominant negative approaches. Again, these may be combined with antibody therapy using anti-intracellular oncoprotein antibodies. Thus, another approach is to use non-functional variants of intracellular oncoprotein polypeptide in this document that compete with the endogenous gene product resulting in inhibition of function.

Intracellular oncoprotein gene expression may also be modulated by as introducing peptides or small molecules which inhibit gene expression or functional activity. Such peptides or small molecules may be administered in combination with anti-intracellular oncoprotein antibodies for the treatment of cancer such as metastatic cancer.

Thus, compounds identified by assays as binding to or modulating, such as down-regulating, the amount, activity or expression of intracellular oncoprotein polypeptide may be administered to tumour or proliferative cells to prevent the function of intracellular oncoprotein polypeptide. Such a compound may be administered along with a pharmaceutically acceptable carrier in an amount effective to down-regulate expression or activity intracellular oncoprotein, or by activating or down-regulating a second signal which controls intracellular oncoprotein expression, activity or amount, and thereby alleviating the abnormal condition.

Alternatively, gene therapy may be employed to control the endogenous production of intracellular oncoprotein by the relevant cells such as cancer cells in the subject. For example, a polynucleotide encoding a intracellular oncoprotein siRNA or a portion of this may be engineered for expression in a replication defective retroviral vector, as discussed below. The retroviral expression construct may then be isolated and introduced into a packaging cell transduced with a retroviral plasmid vector containing RNA encoding an anti-intracellular oncoprotein siRNA such that the packaging cell now produces infectious viral particles containing the sequence of interest. These producer cells may be administered to a subject for engineering cells *in vivo* and regulating expression of the intracellular oncoprotein polypeptide *in vivo.* For overview of gene therapy, see Chapter 20, Gene Therapy and other Molecular Genetic-based Therapeutic Approaches, (and references cited therein) in Human Molecular Genetics, T Strachan and A P Read, BIOS Scientific Publishers Ltd (1996).

In some embodiments, the level of intracellular oncoprotein is decreased in a cancer cell. Furthermore, in such embodiments, treatment may be targeted to, or specific to, such cancer cells. The expression of intracellular oncoprotein may be specifically decreased only in diseased cells (i.e., those cells which are cancerous), and not substantially in other non-diseased cells. In these methods, expression of intracellular oncoprotein may be not substantially reduced in other cells, i.e., cells which are not cancer cells. Thus, in such embodiments, the level of intracellular oncoprotein remains substantially the same or similar in non-cancer cells in the course of or following treatment.

### BIOLOGICAL ACTIVITIES

In some embodiments, the sequences comprise at least one biological activity of an anti-intracellular oncoprotein antibody, as the case may be.

The biological activity may comprise an immunological activity. The anti-intracellular oncoprotein antibody may comprise an identical or similar immunological activity as compared to intracellular oncoprotein antibody or its humanised versions. By "immunological activity" we mean the capability of the anti-intracellular oncoprotein antibody, to induce a specific immune response in appropriate animals or cells on binding with a intracellular oncoprotein antigen.

The biological activity may comprise antigen binding activity. The anti-intracellular oncoprotein antibody may bind to intracellular oncoprotein or an epitope thereof. The anti-intracellular oncoprotein antibody may bind to the antigen or epitope with the same, a reduced or elevated affinity or avidity. For example, the anti-intracellular oncoprotein antibody may bind to the antigen or epitope with at least 10%, such as 20%, such as 30%, 40% 50%, 60%, 70%, 80%, 90% or more, affinity or avidity compared to the cognate antibody, e.g., 269, 223 or 318 or their humanised counterparts, as the case may be.

The activity may include inhibition of cancer activity as for example measured by reduction of tumour size or tumour number, or inhibition of metastatic activity, such as for example measured by the assays described in the Examples. The reduction or inhibition may be conveniently assayed by causing carcinogenesis in a test animal, administering the anti-intracellular oncoprotein antibody to the animal and determining an effect of the anti-intracellular oncoprotein antibody as compared to a similar control animal that has not been so treated. The Examples describe such an assay in detail.

The anti-intracellular oncoprotein antibody may have tumour inhibition or metastasis inhibition activity that is the same as, reduced from, or elevated from, the cognate antibody. For example, the anti-intracellular oncoprotein antibody may be at least 10%, such as 20%, such as 30%, 40% 50%, 60%, 70%, 80%, 90% or more, effective compared to the cognate antibody, e.g., 269, 223 or 318 or their humanised counterparts, as the case may be. By this we mean that, say, if the cognate antibody is capable of reducing tumour number by 90% (see the Examples), the anti-intracellular oncoprotein antibody may be capable of reducing tumour number by 90%, 85%, 80%, 75%, 70%, 65%, 60%, 55%, 50%, 45%, 40%, 35%, 30%, etc, as compared to an untreated animal.

Other assays that detect antibody events can also be used, instead of, or in addition to, the assays described.

### HOMOLOGUES

The anti-intracellular oncoprotein antibody polypeptides disclosed include homologous sequences obtained from any source, for example related viral/bacterial proteins, cellular homologues and synthetic peptides, as well as variants or derivatives thereof. Thus polypeptides also include those encoding homologues of anti-intracellular oncoprotein antibody from other species including animals such as mammals (e.g. mice, rats or rabbits), in particular humans.

In the context of the present document, a homologous sequence or homologue is taken to include an amino acid sequence which is at least 60, 70, 80 or 90% identical, such as at least 95 or 98% identical at the amino acid level over at least 30, such as 50, 70, 90 or 100 amino acids with a relevant polypeptide sequence, for example as shown in the sequence listing herein. In the context of this document, a homologous sequence is taken to include an amino acid sequence which is at least 15, 20, 25, 30, 40, 50, 60, 70, 80 or 90% identical, such as at least 95 or 98% identical at the amino acid level, such as over at least 15, 25, 35, 50 or 100, such as 200, 300,400 or 500 amino acids with the sequence of a relevant polypeptide. Although homology can also be considered in terms of similarity (i.e. amino acid residues having similar chemical properties/functions), in the context of the present document homology may be expressed in terms of sequence identity. The sequence identity may be determined relative to the entirety of the length the relevant sequence, i.e., over the entire length or full length sequence of the relevant gene, for example.

Homology comparisons can be conducted by eye, or more usually, with the aid of readily available sequence comparison programs. These commercially available computer programs can calculate % homology between two or more sequences.

% homology may be calculated over contiguous sequences, i.e. one sequence is aligned with the other sequence and each amino acid in one sequence directly compared with the corresponding amino acid in the other sequence, one residue at a time. This is called an "ungapped" alignment. Typically, such ungapped alignments are performed only over a relatively short number of residues (for example less than 50 contiguous amino acids).

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, the default values may be used when using such software for sequence comparisons. For example when using the GCG Wisconsin Bestfit package (see below) the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, U.S.A.; Devereux et al., 1984, Nucleic Acids Research 12:387). Examples of other software than can perform sequence comparisons include, but are not limited to, the BLAST package (see Ausubel et al., 1999 ibid - Chapter 18), FASTA (Atschul et al., 1990, J. Mol. Biol., 403-410) and the GENEWORKS suite of comparison tools. Both BLAST and FASTA are available for offline and online searching (see Ausubel et al., 1999 ibid, pages 7-58 to 7-60). The GCG Bestfit program may be used.

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). The public default values for the GCG package, or in the case of other software, the default matrix, such as BLOSUM62, may be used.

Once the software has produced an optimal alignment, it is possible to calculate % homology, such as % sequence identity. The software typically does this as part of the sequence comparison and generates a numerical result.

### VARIANTS AND DERIVATIVES

The terms "variant" or "derivative" in relation to the amino acid sequences as described here includes any substitution of, variation of, modification of, replacement of, deletion of or addition of one (or more) amino acids from or to the sequence. The resultant amino acid sequence may retain substantially the same activity as the unmodified sequence, such as having at least the same activity as the anti-intracellular oncoprotein antibody polypeptides shown in this document, for example in the sequence listings. Thus, the key feature of the sequences - namely ability to bind to intracellular oncoprotein polypeptides or tumour reduction activity, as described elsewhere - may be retained.

Polypeptides having the amino acid sequence shown in the Examples, or fragments or homologues thereof may be modified for use in the methods and compositions described here. Typically, modifications are made that maintain the biological activity of the sequence. Amino acid substitutions may be made, for example from 1, 2 or 3 to 10, 20 or 30 substitutions provided that the modified sequence retains the biological activity of the unmodified sequence. Amino acid substitutions may include the use of non-naturally occurring analogues, for example to increase blood plasma half-life of a therapeutically administered polypeptide.

Natural variants of anti-intracellular oncoprotein antibodies are likely to comprise conservative amino acid substitutions. Conservative substitutions may be defined, for example according to the Table below. Amino acids in the same block in the second column such as those in the same line in the third column may be substituted for each other:

| | | | |
|---|---|---|---|
| | ALIPHATIC | Non-polar | G A P |
| | | | |
| | | | I L V |
| | | Polar - uncharged | C S T M |
| | | | N Q |
| | | Polar - charged | D E |
| | | | K R |
| | AROMATIC | | H F W Y |
| | | | |

### FRAGMENTS

Polypeptides disclosed here and useful as markers also include fragments of the above mentioned full length polypeptides and variants thereof, including fragments of the sequences set out in the sequence listings.

Polypeptides also include fragments of the full length sequence of any of the anti-PRL antibody polypeptides. Fragments may comprise at least one epitope. Methods of identifying epitopes are well known in the art. Fragments will typically comprise at least 6 amino acids, such as at least 10, 20, 30, 50 or 100 or more amino acids.

Polypeptide fragments of the anti-intracellular oncoprotein antibody proteins and allelic and species variants thereof may contain one or more (e.g. 5, 10, 15, or 20) substitutions, deletions or insertions, including conserved substitutions. Where substitutions, deletion and/or insertions occur, for example in different species, such as less than 50%, 40% or 20% of the amino acid residues depicted in the sequence listings are altered.

Anti-intracellular oncoprotein antibody and their fragments, homologues, variants and derivatives, may be made by recombinant means. However, they may also be made by synthetic means using techniques well known to skilled persons such as solid phase synthesis. The proteins may also be produced as fusion proteins, for example to aid in extraction and purification. Examples of fusion protein partners include glutathione-S-transferase (GST), 6xHis, GAL4 (DNA binding and/or transcriptional activation domains) and β-galactosidase. It may also be convenient to include a proteolytic cleavage site between the fusion protein partner and the protein sequence of interest to allow removal of fusion protein sequences. The fusion protein may be such that it will not hinder the function of the protein of interest sequence. Proteins may also be obtained by purification of cell extracts from animal cells.

The anti-intracellular oncoprotein antibody polypeptides, variants, homologues, fragments and derivatives disclosed here may be in a substantially isolated form. It will be understood that such polypeptides may be mixed with carriers or diluents which will not interfere with the intended purpose of the protein and still be regarded as substantially isolated. A anti-intracellular oncoprotein antibody variant, homologue, fragment or derivative may also be in a substantially purified form, in which case it will generally comprise the protein in a preparation in which more than 90%, e.g. 95%, 98% or 99% of the protein in the preparation is a protein.

The anti-intracellular oncoprotein antibody polypeptides, variants, homologues, fragments and derivatives disclosed here may be labelled with a revealing label. The revealing label may be any suitable label which allows the polypeptide , etc to be detected. Suitable labels include radioisotopes, e.g. ¹²⁵I, enzymes, antibodies, polynucleotides and linkers such as biotin. Labelled polypeptides may be used in diagnostic procedures such as immunoassays to determine the amount of a polypeptide in a sample. Polypeptides or labelled polypeptides may also be used in serological or cell-mediated immune assays for the detection of immune reactivity to said polypeptides in animals and humans using standard protocols.

The anti-intracellular oncoprotein antibody polypeptides, variants, homologues, fragments and derivatives disclosed here, optionally labelled, my also be fixed to a solid phase, for example the surface of an immunoassay well or dipstick. Such labelled and/or immobilised polypeptides may be packaged into kits in a suitable container along with suitable reagents, controls, instructions and the like. Such polypeptides and kits may be used in methods of detection of antibodies to the polypeptides or their allelic or species variants by immunoassay.

Immunoassay methods are well known in the art and will generally comprise: (a) providing a polypeptide comprising an epitope bindable by an antibody against said protein; (b) incubating a biological sample with said polypeptide under conditions which allow for the formation of an antibody-antigen complex; and (c) determining whether antibody-antigen complex comprising said polypeptide is formed.

The anti-intracellular oncoprotein antibody polypeptides, variants, homologues, fragments and derivatives disclosed here may be used in *in vitro* or *in vivo* cell culture systems to study the role of their corresponding genes and homologues thereof in cell function, including their function in disease. For example, truncated or modified polypeptides may be introduced into a cell to disrupt the normal functions which occur in the cell. The polypeptides may be introduced into the cell *by in situ* expression of the polypeptide from a recombinant expression vector (see below). The expression vector optionally carries an inducible promoter to control the expression of the polypeptide.

The use of appropriate host cells, such as insect cells or mammalian cells, is expected to provide for such post-translational modifications (e.g. myristolation, glycosylation, truncation, lapidation and tyrosine, serine or threonine phosphorylation) as may be needed to confer optimal biological activity on recombinant expression products. Such cell culture systems in which the anti-intracellular oncoprotein antibody polypeptides, variants, homologues, fragments and derivatives disclosed here are expressed may be used in assay systems to identify candidate substances which interfere with or enhance the functions of the polypeptides in the cell.

### POLYNUCLEOTIDE SEQUENCES

The variable regions, monoclonal antibody sequences and humanised antibody sequences may comprise polynucleotides. These may comprise DNA or RNA.

They may be single-stranded or double-stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of the present document, it is to be understood that the polynucleotides described herein may be modified by any method available in the art. Such modifications may be carried out in order to enhance the *in vivo* activity or life span of polynucleotides.

Where the polynucleotide is double-stranded, both strands of the duplex, either individually or in combination, are encompassed by the methods and compositions described here. Where the polynucleotide is single-stranded, it is to be understood that the complementary sequence of that polynucleotide is also included.

### VARIANTS, DERIVATIVES AND HOMOLOGUES

The terms "variant", "homologue" or "derivative" in relation to a nucleotide sequence described in this document include any substitution of, variation of, modification of, replacement of, deletion of or addition of one (or more) nucleotides from or to the sequence. The resulting sequence may be capable of encoding a polypeptide which has intracellular oncoprotein binding activity as described elsewhere in this document.

As indicated above, with respect to sequence identity, a "homologue" has such as at least 5% identity, at least 10% identity, at least 15% identity, at least 20% identity, at least 25% identity, at least 30% identity, at least 35% identity, at least 40% identity, at least 45% identity, at least 50% identity, at least 55% identity, at least 60% identity, at least 65% identity, at least 70% identity, at least 75% identity, at least 80% identity, at least 85% identity, at least 90% identity, or at least 95% identity to a relevant sequence.

There may be at least 95% identity, such as at least 96% identity, such as at least 97% identity, such as at least 98% identity, such as at least 99% identity. Nucleotide homology comparisons may be conducted as described above. A sequence comparison program such as the GCG Wisconsin Bestfit program described above may be used for this purpose. The default scoring matrix has a match value of 10 for each identical nucleotide and -9 for each mismatch. The default gap creation penalty is -50 and the default gap extension penalty is -3 for each nucleotide.

### HYBRIDISATION

We further describe nucleotide sequences that are capable of hybridising selectively to any of the sequences presented herein, such as 269, 223 and 318 variable region, antibody and humanised antibody or any variant, fragment or derivative thereof, or to the complement of any of the above. Nucleotide sequences may be at least 15 nucleotides in length, such as at least 20, 30, 40 or 50 nucleotides in length.

The term "hybridisation" as used herein shall include "the process by which a strand of nucleic acid joins with a complementary strand through base pairing" as well as the process of amplification as carried out in polymerase chain reaction technologies.

Polynucleotides capable of selectively hybridising to the nucleotide sequences presented herein, or to their complement, will be generally at least 70%, such as at least 80 or 90% and such as at least 95% or 98% homologous to the corresponding nucleotide sequences presented herein over a region of at least 20, such as at least 25 or 30, for instance at least 40, 60 or 100 or more contiguous nucleotides.

The term "selectively hybridisable" means that the polynucleotide used as a probe is used under conditions where a target polynucleotide is found to hybridize to the probe at a level significantly above background. The background hybridization may occur because of other polynucleotides present, for example, in the cDNA or genomic DNA library being screened. In this event, background implies a level of signal generated by interaction between the probe and a nonspecific DNA member of the library which is less than 10 fold, such as less than 100 fold as intense as the specific interaction observed with the target DNA. The intensity of interaction may be measured, for example, by radiolabelling the probe, e.g. with ³²P.

Hybridisation conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CA), and confer a defined "stringency" as explained below.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridisation can be used to identify or detect identical polynucleotide sequences while an intermediate (or low) stringency hybridisation can be used to identify or detect similar or related polynucleotide sequences.

We disclose nucleotide sequences that can hybridise to a nucleic acid, or a fragment, homologue, variant or derivative thereof, under stringent conditions (e.g. 65°C and 0.1xSSC {1xSSC = 0.15 M NaCl, 0.015 M Na₃ Citrate pH 7.0}).

Where a polynucleotide is double-stranded, both strands of the duplex, either individually or in combination, are encompassed by the present disclosure. Where the polynucleotide is single-stranded, it is to be understood that the complementary sequence of that polynucleotide is also disclosed and encompassed.

Polynucleotides which are not 100% homologous to the sequences disclosed here but fall within the disclosure can be obtained in a number of ways. Other variants of the sequences described herein may be obtained for example by probing DNA libraries made from a range of individuals, for example individuals from different populations. In addition, other viral/bacterial, or cellular homologues particularly cellular homologues found in mammalian cells (e.g. rat, mouse, bovine and primate cells), may be obtained and such homologues and fragments thereof in general will be capable of selectively hybridising to the sequences shown in the sequence listing herein. Such sequences may be obtained by probing cDNA libraries made from or genomic DNA libraries from other animal species, and probing such libraries with probes comprising all or part of the disclosed sequences under conditions of medium to high stringency.

The polynucleotides described here may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 15, such as at least 20, for example at least 25, 30 or 40 nucleotides in length, and are also encompassed by the term polynucleotides as used herein. Fragments may be less than 500, 200, 100, 50 or 20 nucleotides in length.

Polynucleotides such as a DNA polynucleotides and probes may be produced recombinantly, synthetically, or by any means available to those of skill in the art. They may also be cloned by standard techniques.

In general, primers will be produced by synthetic means, involving a step wise manufacture of the desired nucleic acid sequence one nucleotide at a time. Techniques for accomplishing this using automated techniques are readily available in the art.

Longer polynucleotides will generally be produced using recombinant means, for example using PCR (polymerase chain reaction) cloning techniques. This will involve making a pair of primers (e.g. of about 15 to 30 nucleotides) flanking a region of the sequence which it is desired to clone, bringing the primers into contact with mRNA or cDNA obtained from an animal or human cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector.

### INTRACELLULAR ONCOPROTEIN POLYPEPTIDES AND NUCLEIC ACIDS

Intracellular oncoprotein polypeptide homologues, variants, derivatives and fragments may be defined similarly, as set out in the previous paragraphs.

Where the context permits, a reference to intracellular oncoprotein polypeptide should be taken to include reference to an intracellular oncoprotein polypeptide homologue, variant, derivative or fragment. Similarly, a reference to intracellular oncoprotein polypeptide should be taken to include reference to a intracellular oncoprotein polypeptide homologue, variant, derivative or fragment.

Similarly, where the context permits, a reference to intracellular oncoprotein nucleic acid should be taken to include reference to a intracellular oncoprotein nucleic acid homologue, variant, derivative or fragment. Similarly, a reference to intracellular oncoprotein polypeptide should be taken to include reference to a intracellular oncoprotein nucleic acid homologue, variant, derivative or fragment.

### ANTI-INTRACELLULAR ONCOPROTEIN ANTIBODY PRODUCTION

The anti-intracellular oncoprotein antibody can be produced by recombinant DNA methods or synthetic peptide chemical methods that are well known to those of ordinary skill in the art.

By way of example, the anti-intracellular oncoprotein antibody may be synthesized by techniques well known in the art, as exemplified by "Solid Phase Peptide Synthesis: A Practical Approach" E. Atherton and R. C. Sheppard, IRL Press, Oxford England. Similarly, multiple fragments can be synthesized which are subsequently linked together to form larger fragments. These synthetic peptide fragments can also be made with amino acid substitutions at specific locations in order to test for activity *in vitro and in vivo.*

The anti-intracellular oncoprotein antibody can be synthesized in a standard microchemical facility and purity checked with HPLC and mass spectrophotometry. Methods of peptide synthesis, HPLC purification and mass spectrophotometry are commonly known to those skilled in these arts.

The anti-intracellular oncoprotein antibody may also be expressed under *in vitro* and *in vivo* conditions in a transformed host cell into which has been incorporated the DNA sequences described here (such as variable sequences) or allelic variations thereof and which can be used in the prevention and/or treatment of cancer related diseases.

The term "vector" includes expression vectors and transformation vectors. The term "expression vector" means a construct capable of *in vivo* or *in vitro* expression. The term "transformation vector" means a construct capable of being transferred from one species to another.

Vectors which may be used for expression include recombinant viral vectors, in particular recombinant retroviral vectors (RRV) such as lentiviral vectors, adenoviral vectors including a combination of retroviral vectors.

The term 'recombinant retroviral vector" (RRV) refers to a vector with sufficient retroviral genetic information to allow packaging of an RNA genome, in the presence of packaging components, into a viral particle capable of infecting a target cell. Infection of the target cell includes reverse transcription and integration into the target cell genome. The RRV carries non-viral coding sequences which are to be delivered by the vector to the target cell. An RRV is incapable of independent replication to produce infectious retroviral particles within the final target cell. Usually the RRV lacks a functional gag pol and/or env gene and/or other genes essential for replication. Vectors which may be used include recombinant pox viral vectors such as fowl pox virus (FPV), entomopox virus, vaccinia virus such as NYVAC, canarypox virus, MVA or other non-replicating viral vector systems such as those described for example in WO9530018.

Pox viruses may be engineered for recombinant gene expression and for the use as recombinant live vaccines in a dual immunotherapeutic approach. The principal rationale for using live attenuated viruses, such as viruses, as delivery vehicles and/or vector based vaccine candidates, stems from their ability to elicit cell mediated immune responses. The viral vectors, as outlined above, are capable of being employed as delivery vehicles and as vector based vaccine candidates because of the immunogenicity of their constitutive proteins, which act as adjuvants to enhance the immune response, thus rendering a nucleotide sequence of interest (NOI) such as a nucleotide sequence encoding an anti-intracellular oncoprotein antibody more immunogenic.

The pox virus vaccination strategies have used recombinant techniques to introduce NOIs into the genome of the pox virus. If the NOI is integrated at a site in the viral DNA which is non-essential for the life cycle of the virus, it is possible for the newly produced recombinant pox virus to be infectious, that is to say to infect foreign cells and thus to express the integrated NOI. The recombinant pox virus prepared in this way can be used as live vaccines for the prophylaxis and/or treatment of pathologic and infectious disease and/or cancer.

Other requirements for pox viral vector delivery systems include good immunogenicity and safety. MVA is a replication-impaired vaccinia strain with a good safety record. In most cell types and normal human tissue, MVA does not replicate. Limited replication of MVA is observed in a few transformed cell types such as BHK21 cells. Carroll et al (1997 Vaccine15: 387-394) have shown that the recombinant MVA is equally as good as conventional recombinant vaccinia vectors at generating a protective CD8+T cell response and is an efficacious alternative to the more commonly used replication competent vaccinia virus. The vaccinia virus strains derived from MVA, or independently developed strains having the features of MVA which make MVA particularly suitable for use in a vaccine, are also suitable for use as a delivery vehicle.

The nucleotide sequence of interest, and of which expression is desired, may operably linked to a transcription unit. The term "transcription unit" as described herein are regions of nucleic acid containing coding sequences and the signals for achieving expression of those coding sequences independently of any other coding sequences. Thus, each transcription unit generally comprises at least a promoter, an optional enhancer and a polyadenylation signal. The term "promoter" is used in the normal sense of the art, e. g. an RNA polymerase binding site. The promoter may contain an enhancer element. The term "enhancer" includes a DNA sequence which binds to other protein components of the transcription initiation complex and thus facilitates the initiation of transcription directed by its associated promoter. The term "cell" includes any suitable organism. The cell may comprise a mammalian cell, such as a human cell.

The term "transformed cell" means a cell having a modified genetic structure. For example, as described here, a cell has a modified genetic structure when a vector such as an expression vector has been introduced into the cell. The term "organism" includes any suitable organism. The organism may comprise a mammal such as a human.

Here the term "transgenic organism" means an organism comprising a modified genetic structure. For example, the organism may have a modified genetic structure if a vector such as an expression vector has been introduced into the organism.

### ANTIBODY EXPRESSION

We further describe a method comprising transforming a host cell with a or the nucleotide sequences described in this document, antibody sequences or humanized antibody sequences.

We also provide a method comprising culturing a transformed host cell-which cell has been transformed with a or the such nucleotide sequences under conditions suitable for the expression of the anti-intracellular oncoprotein antibody encoded by said nucleotide sequences.

We further provide a method comprising culturing a transformed host cell-which cell has been transformed with a or the such nucleotide sequences under conditions suitable for the expression of the anti-intracellular oncoprotein antibody encoded by said nucleotide sequences; and then recovering said anti-intracellular oncoprotein antibody from the transformed host cell culture.

Thus, anti-intracellular oncoprotein antibody encoding nucleotide sequences, fusion proteins or functional equivalents thereof, may be used to generate recombinant DNA molecules that direct the expression thereof in appropriate host cells.

By way of example, anti-intracellular oncoprotein antibody may be produced in recombinant E. coli, yeast or mammalian expression systems, and purified with column chromatography.

In certain circumstances there are advantages of using antibody fragments, rather than whole antibodies. The smaller size of the fragments allows for rapid clearance, and may lead to improve tumour to non-tumour ratios. Fab, Fv, ScFv antibody fragments can all be expressed in and secreted from *E. coli,* thus allowing the production of large amounts of the such fragments.

The nucleotide sequences encoding the anti-intracellular oncoprotein antibody may be operably linked to a promoter sequence capable of directing expression of the anti-intracellular oncoprotein antibody encoding nucleotide sequences in a suitable host cell. When inserted into the host cell, the transformed host cell may be cultured under suitable conditions until sufficient levels of the anti-intracellular oncoprotein antibody are achieved after which the cells may be lysed and the anti-intracellular oncoprotein antibody is isolated.

Host cells transformed with the anti-intracellular oncoprotein antibody encoding nucleotide sequences may be cultured under conditions suitable for the expression and recovery of the anti-intracellular oncoprotein antibody from cell culture. The protein produced by a recombinant cell may be secreted or may be contained intracellularly depending on the sequence and/or the vector used. As will be understood by those of skill in the art, expression vectors containing the

Anti-intracellular oncoprotein antibody encoding nucleotide sequences can be designed with signal sequences which direct secretion of the anti-intracellular oncoprotein antibody encoding nucleotide sequences through a particular prokaryotic or eukaryotic cell membrane. Other recombinant constructions may join the anti-intracellular oncoprotein antibody encoding nucleotide sequence to a nucleotide sequence encoding a polypeptide domain which will facilitate purification of soluble proteins (Kroll DJ et al(1993) DNA Cell Biol 12:441- 5 3', see also the discussion below on vectors containing fusion proteins).

The anti-intracellular oncoprotein antibody may also be expressed as a recombinant protein with one or more additional polypeptide domains added to facilitate protein purification. Such purification facilitating domains include, but are not limited to, metal chelating peptides such as histidine-tryptophan modules that allow purification on immobilized metals (Porath J (1992) Protein Expr Purif 3-26328 1), protein A domains that allow purification on immobilized immunoglobulin, and the domain utilized in the FLAGS extension/affinity purification system (Immunex Corp, Seattle, WA). The inclusion of a cleavable linker sequence such as Factor XA or enterokinase (Invitrogen, San Diego, CA) between the purification domain and the anti-intracellular oncoprotein antibody is useful to facilitate purification.

The nucleotide sequences described here may be engineered in order to alter a the anti-intracellular oncoprotein antibody encoding sequences for a variety of reasons, including but not limited to alterations which modify the cloning, processing and/or expression of the gene product. For example, mutations may be introduced using techniques which are well known in the art, e.g., site-directed mutagenesis to insert new restriction sites, to alter glycosylation patterns or to change codon preference.

In another embodiment, a or the natural, modified or recombinant anti-intracellular oncoprotein antibody encoding nucleotide sequences may be ligated to a heterologous sequence to encode a fusion protein. By way of example, fusion proteins comprising the anti-intracellular oncoprotein antibody or an enzymatically active fragment or derivative thereof linked to an affinity tag such as glutathione-S-transferase (GST), biotin, His6, ac-myc tag (see Emrich etal 1993 BiocemBiophys Res Commun 197(1): 21220), hemagglutinin (HA) (as described in Wilson et al (1984 Cell 37 767) or a FLAG epitope (Ford etal 1991 Protein Expr Purif Apr; 2 (2):95-107). May be produced

The fused recombinant protein may comprise an antigenic coprotein such as GST, beta-galactosidase or the lipoprotein D from *Haemophillls influenzae* which are relatively large co-proteins, which solubilise and facilitate production and purification thereof. Alternatively, the fused protein may comprise a carrier protein such as bovine serum albumin (BSA) or keyhole limpet haemocyanin (KLH). In certain embodiments, the marker sequence may comprise a hexa-histidine peptide, as provided in the pQE vector (Qiagen Inc) and described in Gentz et al (1989 PNAS 86: 821-824). Such fusion proteins are readily expressable in yeast culture (as described in Mitchell et al 1993 Yeast 5:715-723) and are easily purified by affinity chromatography. A fusion protein may also be engineered to contain a cleavage site located between the nucleotide sequence encoding the anti-intracellular oncoprotein antibody and the heterologous protein sequence, so that the anti-intracellular oncoprotein antibody may be cleaved and purified away from the heterologous moiety. In another embodiment, an assay for the target protein may be conducted using the entire, bound fusion protein. Alternatively, the co-protein may act as an adjuvant in the sense of providing a generalised stimulation of the immune system. The co-protein may be attached to either the amino or carboxy terminus of the first protein.

Although the presence/absence of marker gene expression suggests that the nucleotide sequence for anti-intracellular oncoprotein antibody is also present, its presence and expression should be confirmed. For example, if the anti-intracellular oncoprotein antibody encoding nucleotide sequence is inserted within a marker gene sequence, recombinant cells containing the anti-intracellular oncoprotein antibody coding regions may be identified by the absence of the marker gene function. Alternatively, a marker gene may be placed in tandem with a anti-intracellular oncoprotein antibody encoding nucleotide sequence under the control of a single promoter.

Expression of the marker gene in response to induction or selection usually indicates expression of the anti-intracellular oncoprotein antibody as well.

Additional methods to quantitate the expression of a particular molecule include radiolabeling (Melby PC etal 1993 J lmmunol Methods 159:235-44) or biotinylating (Duplaa C et al 1993 Anal Biochem229-36) nucleotides, co amplification of a control nucleic acid. and standard curves onto which the experimental results are interpolated.

Quantitation of multiple samples may be speeded up by running the assay in an ELISA format where the anti-intracellular oncoprotein antibody of interest is presented in various dilutions and a spectrophotometric or calorimetric response gives rapid quantitation.

Altered anti-intracellular oncoprotein antibody nucleotide sequences which may be made or used include deletions, insertions or substitutions of different nucleotide residues resulting in a nucleotide sequence that encodes the same or a functionally equivalent anti-intracellular oncoprotein antibody. By way of example, the expressed anti-intracellular oncoprotein antibody may also have deletions, insertions or substitutions of amino acid residues which produce a silent change and result in a functionally equivalent anti-intracellular oncoprotein antibody. Deliberate amino acid substitutions may be made on the basis of similarity in polarity, charge. solubility, hydrophobicity, hydrophilicity. and/or the amphipathic nature of the residues as long as the binding affinity of the anti-intracellular oncoprotein antibody is retained. For example, negatively charged amino acids include aspartic acid and glutamic acid: positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tvrosine.

Gene therapy whereby the anti-intracellular oncoprotein antibody encoding nucleotide sequences as described here is regulated *in vivo* may also be employed. For example, expression regulation may be accomplished by administering compounds that bind to the anti-intracellular oncoprotein antibody encoding nucleotide sequences, or control regions associated with the anti-intracellular oncoprotein antibody encoding nucleotide sequence or its corresponding RNA transcript to modify the rate of transcription or translation.

By way of example, the anti-intracellular oncoprotein antibody encoding nucleotide sequences described here may be under the expression control of an expression regulatory element, usually a promoter or a promoter and enhancer. The enhancer and/or promoter may be preferentially active in a hypoxic or ischaemic or low glucose environment, such that the anti-intracellular oncoprotein antibody encoding nucleotide sequences is preferentially expressed in the particular tissues of interest, such as in the environment of a tumour cell or mass. Thus, any significant biological effect or deleterious effect of the anti-intracellular oncoprotein antibody encoding nucleotide sequences on the individual being treated may be reduced or eliminated. The enhancer element or other elements conferring regulated expression may be present in multiple copies.

The promoter and/or enhancer may be constitutively efficient, or may be tissue or temporally restricted in their activity. Examples of suitable tissue restricted promoters/enhancers are those which are highly active in tumour cells such as a promoter/enhancer from a MUC1 gene, a CEA gene or a STV antigen gene. Examples of temporally restricted promoters/enhancers are those which are responsive to ischaemia and/or hypoxia, such as hypoxia response elements or the promoter/enhancer of agrp78 or agrp94 gene. The alpha fetoprotein (AFP) promoter is also a tumour-specific promoter. Another promoter-enhancer combination is a human cytomegalovirus (hCMV) major immediate early (MIE) promoter/enhancer combination.

The promoters may be tissue specific. That is, they may be capable of driving transcription of a anti-intracellular oncoprotein antibody encoding nucleotide sequences in one tissue while remaining largely "silent" in other tissue types.

The term "tissue specific" means a promoter which is not restricted in activity to a single tissue type but which nevertheless shows selectivity in that they may be active in one group of tissues and less active or silent in another group. A desirable characteristic of such promoters is that they possess a relatively low activity in the absence of activated hypoxia-regulated enhancer elements, even in the target tissue. One means of achieving this is to use "silencer" elements which suppress the activity of a selected promoter in the absence of hypoxia.

The term "hypoxia" means a condition under which a particular organ or tissue receives an inadequate supply of oxygen.

The level of expression of a or the anti-intracellular oncoprotein antibody encoding nucleotide sequences under the control of a particular promoter may be modulated by manipulating the promoter region. For example, different domains within a promoter region may possess different gene regulatory activities. The roles of these different regions are typically assessed using vector constructs having different variants of the promoter with specific regions deleted (that is, deletion analysis). This approach may be used to identify, for example, the smallest region capable of conferring tissue specificity or the smallest region conferring hypoxia sensitivity.

A number of tissue specific promoters, described above, may be used. In most instances, these promoters may be isolated as convenient restriction digestion fragments suitable for cloning in a selected vector. Alternatively, promoter fragments may be isolated using the polymerase chain reaction. Cloning of the amplified fragments may be facilitated by incorporating restriction sites at the 5' end of the primers.

### COMBINATION THERAPY

The methods and compositions described here, including anti-intracellular oncoprotein antibodies and intracellular oncoprotein vaccines, may be used in combination with other compositions and procedures for the treatment of diseases.

By way of example, the anti-intracellular oncoprotein antibodies and intracellular oncoprotein vaccines may also be used in combination with conventional treatments of diseases such as cancer. For example, a tumor may be treated conventionally with surgery, radiation or chemotherapy combined with anti-intracellular oncoprotein antibody and/or intracellular oncoprotein vaccine may be subsequently administered to the patient to extend the dormancy of micrometastases and to stabilize any residual primary tumor.

The anti-intracellular oncoprotein antibody and/or intracellular oncoprotein vaccine can be delivered with a therapeutically effective agent at the same moment in time and at the same site. Alternatively, the anti-intracellular oncoprotein antibody and/or intracellular oncoprotein vaccine and the therapeutically effective agent may be delivered at a different time and to a different site. The anti-intracellular oncoprotein antibody and/or intracellular oncoprotein vaccine and the therapeutically effective agent may even be delivered in the same delivery vehicle for the prevention and/or treatment of cancer.

Anti-intracellular oncoprotein antibody and/or intracellular oncoprotein vaccine may be used in combination with cytotoxic agents for the prevention and/or treatment of angiogenesis and/or cancer. Cytotoxic agents such as ricin, linked to anti-intracellular oncoprotein antibodies, anti-intracellular oncoprotein antibodies antisera, anti-intracellular oncoprotein antibodies receptor agonists and antagonists provide a tool for the destruction of cells that express intracellular oncoprotein or intracellular oncoprotein. These cells may be found in many locations, including but not limited to, micrometastases and primary tumours.

Anti-intracellular oncoprotein antibody and/or intracellular oncoprotein vaccine may be used in combination with a pro-drug activating enzyme in gene therapy. Instead of or as well as being selectively expressed in target tissues, the anti-intracellular oncoprotein antibody and/or intracellular oncoprotein vaccine may be used in combination with another molecule, such as a pro-drug activation enzyme or enzymes which have no significant effect or no deleterious effect until the individual is treated with one or more pro-drugs upon which the enzyme or enzymes act. In the presence of the pro-drug activation enzyme, active treatment of an individual with the appropriate pro-drug leads to enhanced reduction in tumour growth or survival.

A pro-drug activating enzyme may be delivered to a tumour site for the treatment of a cancer. In each case, a suitable pro-drug is used in the treatment of the patient in combination with the appropriate pro-drug activating enzyme. An appropriate pro-drug is administered in conjunction with the vector. Examples of pro-drugs include: etoposide phosphate (with alkaline phosphatase, Senter et al 1988 Proc Natl Acad Sci 85: 48424846); 5-fluorocytosine (with cytosine deaminase, Mullen et al 1994 Cancer Res 54: 1503-1506); Doxorubicin-N -p-hydroxyphenoxyacetamide (with Penicillin- V -Amidase, Kerr et al 1990 Cancer Immunol Immunother 31: 202-206); Para-N-bis(2-chloroethyl) aminobenzoyl glutamate (with carboxypeptidase G2); Cephalosporin nitrogen mustard carbamates (with beta-lactamase); SR4233 (with P450 Reductase); Ganciclovir (with HSV thymidine kinase, Borrelli et al 1988 Proc Natl Acad Sci 85: 7572-7576); mustard pro- drugs with nitro reductase (Friedlos el al 1997 J Med Chem 40: 1270-1275) and Cyclophosphamide (with P450 Chen et a/1996 Cancer Res 56: 1331-1340).

Examples of pro-drug activation enzymes include a thymidine phosphorylase which activates the 5-fluoro-uracil pro-drugs capcetabine and furtulon; thymidine kinase from Herpes Simplex Virus which activates ganciclovir; a cytochrome P450 which activates a pro-drug such as cyclophosphamide to a DNA damaging agent; and cytosine deaminase which activates 5-fluorocytosine. An enzyme of human origin may be used.

Other suitable molecules include those that are of therapeutic and/or diagnostic application such as, but are not limited to: sequences encoding cytokines, chemokines, hormones, antibodies, engineered immunoglobulin-like molecules, a single chain antibody, fusion proteins, enzymes, immune co-stimulatory molecules, immunomodulatory molecules, anti-sense RNA, a transdominant negative mutant of a target protein, a toxin, a conditional toxin, an antigen, a tumour suppressor protein and growth factors, membrane proteins, vasoactive proteins and peptides, anti-viral proteins and ribozymes, and derivatives thereof (such as with an associated reporter group). When included, such coding sequences may be typically operatively linked to a suitable promoter, which may be a promoter driving expression of a ribozyme(s), or a different promoter or promoters, such as in one or more specific cell types.

The molecules may be proteins which are secreted from the cell. Alternatively the molecules are not secreted and are active within the cell. In either event, the molecules may demonstrate a bystander effector or a distant bystander effect; that is the production of the expression product in one cell leading to the killing of additional, related cells, either neighbouring or distant (e.g. metastatic), which possess a common phenotype.

Suitable molecules for use in the treatment or prophylaxis of cancer include proteins (or nucleic acids encoding proteins) which: destroy the target cell (for example a ribosomal toxin), act as: tumour suppressors (such as wild-type p53); activators of anti-tumour immune mechanisms (such as cytokines, co-stimulatory molecules and immunoglobulins); inhibitors of angiogenesis; or which provide enhanced drug sensitivity (such as pro-drug activation enzymes); indirectly stimulate destruction of target cell by natural effector cells (for example, strong antigen to stimulate the immune system or convert a precursor substance to a toxic substance which destroys the target cell (for example a prodrug activating enzyme). Encoded proteins could also destroy bystander tumour cells (for example with secreted antitumour antibody-ribosomal toxin fusion protein), indirectly stimulate destruction of bystander tumour cells (for example cytokines to stimulate the immune system or procoagulant proteins causing local vascular occlusion) or convert a precursor substance to a toxic substance which destroys bystander tumour cells (eg an enzyme which activates a prodrug to a diffusible drug).

Antisense transcripts or ribozymes which interfere with expression of cellular genes for tumour persistence (for example against aberrant myc transcripts in Burkitts lymphoma or against bcr-abl transcripts in chronic myeloid leukemia) may be delivered to enhance cancer cell killing function or metastasis preventing function of the anti-intracellular oncoprotein antibody and/or intracellular oncoprotein vaccine. The use of combinations of such molecules is also envisaged.

Examples of hypoxia regulatable therapeutic molecules can be found in PCT/GB95/00322 (WO-A-9521927).

### ANTI-INTRACELLULAR ONCOPROTEIN ANTIBODY CONJUGATES

The targeting of cells expressing intracellular oncoprotein antigen with the anti-intracellular oncoprotein antibodies described here facilitates the development of drugs to modulate the activity of cells expressing intracellular oncoprotein.

Different anti-intracellular oncoprotein antibodies can be synthesized for use in several applications including but not limited to the linkage of an anti-intracellular oncoprotein antibody to cytotoxic agents for targeted killing of cells that bind the anti-intracellular oncoprotein antibody.

The anti-intracellular oncoprotein antibody described here can be coupled to other molecules using standard methods. The amino and carboxyl termini of the anti-intracellular oncoprotein antibody may be isotopically and nonisotopically labeled with many techniques, for example radiolabeling using conventional techniques (tyrosine residues- chloramine T. iodogen. lactoperoxidase; lysine residues- Bolton-Hunter reagent). These coupling techniques are well known to those skilled in the art. The coupling technique is chosen on the basis of the functional groups available on the amino acids including, but not limited to amino, sulfhydral, carboxyl, amide, phenol, and imidazole. Various reagents used to effect these couplings include among others, glutaraldehyde, diazotized benzidine, carbodiimide, and p- benzoquinone.

The anti-intracellular oncoprotein antibodies may be chemically coupled to isotopes, enzymes, carrier proteins, cytotoxic agents, fluorescent molecules and other compounds for a variety of applications. The efficiency of the coupling reaction is determined using different techniques appropriate for the specific reaction. For example, radiolabeling of an intracellular oncoprotein polypeptide with ¹²⁵I is accomplished using chloramine T and Nal ²⁵I of high specific activity, The reaction is terminated with sodium metabisulfite and the mixture is desalted on disposable columns. The labeled antibody is eluted from the column and fractions are collected. Aliquots are removed from each fraction and radioactivity measured in a gamma counter. In this manner, the unreacted Na¹²⁵I is separated from the labeled intracellular oncoprotein polypeptide. The peptide fractions with the highest specific radioactivity are stored for subsequent use such as analysis of the ability to bind to a anti-intracellular oncoprotein antibody.

The use of labelled anti-intracellular oncoprotein antibodies with short lived isotopes enables visualization quantitation of intracellular oncoprotein binding sites *in vivo* using autoradiographic; or modem radiographic or other membrane binding techniques such as positron emission tomography in order to locate tumours with anti-intracellular oncoprotein antibody binding sites. This application provides important diagnostic and research tools.

In other embodiments, the anti-intracellular oncoprotein antibody may be coupled to a scintigraphic radiolabel, a cytotoxic compound or radioisotope, an enzyme for converting a non-toxic prodrug into a cytotoxic drug, a compound for activating the immune system in order to target the resulting conjugate to a colon tumour, or a cell-stimulating compound. Such conjugates have a "binding portion", which consists of the anti-intracellular oncoprotein antibody, and a "functional portion", which consists of the radiolabel, toxin or enzyme.

The antibody may alternatively be used alone in order simply to block the activity of the intracellular oncoprotein antigen, particularly by physically interfering with its binding of another compound.

The binding portion and the functional portion of the conjugate (if also a peptide or poypeptide) may be linked together by any of the conventional ways of cross linking polypeptides, such as those generally described in O'Sullivan et af (Anal. Biochem 1979: 100, 100-108). For example, one portion may be enriched with thiol groups and the other portion reacted with a bifunctional agent capable of reacting with those thiol groups, for example the N-hydroxysuccinimide ester of iodoacetic acid (NHIA) or N-succinimidyl-3,(2-pyridyldithio)propionate (SPDP). Amide and thioetherbonds, for example achieved with m-maleimidobenzoyl-N-hydroxysuccinimide ester, are generally more stable *in vivo* than disulphide bonds.

Alternatively, if the binding portion contains carbohydrates, such as would be the case for an antibody or some antibody fragments, the functional portion may be linked via the carbohydrate portion using the linking technology in EP 0 088 695.

The functional portion of the conjugate may be an enzyme for converting a non-toxic prodrug into a toxic drug, for example the conjugates of Bagshawe and his colleagues (Bagshawe (1987) Br. 1. Cancer 56, 531; Bagshawe et af (Br. 1. Cancer 1988: 58, 700); WO 88/07378) or cyanide-releasing systems (WO 91/11201).

The functional portion of the anti-intracellular oncoprotein antibody conjugate, when the anti-intracellular oncoprotein antibody conjugate is used for diagnosis, may comprise or consist of a radioactive atom for scintigraphic studies, for example technetium 99m (^{99m}Tc) or iodine-123 (¹²³I), or a spin label for nuclear magnetic resonance (nmr) imaging (also known as magnetic resonance imaging, mri), such as iodine-123 again, iodine-313, indium-111, fluorine-19, carbon-13, nitrogen-15, oxygen-17, gadolinium, manganese or iron.

When used in a compound for selective destruction of the tumour, the functional portion of the anti-intracellular oncoprotein antibody may comprise a highly radioactive atom, such as iodine-131 , rhenium-186; rhenium-188, yttrium-90 or lead-212, which emits enough energy to destroy neighbouring cells, or a cytotoxic chemical compound such as methotrexate, adriamicin, vinca alkaliods (vincristine, vinblastine, etoposide), daunorubicin or other intercalating agents.

The radio- or other labels may be incorporated in the anti-intracellular oncoprotein antibody conjugate in known ways. For example, the peptide may be biosynthesised or may be synthesised by chemical amino acid synthesis using suitable amino acid precursors involving, for example, fluorine-19 in place of hydrogen. Labels such as ^{99m}Tc, ¹²³I, ¹⁸⁶Rh, ¹⁸⁸Rh and ¹¹¹In can be attached via a cysteine residue in the peptide. Yttrium-90 can be attached via a lysine residue. The IODOGEN method (Fraker et af (1978) Biochem. Biophys. Res. Commun. 80: 49-57 can be used to incorporate iodine-123. "Monoclonal Antibodies in Immunoscinigraphy" (Chatal, CRC Press 1989) describes other methods in detail.

It may not be necessary for the whole enzyme to be present in the conjugate but, of course, the catalytic portion must be present. So-called "abzymes" may be used, where a anti-intracellular oncoprotein antibody is raised to a compound involved in the reaction one wishes to catalyse, usually the reactive intermediate state. The resulting antibody can then function as an enzyme for the reaction.

The conjugate may be purified by size exclusion or affinity chromatography, and tested for dual biological activities. The antigen immunoreactivity may be measured using an enzyme-linked immunosorbent assay (ELISA) with immobilised antigen and in a live cell radioimmunoassay. An enzyme assay may be used for β-glucosidase using a substrate which changes in absorbance when the glucose residues are hydrolysed, such as *o*NPG (*o*-nitrophenyl-β-D-glucopyranoside), liberating 2-nitrophenol which is measured spectrophotometrically at 405 nm.

The stability of the conjugate may be tested *in vitro* initially by incubating at 37°C in serum, followed by size exclusion FPLC analysis. Stability *in vivo* can be tested in the same way in mice by analysing the serum at various times after injection of the conjugate. In addition, it is possible to radiolabel the anti-intracellular oncoprotein antibody with ¹²⁵I , and the enzyme with ¹³¹I before conjugation, and to determine the biodistribution of the conjugate, free anti-intracellular oncoprotein antibody and free enzyme in an animal, for example a mouse.

Alternatively, the conjugate may be produced as a fusion compound by recombinant DNA techniques whereby a length of DNA comprises respective regions encoding the two portions of the conjugate either adjacent to one another or separated by a region encoding a linker peptide which does not destroy the desired properties of the conjugate.

Conceivably, two of the functional portions of the compound may overlap wholly or partly. The DNA is then expressed in a suitable host in known ways.

### DIAGNOSTIC KITS

We also disclose diagnostic methods and kits for detection and measurement of intracellular oncoprotein in biological fluids and tissues, and for localization of intracellular oncoprotein in tissues.

The anti-intracellular oncoprotein antibodiess can also be used in a diagnostic method and kit to detect and quantify antibodies capable of binding intracellular oncoprotein. These kits may permit detection intracellular oncoprotein which, in certain situations, may indicate the spread of micrometastases by primary tumours *in situ.* Patients that have such circulating anti-intracellular oncoprotein antibodies may be more likely to develop tumours and cancers, and may be more likely to have recurrences of cancer after treatments or periods of remission.

Kits for measurement of intracellular oncoprotein are also contemplated. The anti-intracellular oncoprotein antibodies that possess high titer and speciticity can be used to establish easy to use kits for rapid, reliable, sensitive. and specific measurement and localization of intracellular oncoprotein in extracts of plasma, urine, tissues. and in cell culture media.

These assay kits include but are not limited to the following techniques; competitive and non-competitive assays, radioimmunoassay, bioluminescence and chemiluminescence assays, fluorometric assays, sandwich assays, immunoradiometric assays. dot blots, enzyme linked assays including ELISA, microtiter plates, antibody coated strips or dipsticks for rapid monitoring of urine or blood. and immunocytochemistry. For each kit the range, sensitivity, precision, reliability, specificity and reproducibility of the assay are established. Intraassay and interassay variation is established at 20%, 50% and 80% points on the standard curves of displacement or activity.

One example of an assay kit commonly used in research and in the clinic is a radioimmunoassay (RIA) kit. After successful radioiodination and purification of an anti-intracellular oncoprotein antibody, the antiserum possessing the highest titer is added at several dilutions to tubes containing a relatively constant amount of radioactivity, such as 10,000 cpm, in a suitable buffer system. Other tubes contain buffer or pre immune serum to determine the non-specific binding. After incubation at 4°C for 24 hours, protein A is added and the tubes are vortexed, incubated at room temperature for 90 minutes, and centrifuged at approximately 2000-2500 times g at 4°C to precipitate the complexes of antiserum bound to the labeled anti-intracellular oncoprotein antibody. The supernatant is removed by aspiration and the radioactivity in the pellets counted in a gamma counter. The antiserum dilution that binds approximately 10 to 40% of the labeled anti-intracellular oncoprotein antibody after subtraction of the non-specific binding is further characterized.

An immunohistochemistry kit may also be used for localization of intracellular oncoprotein in tissues and cells. This immunohistochemistry kit provides instructions, an anti-intracellular oncoprotein antibody, and possibly blocking serum and secondary antiserum linked to a fluorescent molecule such as fluorescein isothiocyanate, or to some other reagent used to visualize the primary antiserum. Immunohistochemistry techniques are well known to those skilled in the art.

This immunohistochemistry kit permits localization of intracellular oncoprotein in tissue sections and cultured cells using both light and electron microscopy. It is used for both research and clinical purposes. For example, tumours are biopsied or collected and tissue sections cut with a microtome to examine sites of intracellular oncoprotein production. Such information is useful for diagnostic and possibly therapeutic purposes in the detection and treatment of cancer.

### PHARMACEUTICAL COMPOSITIONS

The anti-intracellular oncoprotein antibodies and intracellular oncoprotein vaccines may be effective in treating cancer related diseases.

We disclose a method of treating a cancer related disease with an effective amount of a anti-intracellular oncoprotein antibody antibody described here. We further disclose a method of preventing a cancer related disease with vaccination with an intracellular oncoprotein as a vaccine,

The anti-intracellular oncoprotein antibodies or intracellular oncoproteins for use as vaccines may be provided as isolated and substantially purified proteins and protein fragments in pharmaceutically acceptable compositions using formulation methods known to those of ordinary skill in the art.

The anti-intracellular oncoprotein antibody or intracellular oncoprotein vaccine may be administered in the form of a pharmaceutical composition. Such a pharmaceutical composition may include a therapeutically effective amount of anti-intracellular oncoprotein antibody and/or intracellular oncoprotein vaccine, together with a suitable excipient, diluent or carrier.

The anti-intracellular oncoprotein antibody and/or intracellular oncoprotein vaccine may in particular be introduced into the circulation of a patient, for example by being injected into a patient via, e.g., a vein.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampoules and vials, and may be stored in a freeze-dried (lyophilized) condition requiring only the addition of the sterile liquid carrier, for example, water for injections, immediately prior to use. Extemporaneous injection solutions and suspensions may be prepared from sterile powders, granules and tablets of the kind previously described.

These compositions can be administered by standard routes. These include but are not limited to: oral, rectal, ophthalmic (including intravitreal or intracameral), nasal, topical (including buccal and sublingual), intrauterine, vaginal or parenteral (including subcutaneous, intraperitoneal, intramuscular, intravenous, intradermaL intracranial, intratracheal, and epidural) transdermal, intraperitoneal. intracranial, intracerebroventricular, intracerebral, intravaginal, intrauterine, or parenteral (e.g., intravenous, intraspinal, subcutaneous or intramuscular) routes.

The anti-intracellular oncoprotein antibody and/or intracellular oncoprotein vaccine formulations may conveniently be presented in unit dosage form and may be prepared by conventional pharmaceutical techniques. Such techniques include the step of bringing into association the active ingredient and the pharmaceutical carrieres) or excipient(s). In general, the formulations are prepared by uniformly and intimately bringing into association the active ingredient with liquid carriers or finely divided solid carriers or both, and then, if necessary, shaping the product.

In addition, the anti-intracellular oncoprotein antibody and/or intracellular oncoprotein vaccine may be incorporated into biodegradable polymers allowing for sustained release of the compound, the polymers being implanted in the vicinity of where drug delivery is desired, for example, at the site of a tumor or implanted so that the anti-intracellular oncoprotein antibody and/or intracellular oncoprotein vaccine is slowly released systemically. The biodegradable polymers and their use are described, for example, in detail in Brem et af (1. Neurosurg 1991 74:441-446). Osmotic minipumps may also be used to provide controlled delivery of high concentrations of anti-intracellular oncoprotein antibody and/or intracellular oncoprotein vaccine through cannulae to the site of interest, such as directly into a metastatic growth or into the vascular supply to that tumor.

The anti-intracellular oncoprotein antibody and/or intracellular oncoprotein vaccine may be linked to cytotoxic agents which are infused in a manner designed to maximize delivery to the desired location. For example, ricin-linked high affinity anti-intracellular oncoprotein antibody and/or intracellular oncoprotein vaccine are delivered through a cannula into vessels supplying the target site or directly into the target. Such agents are also delivered in a controlled manner through osmotic pumps coupled to infusion cannulae.

Preferred unit dosage formulations are those containing a daily dose or unit, daily subdose, as herein above recited, or an appropriate fraction thereof, of the administered ingredient. It should be understood that in addition to the ingredients, particularly mentioned above, the formulations described here may include other agents conventional in the art having regard to the type of formulation in question.

The anti-intracellular oncoprotein antibody and/or intracellular oncoprotein vaccine conjugates may be administered in any suitable way. usually parenterally, for example intravenously or intraperitoneally, in standard sterile, non-pyrogenic formulations of diluents and carriers, for example isotonic saline (when administered intravenously). Once the anti-intracellular oncoprotein antibody and/or intracellular oncoprotein vaccine conjugate has bound to the target cells and been cleared from the bloodstream (if necessary), which typically takes a day or so, the pro-drug is administered, usually as a single infused dose, or the tumour is imaged. If needed, because the anti-intracellular oncoprotein antibody and/or intracellular oncoprotein vaccine conjugate may be immunogenic, cyclosporin or some other immunosuppressant can be administered to provide a longer period for treatment but usually this will not be necessary.

The dosage of the anti-intracellular oncoprotein antibody and/or intracellular oncoprotein vaccine described here will depend on the disease state or condition being treated and other clinical factors such as weight and condition of the human or animal and the route of administration of the compound.

Depending upon the half-life of the anti-intracellular oncoprotein antibody and/or intracellular oncoprotein vaccine in the particular animal or human, the anti-intracellular oncoprotein antibody and/or intracellular oncoprotein vaccine can be administered between several times per day to once a week. It is to be understood that the methods and compositions described here have application for both human and veterinary use. The methods described here contemplate single as well as multiple administrations, given either simultaneously or over an extended period of time.

The timing between administrations of the anti-intracellular oncoprotein antibody and/or intracellular oncoprotein vaccine conjugate and pro-drug may be optimised in a routine way since tumour/normal tissue ratios of conjugate (at least following intravenous delivery) are highest after about 4-6 days, whereas at this time the absolute amount of conjugate bound to the tumour, in terms of percent of injected dose per gram, is lower than at earlier times.

Therefore, the optimum interval between administration of the anti-intracellular oncoprotein antibody and/or intracellular oncoprotein vaccine conjugate and the pro-drug will be a compromise between peak tumour concentration of enzyme and the best distribution ratio between tumour and normal tissues. The dosage of the anti-intracellular oncoprotein antibody and/or intracellular oncoprotein vaccine conjugate will be chosen by the physician according to the usual criteria. At least in the case of methods employing a targeted enzyme such as β-glucosidase and intravenous amygdalin as the toxic pro-drug, 1 to 50 daily doses of 0.1 to 10.0 grams per square metre of body surface area, preferably 1.0-5.0 g/m² are likely to be appropriate. For oral therapy, three doses per day of 0.05 to 10.0g, preferably 1.0-5.0g, for one to fifty days may be appropriate. The dosage of the anti-intracellular oncoprotein antibody and/or intracellular oncoprotein vaccine conjugate will similarly be chosen according to normal criteria, particularly with reference to the type, stage and location of the tumour and the weight of the patient. The duration of treatment will depend in part upon the rapidity and extent of any immune reaction to the anti-intracellular oncoprotein antibody and/or intracellular oncoprotein vaccine conjugate.

### DISEASES

Anti-intracellular oncoprotein antibody and/or intracellular oncoprotein vaccine described here, for example in the form of pharmaceutical compositions, may be used in the treatment of cancer.

For the purposes of this document, the term "cancer" can comprise any one or more of the following: acute lymphocytic leukemia (ALL), acute myeloid leukemia (AML), adrenocortical cancer, anal cancer, bladder cancer, blood cancer, bone cancer, brain tumor, breast cancer, cancer of the female genital system, cancer of the male genital system, central nervous system lymphoma, cervical cancer, childhood rhabdomyosarcoma, childhood sarcoma, chronic lymphocytic leukemia (CLL), chronic myeloid leukemia (CML), colon and rectal cancer, colon cancer, endometrial cancer, endometrial sarcoma, esophageal cancer, eye cancer, gallbladder cancer, gastric cancer, gastrointestinal tract cancer, hairy cell leukemia, head and neck cancer, hepatocellular cancer, Hodgkin's disease, hypopharyngeal cancer, Kaposi's sarcoma, kidney cancer, laryngeal cancer, leukemia, leukemia, liver cancer, lung cancer, malignant fibrous histiocytoma, malignant thymoma, melanoma, mesothelioma, multiple myeloma, myeloma, nasal cavity and paranasal sinus cancer, nasopharyngeal cancer, nervous system cancer, neuroblastoma, non-Hodgkin's lymphoma, oral cavity cancer, oropharyngeal cancer, osteosarcoma, ovarian cancer, pancreatic cancer, parathyroid cancer, penile cancer, pharyngeal cancer, pituitary tumor, plasma cell neoplasm, primary CNS lymphoma, prostate cancer, rectal cancer, respiratory system, retinoblastoma, salivary gland cancer, skin cancer, small intestine cancer, soft tissue sarcoma, stomach cancer, stomach cancer, testicular cancer, thyroid cancer, urinary system cancer, uterine sarcoma, vaginal cancer, vascular system, Waldenstrom's macroglobulinemia and Wilms' tumor.

Anti-intracellular oncoprotein antibody and/or intracellular oncoprotein vaccine described here, for example in the form of pharmaceutical compositions, can also be used in the treatment of cancer related disorders.

Such disorders include but not limited to: solid tumours; blood born tumours such as leukemias; tumor metastasis; benign tumours, for example hemangiomas, acoustic neuromas, neurofibromas, trachomas, and pyogenic granulomas; rheumatoid arthritis; psoriasis; ocular angiogenic diseases, for example, diabetic retinopathy, retinopathy of prematurity, macular degeneration, corneal graft rejection, neovascular glaucoma, retrolental fibroplasia, rubeosis; Osler- Webber Syndrome; myocardial angiogenesis; plaque neovascularization; telangiectasia; hemophiliac joints; angiofibroma; wound granulation; coronary collaterals; cerebral collateralsl arteriovenous malformations; ischemic limb angiogenesis; neovascular glaucoma; retrolental fibroplasia; diabetic neovascularisation; helicobacter related diseases, fractures, vasculogenesis, hematopoiesis, ovulation, menstruation and placentation.

### EXAMPLES

Our results demonstrated that upon tumor-specific antigen challenge; host immunity could be stimulated to produce endogenous antibodies against the specific antigen leading to tumor inhibition. This concept of `cancer vaccination' has long been a promising but challenging prospect²¹. Importantly, we found that *antigen-induced antibody therapy* could achieve similar anti-tumor therapeutic efficacy compared to exogenously delivered antibodies. In addition to being more economical, we believe that the former approach may be more useful than the latter as we naturally have huge potential flexibility to generate high titers of antigen-induced anti-tumor antibodies within ourselves. Although using 'oncoprotein' for vaccination does not seem a practical thought, we do believe this approach is worth for future investigation. An 'oncoprotein' should sit properly at its native subcellular localization with its neighborhood partners for correct communication in order to preserve an oncogenic function in cancer cell signaling network. When an 'oncoprotein,' was isolated from its native dynamic complexity of sub-cellular localization, it may lose its connections and unable to perform its normal biological roles in such an 'isolated' situation.

Our *in vivo* data reveal a hitherto unrecognized phenomenon that immune-therapies can target not only extracellular- but also intracellular-oncoproteins for anticancer activity.

### Examples Section E1 (Examples 1 to 18). Proof-of-concept for targeting intracellular oncoproteins with antibody therapy or vaccination

Section E1 comprises Examples 1 to 18. Example 1 is an Introduction to Section E1, Examples 2 to 10 are Materials and Methods for Section E1, Examples 11 to 16 are Results for Section E1, Example 17 is a Discussion of Section E1, Example 18 is References for Section E1.

### Example 1. Introduction (Section E1)

Monoclonal antibodies (mAbs) have proven to be potent and highly specific biological agents against some of humanity's most deadly diseases'. To exploit the ability of antibodies to specifically bind to biological targets, significant research and development have focused on developing monoclonal antibodies against extracellular/cell surface oncogenic targets. The first wave of success from such molecular targeted cancer therapeutics was the FDA approval of the anti-vascular endothelial growth factor antibody bevacizumab (Avastin) and the anti-HER2/neu antibody trastuzumab (Herceptin) for use in treatment of colorectal and breast cancers respectively². These are examples of antibodies which work conventionally against extracellular proteins. Unfortunately, the majority of cellular proteins is intracellular and has thus remained underexplored by the approach of antibody therapies³ due to the general view that intracellular locations are inaccessible to antibodies. However, numerous experimental findings and clinical observations since 1978 have suggested otherwise, immunologists have showed that penetration of auto-antibodies into living cells may be a common cellular phenomenon^{4,5}.

To prove the concept of possible immunotherapies against intracellular targets, we selected three intracellular targets for cancer treatments in this study, namely phosphatase of regenerating liver 3 (PRL-3), Enhanced Green Fluorescent Protein (EGFP), and polyomavirus middle T (mT) oncoprotein. PRL-3 is one of the three PRL-phosphatases identified between 1994 and 1998^{6,7}, and the PRL-1, PRL-2, and PRL-3 form a subgroup of the protein tyrosine phosphatase (PTP) family⁸. PRLs are intracellular C-terrninally prenylated phosphatases^{9,10}, and the localization of PRL-1 and PRL-3 to the inner leaflet of the plasma membrane and early endosomes have been revealed by EM immunogold labeling". The PRL phosphatases represent an intriguing group of proteins being validated as biomarkers and therapeutic targets in human cancers. Upregulation of PRL-3 mRNA level was first found to tightly correlate with colorectal cancer metastasis in 2001 by the global gene expression profiles of metastatic colorectal cancer with that of primary cancers, benign colorectal tumors, and normal colorectal epithelium using serial analysis of gene expression (SAGE) technology¹². PRL-3 protein levels also have been reported to be elevated in an average of 22.3 % of cancer samples (n=1008)¹³. Additionally, upregulation of individual PRLs have been reported to be correlated with numerous types of advanced human metastatic cancers when compared with their normal counterparts¹⁴. Thus, oncogenic PRL-3 was chosen as an ideal intracellular target for the study.

Secondly, to explore whether the antibody therapy could have a general application against other intracellular proteins, we chose a popular reporter protein, the cytosolic enhanced green fluorescent protein (EGFP) originally isolated from the jellyfish *Aequorea victoria.* EGFP expresses itself as an intracellular protein with a nucleo-cytoplasmic localization pattern. Since EGFP is not expressed in the host tissues, when artificially overexpressed in cancer cells, EGFP serves as a cancer cell specific intracellular protein. This allowed us to check whether EGFP-directed antibody therapy would specifically eradicate EGFP-expressing tumors and show any non-specific undesired side effects to the host.

Finally, to test the principle of antibody therapy in another animal tumor model, we use a well known mammary cancer model of MMTV-PymT transgenic mice¹⁵ carrying the middle T (mT) oncogene under the transcriptional control of the mouse mammary tumor virus promoter/enhancer. Such transgenic mice have been widely used as excellent spontaneous tumor models for decades in the cancer research community to assess the relative contribution of the metastatic mammary tumor phenotype^{16,17}.

Vaccines are inexpensive and yet effective in treating existing cancer or prevent cancer development¹⁸. Such specific active immunotherapy of cancer, if successful, has clear advantages over passive immunotherapy. If an antibody could recognize its intracellular antigen we can expect that an intracellular antigen could be used to trigger natural antibody production by the host immune system to achieve a similar effect of the antibody therapy against cancer. Here, we extend our study to evaluate the reliability of vaccinations with purified PRL-3, EGFP and mT proteins for antigen-induced antibody therapies.

### Example 2. Materials and Methods (Section E1): Cell lines and cell culture

B16F0 (CRL-6322) and B16F10 (CRL-6475) murine melanoma cell lines (derived from the C57BL/6J strain) were purchased from the American Type Culture Collection (ATCC, Manassas, VA). Cells were grown in RPMI medium supplemented with 10% fetal bovine serum and antibiotics and maintained in 37°C incubator supplied with 5% CO₂.

### Example 3. Materials and Methods (Section E1): Western blot analysis

Detailed steps were described in our previous study²².

### Example 4. Materials and Methods (Section E1): Antibodies

Mouse monoclonal antibodies (clone #318, IgG1) were generated in-house, as described previously²² . EGFP mouse monoclonal antibody (sc-9996, IgG2a) and Polyoma virus-middle T antigen (PymT) rat monoclonal antibodies (sc-53481, IgG2b) were from Santa Cruz biotechnology, Inc.

### Example 5. Materials and Methods (Section E1): Construction of GST-PRL-3, GST-middle T plasmids, and preparation of the GST-fusion proteins

The detailed steps for generating GST-PRL-3 fusion proteins were described previously²². To make GST-middle T: forward primer 5' gcggatccatggatagagttctgagcagagctgac 3' and reverse primer 5' ctgaattcctagaaatgccgggaacg 3' were used for PCR using pXJ40 vector (i.e. pXJ40-PyMT) as a template. The PCR fragments were digested with BamH1 and EcoR1 and ligated into respective sites of the pGEX-KG vector. The preparations of GST-fusion protein were described in detail previously²².

### Example 6. Materials and Methods (Section E1): Experimental metastasis assay²³

All animal studies were approved by the Institutional Animal Care and Use Committee (IACUC) and were carried out in accordance with the policies of Institute of Molecular and Cell Biology's Review Board (IRB), Singapore¹⁸. C57BL6 and *Scid* mice were from BRC (Biological Resources Centre. Agency for Science, technology and Research, Singapore), muMT mice were from The Jackson Laboratory, USA, Rag 2^{-/-} mice were from Taconic, USA, Rag 2^{-/-} mice were from Taconic, USA. 8-week old mice were injected with one million cancer cells via their tail vein (day 1). The treated mice were administrated with mAbs via tail vein on day 3 and subsequent administration twice a week. 4-week old transgenic mice (PymT) were divided into two groups receiving anti-mT antibody or PBS, twice per week. Mice were sacrificed and all organs were inspected for the presence of macroscopic metastases. Metastatic tumors or organs were removed and fixed in 4% paraformaldehyde for histological analysis. Lung metastatic nodules were counted under a dissecting microscope. The numbers of mice used in antibody therapies in each experiment are summarized in Figure 5G.

### Example 7. Materials and Methods (Section E1): Genotyping of MMTV-PymT mice

The MMTV-PymT mouse strain was purchased from Mouse Models of Human Cancers Consortium (MMHCC). Females fail to lactate so this strain is maintained by breeding heterozygous (Tg/+) males with FVB/N (+/+) wild type females. Tail tip DNAs were extracted with DNA digestion buffer (12 ml: 10.08 ml, 1.2 ml of 10x GB* buffer, 0.6 ml 10 % Triton X-100, 0.12 ml of 2-mercaptoethanol). *Gitschier's Buffer (10 ml: 3.35ml of 2M Tris pH 8.8, 1.66ml of 1M (NH₄)₂SO_{4,}1.34ml of 0.5MMgCl₂,1.65m) MQ H₂O). Forward primer P001: 5'-cAA ATG TTG cTT GTc TGG TG-3' and reverse primer P002: 5'-GTc AGT cGA GTG cAc AGT TT-3' were used to PCR wild type 200bp fragment. Forward primer P001: 5'-GGA AGc AAG TAc TTc AcA AGG G-3' and reverse primer P004: 5'-GGA AAG TcA cTA GGA GcA GGG-3 were used to PCR 566bp transgene fragment. The genotyping procedure has been described previously (http://mouse.ncicrf.gov/protocols).

### Example 8. Materials and Methods (Section E1): Whole Mount Preparation and Carmine Alum Stain

All procedures below were carried out at room temperature. Abdominal mammary glands were excised during necropsy for whole mount preparations, spread on glass slides, and fixed in Carnoy's fixative (6 parts 100% ethanol, 3 parts chloroform, and 1 part glacial acetic acid) for 4 h. Subsequently, the tissue was washed in 70% ethanol for 15 min, and the ethanol was changed gradually to distilled water then was finally rinsed in distilled water for 5 min. Staining was carried out overnight in carmine alum stain. The tissue was then dehydrated in graded alcohol solutions (70, 95, and 100%; 15 min each) and was cleared in two changes of xylene, mounted, and coverslipped using Permount. Whole mounts were observed under a Leica dissecting microscope (Leica Microsystems GmbH, Wetzlar, Germany), and digital images were recorded using a SPOT FLEX® color digital camera (Diagnostic Instruments, Inc. Sterling Heights, MI) using a SPOT software package (Version 4.5, Diagnostic Instruments, Inc. Sterling Heights, MI).

### Example 9. Materials and Methods (Section E1): Immunization of C57BL6 mice and MMTV-PymT transgenic mice

Freund's Complete Adjuvant is the form that contains killed cells of *Mycobacterium butyricum* to enhance the immune response. The Complete Adjuvant is used in initial injections. The form that does not contain this bacterium is known as Freund's Incomplete Adjuvant that is used to boost in subsequence injections. 8-week old C57BL6 mice were immunized by intraperitoneal injection with a total volume of 200 µl Freund's Adjuvant: PRL-3 (20 µg) or EGFP (20 µg) in -100 µl saline mixed with 100 µl of complete adjuvant (Cat#77140, Pierce). 4-week old MMTV-PymT TG mice were immunized by intraperitoneal injection with a total volume of 200 µl Freund's Adjuvant: mT (10 µg) antigen in 100 µl saline mixed with 100 µl of complete adjuvant (Cat#77140, Pierce). The next two immunizations were injected with a total volume of 200 µl incomplete adjuvant (Cat#77145, Pierce). The second and third injections were done two-week intervals. Subsequently, 100-200 µl of tail bleed was collected in a heparin-coated capillary tube, plasma was prepared from the blood sample, and the antibody titer was measured by ELISA. The detailed steps of ELISA were described previously²². Mice with high titers of PRLs-3, EGFP, or mT antibodies in their sera were selected for experiment and further analysis. The numbers of mice used for vaccinations are summarized in Figure 6D.

### Example 10. Materials and Methods (Section E1): ELISA assay

PRL-3 and mT antigen stocks were made in carbonate-bicarbonate buffer with pH 7.4. 100 µl solution containing 50 ng of appropriate antigen was added to each 96-well plate and incubated at 4 °C overnight to coat the antigen onto the plate. The plate was then blocked with 3 % BSA in PBS containing 0.05 % Tween 20 for 1 hour at 37 °C and then washed three times with PBS. Blood serum (1.0 µl) from each mouse was diluted in 100 µl of blocking solution and added to each well, and then incubated for 2 hours at 37 °C. 100 µl of appropriate secondary antibody conjugated with HRP (Pierce, USA) diluted at 1:5000 in PBS was added to each well and incubated at 37 °C for 1 hour. The plate was rinsed with PBS containing 0.05 % Tween-20 three times followed by 3 washes with sterile water. The substrate, 100 µl of Turbo-TMB™ (Pierce, USA)), was added to each well and incubated for 10 min at room temperature. The reaction was stopped by adding 100 µl of concentrated H₂SO₄. Absorbance was measured at 450 nm using a Dynatech MR7000 plate reader (Dynatech, USA).

### Example 11. Materials and Methods (Section E1): Statistical analysis

Use Kaplan-Meier method to estimate cumulative survival rates for 'treated' verse 'untreated' mice. A p value of <0.05 was considered statistically significant.

### Example 12. Results (Section E1): In C57BL6 wild type mice, PRL-3 mAb (IgG1) effectively retards metastatic tumors that express endogenous PRL-3 protein

We have previously demonstrated the efficacy of antibody therapy against intracellular PRL proteins in an immunocompromised *nude* mice metastatic tumor model¹⁹. To examine this therapeutic approach in a more relevant model for future clinical applications, we extended this study to examine if the antibody treatment could function in immunocompetent C57BL6 wild type mice. On day 1, mice (8-week old) were intravenously (i.v.) injected via lateral tail vein with C57BL6-derived B16F0 (F0) melanoma cancer cells, which express endogenous PRL-3 (Figure 1A, lane 1). On day 3, mice were divided into 'untreated' and 'treated' groups which then received two administrations of PBS or PRL-3 mAb respectively per week during the whole duration of the experiment (Figure 1B). At the end of the experiment (day 17), 'untreated' mice displayed severe weight loss, multiple metastatic tumors in various organs including the lung, liver, adrenal gland, kidney and bone. In contrast, 'treated' mice appeared more active and healthier in appearance. The 'treated' mice group, but not the 'untreated' group, constantly gained body weight throughout the duration of the experiment, in line with a reduction in tumor metastases (Figure 1C-D). To confirm the specificity of the PRL-3 mAb, an identical experiment was conducted in parallel, in which mice were intravenously injected with C57BL6-derived B16F10 (F10) cells, another melanoma cell line, which expresses very low levels of PRL-3 (Figure 1A, lane 2). By the end of the experiment (on day 17), all F10 recipients had developed metastases in the ovary, adrenal gland, kidney, and bone, regardless of whether they had received PRL-3 mAb. Both groups of mice experienced significant loss in body weight and appeared weak/inactive (Figure 1E-F), indicating that non-PRL-3 expressing F10 recipients had a poor response to PRL-3 antibody treatment. Note that F10 recipients develop more aggressive metastatic tumors in lungs (Figure 1E) compared to lungs from F0 recipients (Figure 1C). These data suggest that the efficiency of the PRL-3 antibody treatment was tightly correlated with PRL-3 expression status of the cancer cells but not with the degree of metastatic activities. The current data derived from immunocompetent wild-type C57BL6 mice thus substantiate our previous findings from inmmunocompromised *nude* mice¹⁹

### Example 13. Results (Section E1): In B-cell-deficient mice, PRL-3 mAb (IgG1) fails to retard metastatic tumors that express endogenous PRL-3 protein

To investigate if lymphocytes are involved in the effect of antibody therapy observed, we employed two engineered strains of immunodeficient mice: muMT mice, which are C57BL6 mice homozygous for an inactivating mutation of the membrane exon of the *mu* chain gene. They cannot form a preBCR and are consequently devoid of mature B lymphocytes²⁰. We also employed Rag-2 mice that carry a germline mutation in which a large portion of the RAG-2 coding region is deleted, thus giving rise to homozygous mutants (Rag2^{-/-}) which are viable but fail to produce mature B or T lymphocytes ²¹. Kaplan-Meier survival curves were used to compare 'treated' and 'untreated' mice among muMT-F0, C57BL6-F0, and C57BL6-F10 mice. Surprisingly, we did not observe any PRL-3 mAb therapeutic efficacy in repressing tumors in muMT-F0 mice, with PRL-3 mAb therapy having no impact in extending the life-span for muMT-F0 mice with a median survival of 19 days for 'treated' and 18.5 days for 'untreated' mice (Figure 2A, *p* value= 0.8661). Similarly, no difference in tumor metastases was observed between 'treated' and 'untreated' groups in B- and T-cell deficient Rag2-F0 mice (data not shown). In contrast, PRL-3 mAb therapy prolonged the survival rates for C57BL6-F0 mice with a median survival of 21.5 days for 'treated' but 17 days for 'untreated' mice (Figure 2B*, p=* 0002). In agreement with the previous finding, PRL-3 mAb therapy did not prolong the overall survival of C57BL6-F10 mice with non-PRL-3 expressing tumors with a median survival of 17.5 days for 'treated' and 16.5 day for untreated mice (Figure 2C,p=0.535). To summarize the effectiveness of antibody therapy among the different mice backgrounds, we scored the ability for PRL-3 mAb to reduce the degree of metastatic tumor formation relative to untreated mice in each group. When the reduction of metastatic tumor formation by at least 70% (±10 %) was scored as an effective outcome, a clear beneficial effect was seen only in the C57BL6-F0 group but not in the muMT-F0 (Figure 2D). Thus, PRL-3 antibody therapy likely requires mature B-cell function for anti-tumor activity.

### Example 14. Results (Section E1): In C57BL6 mice, EGFP mAb (IgG2a) effectively blocks metastatic tumors that express EGFP protein

The intriguing ability of PRL-3 mAb to inhibit tumor metastasis motivated us to investigate whether the therapeutic strategy could be applied to other intracellular proteins as well. Since EGFP is a non-cancer related protein, we chose to use EGFP as an intracellular protein marker specifically expressed in cancer cells. As EGFP is not expressed in the host tissues, we predicted that EGFP antibody should have few undesired side effects in the animal model. Utilizing pooled F0 and F10 melanoma cancer cell lines heterogeneously overexpressing exogenous EGFP protein (Figure 5A, Figure 5B), the EGFP-F0 and EGFP-F10 expressing cells were injected and treated with EGFP mAb following the same therapeutic schedule (Figure 5C). As predicted, both EGFP-F0 and EGFP-F10 cells responded equally well to EGFP antibody treatment in terms of tumor regressions regardless of PRL-3 expression. Notably, discrete clusters of green fluorescent or black metastatic tumors were found in the lungs (Figure 5D-E, panels a, e). The non-EGFP expressing metastases in black thus served as internal negative controls to the green metastatic tumors in this model. Remarkably, EGFP mAb could specifically reduce numbers of EGFP-metastases but not that of non-EGFP-metastases (Figure 5D-E, panels e), indicating that only EGFP-expressing tumors respond to EGFP mAb therapy. Upon scoring the results based on metastasis tumor load reduction, we found that the efficacy of the EGFP mAb effect was tightly correlated with EGFP expression status in cancer cells (Figure 5G). Thus, antibody therapy depends on specific antibody-antigen interactions on (or within) cells for therapeutic efficacy.

### Example 15. Results (Section E1): In MMTV-PymT transgenic mice, mT (rat IgG2b) mAb prevents the formation of mT-expressing mammary tumor progression

Having addressed that antibody therapy could work against both endogenous (such as PRL-3) and exogenous (such as EGFP) intracellular proteins, we asked if this approach could inhibit tumor formation in spontaneous tumor animal models. To this end, we chose popular MMTV-PymT transgenic mouse model of a spontaneous mammary tumorigenesis. MMTV-PymT transgenic mice express the polyomavirus middle T (mT) oncogenic protein, a DNA viral protein containing 421 amino acids which acts as a potent oncogene in the mammary epithelium. mT is represented linearly, tethered (21 aa) to the membrane at its carboxy-terminal end with a short KRSRHF motif likely exposed at the cell surface (too short to be an external epitope, in general). Therefore, most of this protein is facing the cytosolic compartment and has thus considered an intracellular protein¹⁵. Cells expressing the mT possess an enhanced metastatic potential ¹⁶. All female carriers (genotype+/-) develop palpable mammary tumors (adenocarcinomas) at the age of 2-3 months (http://mouse.ncifcrf.gov/information/order livernice.asp). Middle T expression is detected at high levels in male and female mammary glands, and the expression of mT oncogene is sufficient for mammary epithelial cell transformation. We used 44 heterozygous (+/-) young transgenic females-using RT-PCR to confirm their genotypes (Figure 3A) and then divided them into 'treated' (n=18) and 'untreated' (n=26) groups. To determined if mT antibody could reduce mT-expressing mammary tumor development, 'treated' mice were i.v. injected with mT antibody at 4-week of age, followed by two administrations of antibody weekly for the whole duration of the experiment for about 3-months (Figure 3B). In Figure 3C, 'untreated' mice (#2, #6) and 'treated' mice (#7, #10) were selected as representative examples; an FVB/N non-transgenic female (#3) was used as a control for normal sizes of mammary glands (5 pairs). Histopathological examination of the mammary glands of 'untreated' mice exhibited irregular formation of side branches, enlarged terminal buds, and some large multi-lobular tumor masses (Figure 3D, a). In contrast, breast tissue from treated mice, displayed more normal growth and development compared with a normal breast from wild-type mouse (Figure 3D, b-c). Using the Kaplan-Meier method to estimate cumulative survival rates for 'treated' verse 'untreated' MMTV-PymT transgenic mice, we found that the mT Ab therapy could prolong the life-span of treated mice, leading an increased in the median survival for 'treated' MMTV-PymT mice to 19.5-week compared to 15 weeks for untreated mice (Figure 3E,*p*=0.0018). In all, we found that 100% (26/26) of 'untreated' mice carried dramatic breast tumors, while only 16.6% (3/18) of mT antibody treated mice developed tumor-bearing breasts, with the rest showing significant reduction in the formation of metastatic breast tumors that express mT oncogene (Figure 3F, and Figure 5F). These results suggested that the extensive repression of spontaneous tumor formation could be achieved by treating MMTV-PymT mice with mT antibody alone.

### Example 16. Results (Section E1): C57BL6 mice vaccinated with PRL-3 protein or EGFP resisted the formation of PRL-3- or EGFP-expressing tumors

Having successfully demonstrated how three distinct intracellular proteins (PRL-3, EGFP, and mT) could be targeted with their respective antibodies, we next asked if intracellular proteins could be targeted by natural antibodies generated by the immune response upon antigen challenge (vaccination). We proceeded to immunize 8-week old C57BL6 mice with three doses (20 µg each) of PRL-3 antigen (n=16) or EGFP protein (n=14) at 2-week intervals (Figure 6A). At the end of the immunization, sera from 14-week old immunized C57B16 mice were tested for their antibody titers against PRL-3 antigen or EGFP protein by ELISA (data not shown). Successfully immunized mice were subsequently divided into two groups, i.v. injected with either EGFP-F0 (PRL-3 positive) or EGFP-F10 (PRL-3 negative) melanoma cells. Compared to un-immunized mice (n=18) (Figure 6B, a-d), PRL-3-immunized mice displayed reduced metastatic tumors formed by EGFP-F0 cancer cells in adrenal, ovary and lung (Figure 6B, e-g). This was expected as EGFP-F0 melanoma cells express both EGFP and PRL-3 proteins, which should respond to both EGFP and PRL-3 antibody therapies. Remarkably, compared to metastatic lung from unimmunized-mice (Figure 6B, d), the PRL-3-immunized mice were not able to reduce the degree of aggressiveness of metastatic lung tumors formed by EGFP-F10 melanoma cells (Figure 6B, h), which do not express PRL-3 protein (Figure 5A). Again, this suggested that antigen-induced, naturally produced PRL-3 antibodies have no impact on inhibiting non-PRL-3 expressing EGFP-F10 tumors. Elegantly, we showed that GFP-immunization could reduce metastatic tumors formed by both EGFP-F0 and EGFPF10 cancer cells in the adrenal gland, ovary and lung (Figure 6B, i-l) regardless of PRL-3 expressing levels. Taken together, these results suggested that the induction of anti-PRL-3 or anti-EGFP antibodies in the host immune system upon vaccination could specifically inhibit the formation of PRL-3- or EGFP-expressing tumors respectively.

### Example 17. Results (Section E1): MMTV-PymT transgenic young females vaccinated with mT antigen prevented the formation of mammary gland mT tumors

To further confirm the possibility of cancer vaccination with intracellular proteins; we again employed the MMTV-PymT transgenic mammary tumor model. 38 FVB/N heterozygous females (4-week old) were divided into GST-immunized (n=3), GST-mT-immunized (n=17), and uniminunized (n=18) control groups. Mice were subjected to immunize with three doses (10 µg each) of GST or GST-mT antigen respectively, at 2-week intervals (Figure 4A). Such mice were examined by ELISA for the titers of mT antibody to confirm the existence of mT antibody in their blood circulation (see Figure 4B for representative example). Comparing to GST-immunized mice (#43, #44, #45), remarkably, mT-immunized mice (#37, #40, #41) exhibited a dramatic reduction in size and weight of mammary gland tumors (Figure 4C-D), with the average weights of breast tissues/mouse from GST-immunized, GST-mT-immunized, and wild-type control mice found to be 5.6g, 1.3g, and 0.6g, respectively (Figure 4D). The results of the vaccination experiment are summarized in Figure 6C-D. Kaplan-Meier survival curves demonstrated that the mT antigen could prolong the mean survival rates (p=0.0004) for immunized mice (19.5-week) compared to the unimmunized group (14.5-week) (Figure 4E), indicating a positive benefit of cancer vaccination in extending survival time dramatically. Collectively, the data suggest that antigen-induced host mT antibody could again effectively prevent spontaneous breast tumor formations by cancer cells expressing mT.

### Example 18. Discussion (Section E1)

Despite advancements in extracellular protein targeting, intracellular protein targeting has received little attention in terms of antibody therapy. Our findings presented here document a unique proof-of-concept in which anti-cancer antibody therapy against intracellular proteins could dramatically reduce tumor progression *in vivo.*

Here, we demonstrated the efficacy of antibody therapy against two intracellular proteins (PRL-3 and EGFP) against murine melanoma metastases in wild type C57BL6 mice. Similar success was obtained against intracellular mT antigen in the MMTV-PymT transgenic mice spontaneous tumor model as well. The therapeutic response observed appears to be isotype-independent, as IgG1 (PRL-3 mAb), IgG2a (EGFP mAb), IgG2b (PymT rat mAb), and self-generated antibodies could effectively inhibit tumor progression. Proper antibody-antigen interactions seem to be the predominating factor; targeting tumors with non-related antibodies produced no beneficial response at all. Intriguingly, we also found that mature B-cells constitute an important component of the antibody therapy response. These results mirror a recent report in which B-cells were found to be essential to the anti-DR5 antibody therapy against colon adenocarcinoma²³.

Our results also demonstrated that upon tumor-specific antigen challenge, host immunity could be stimulated to produce endogenous antibodies against the specific antigen leading to tumor inhibition. This concept of 'cancer vaccination' has long been a promising but challenging prospect¹⁹. Importantly, we found that compared to exogenously delivered antibodies, *antigen-induced-antibody therapies* can achieve similar anti-tumor therapeutic efficacy. We believe that it may be more useful and economical as we naturally have huge potential flexibility to generate high titers of antigen-induced antibodies ourselves. Although using 'oncoproteins' for vaccination does not seem a practical thought, we do believe this approach is worth for future investigation. In order to preserve an oncogenic function in cancer cell signaling network, an 'oncoprotein' should precisely locate at its native subcellular context coordinated with its neighborhood partners for correct communication. When an 'oncoprotein' is isolated from its native dynamic complexity of sub-cellular localization, it may lose its connections and be unable to perform its normal biological roles.

The work presented here outlines a potential methodology for future clinical cancer treatments. First, primary tumors are removed and examined to identify of tumor-specific antigens. This is followed by the introduction of specific chimeric or humanized antibodies against the tumor antigen to eliminate disseminated cells, thus inhibiting micro-metastases formation²⁴. This step may well prevent further spreading or relapse in cancer patients. Alternatively, for cancers that are tightly genetically predisposed, immunization of immunocompetent young susceptible family members with an antigen that is associated with the familial cancer could 'prime' the immune system against that oncoprotein. These endogenously stimulated antibodies would be long-lasting, and would neutralize cancer cells expressing that particular oncoprotein. We base this approach on our observation that a very small amount of mT antigen (10 µg) introduced into a young MMTV-PymT transgenic mice could activate host immune system and produce mT antibody to inhibit mT-expressing tumor formation, allowing asymptomatic survival for up to 4-months. Extending weeks of life-span in mice may be comparable to prolonging decades in humans.

Ultimately, the pre-clinical data presented here suggests a general application for cancer treatments by using exogenous and endogenous antibody therapy targeting both extra- and intracellular tumor specific-antigens. Since existing conventional clinical antibody therapy is costly, we urge researchers to look at further developing the robust, *antigen-induced antibody therapy* response investigated here. For greater therapeutic response, we feel that this approach could be combined together with other immune-stimulatory agents for best effect. With cancer research rapidly moving towards individualized cancer therapy, our strategy for specific targeting of intracellular (or extracellular) oncoproteins hold enormous promise for tailored cancer therapy in the future.

### Example 19. References (Section E1)

1. K. Imai and A. Takaoka, Comparing Antibody and Small-Molecule Therapies for Cancer. Nat Rev Cancer. 6(9), 714-727 (2006).
2. J. Cohen and A. Wilson, New Challenges to Medicare Beneficiary Access to mAbs. mAbs. 1(1), 56-66 (2009).
3. M. Baker, Upping the Ante on Antibodies. Nat Biotechnol. 23(9), 1065-1072 (2005).
4. A. Ruiz-Arguelles and D. Alarcon-Segovia, Penetration of Autoantibodies into Living Cells. Isr Med Assoc J. 3(2), 121-126 (2001).
5. D. Alarcon-Segovia, A. Ruiz-Arguelles and E. Fishbein, Antibody to Nuclear Ribonucleoprotein Penetrates Live Human Mononuclear Cells through Fc Receptors. Nature. 271(5640), 67-69 (1978).
6. R. H. Diamond, D. E. Cressman, T. M. Laz, C. S. Abrams and R. Taub, Prl-1, a Unique Nuclear Protein Tyrosine Phosphatase, Affects Cell Growth. Mol Cell Biol. 14(6), 3752-3762 (1994).
7. Q. Zeng, W. Hong and Y. H. Tan, Mouse Prl-2 and Prl-3, Two Potentially Prenylated Protein Tyrosine Phosphatases Homologous to Prl-1. Biochem Biophys Res Comm. 244(2), 421-427 (1998).
8. A. Alonso, J. Sasin, N. Bottini, I. Friedberg, I. Friedberg, A. Osterman, A. Godzik, T. Hunter, J. Dixon and T. Mustelin, Protein Tyrosine Phosphatases in the Human Genome. Cell. 117(6), 699-711 (2004).
9. X. Si, Q. Zeng, C. H. Ng, W. Hong and C. J. Pallen, Interaction of Farnesylated Prl-2, a Protein-Tyrosine Phosphatase, with the Beta-Subunit of Geranylgeranyltransferase II. J Bio Chem. 276(35), 32875-32882 (2001).
10. J. Wang, C. E. Kirby and R. Herbst, The Tyrosine Phosphatase Prl-1 Localizes to the Endoplasmic Reticulum and the Mitotic Spindle and Is Required for Normal Mitosis. J Biol Chem. 277(48), 46659-46668 (2002).
11. Q. Zeng, X. Si, H. Horstmann, Y. Xu, W. Hong and C. J. Pallen, Prenylation-Dependent Association of Protein-Tyrosine Phosphatases Prl-1, -2, and -3 with the Plasma Membrane and the Early Endosome. JBiol Chem. 275(28), 21444-21452 (2000).
12. S. Saha, A. Bardelli, P. Buckhaults, V. E. Velculescu, C. Rago, B. St Croix, K. E. Romans, M. A. Choti, C. Lengauer, K. W. Kinzler and B. Vogelstein, A Phosphatase Associated with Metastasis of Colorectal Cancer. Science. 294(5545), 1343-1346 (2001).
13. H. Wang, L. A. Vardy, C. P. Tan, J. M. Loo, K. Guo, J. Li, S. G. Lim, J. Zhou, W. J. Chng, S. B. Ng, H. X. Li and Q. Zeng, Pcbp1 Suppresses the Translation of Metastasis-Associated Prl-3 Phosphatase. Cancer Cell. 18(1), 52-62 (2010).
14. J. A. Sager, S. Benvenuti and A. Bardelli, Prl-3: A Phosphatase for Metastasis? Cancer Biol Ther. 3(10), 952-953 (2004).
15. S. M. Dilworth, Polyoma Virus Middle T Antigen and Its Role in Identifying Cancer-Related Molecules. Nat Rev Cancer. 2(12), 951-956 (2002).
16. C. T. Guy, R. D. Cardiff and W. J. Muller, Induction of Mammary Tumors by Expression of Polyomavirus Middle T Oncogene: A Transgenic Mouse Model for Metastatic Disease. Mol Cell Biol. 12(3), 954-961 (1992).
17. Y. Husemann, J. B. Geigl, F. Schubert, P. Musiani, M. Meyer, E. Burghart, G. Forni, R. Eils, T. Fehm, G. Riethmuller and C. A. Klein, Systemic Spread Is an Early Step in Breast Cancer. Cancer Cell. 13(1), 58-68 (2008).
18. E. Gilboa, The Promise of Cancer Vaccines. Nat Rev Cancer. 4(5), 401-411 (2004).
19. K. Guo, J. P. Tang, C. P. B. Tan, H. Wang and Q. Zeng, Monoclonal Antibodies Target Intracellular Prl Phosphatases to Inhibit Cancer Metastases in Mice. Cancer Biol Ther. 7(5), 750-757 (2008).
20. T. von der Weid, N. Honarvar and J. Langhorne, Gene-Targeted Mice Lacking B Cells Are Unable to Eliminate a Blood Stage Malaria Infection. J Immunol. 156(7), 2510-2516 (1996).
21. Y. Shinkai, G. Rathbun; K. P. Lam, E. M. Oltz, V. Stewart, M. Mendelsohn, J. Charron, M. Datta, F. Young and A. M. Stall, Rag-2-Deficient Mice Lack Mature Lymphocytes Owing to Inability to Initiate V(D)J Rearrangement. Cell. 68(5), 855-867 (1992).
22. J. Li, K. Guo, V. W. C. Koh, J. P. Tang, B. Q. Gan, H. Shi, H. X. Li and Q. Zeng, Generation of Prl-3- and Prl-1-Specific Monoclonal Antibodies as Potential Diagnostic Markers for Cancer Metastases. Clin Cancer Res. 11(6), 2195-2204 (2005).
23. Haynes NM, Hawkins ED, Li M, McLaughlin NM, Hammerling GJ, Schwendener R, Winoto A, Wensky A, Yagita H, Takeda K, Kershaw MH, Darcy PK, Smyth MJ. CD1 1c+ dendritic cells and B cells contribute to the tumoricidal activity of anti-DR5 antibody therapy in established tumors. J Immunol. 185(1):532-41 (2010).
24. B. Weigelt, J. L. Peterse and L. J. van 't Veer, Breast Cancer Metastasis: Markers and Models. Nat Rev Cancer. 5(8), 591-602 (2005).

### Examples Section E2 (Examples 20 to 38). Host Immunity is Crucial for the Anticancer Efficacy of a Chimeric Antibody Targeting Intracellular PRL-3 in Animal Models

Section E2 comprises Examples 20 to 38. Example 20 is an Introduction to Section E2, Examples 21 to 29 are Materials and Methods for Section E2, Examples 30 to 36 are Results for Section E2, Example 37 is a Discussion of Section E2, Example 38 is References for Section E2.

### Example 20. Introduction (Section E2)

A century ago, the German chemist Paul Ehrlich proposed the concept of antibodies as "magic bullets". Indeed, monoclonal antibodies (mAbs) have proven to be nature's biological warheads against some of humanity's most deadly diseases^{1,2}. In general, antibody targeted therapy has much less toxicity than chemotherapy with small molecule inhibitors. Antibodies constitute the most rapidly growing class of human therapeutics and are ideal agents for recognizing and destroying malignant cells via the immune system. However, this therapeutic approach has been limited to surface or secreted proteins expressed by cancer cells^{3,4}, in part due to the assumption that antibodies are too large (150 kDa) to penetrate the cell membrane. As a consequence, a wide spectrum of intracellular oncoproteins remains unexplored in terms of an antibody therapy approach. However, the concept that intact antibodies are unable to penetrate into viable cells has been challenged by a plethora of experimental findings and clinical observations^{5,6,7}. Over the past 30 years, immunologists have found that autoantibodies found in the serum of patients with different autoimmune diseases can somehow bind their respective intracellular antigens^{6,7,8}. Although it is not certain that the antibodies to intracellular proteins that are found in autoimmune patients actually cause the disease, or even cell destruction, we nevertheless emphasize our main contribution of this work is that antibodies to intracellular protein can exert therapeutic effects.

PRL-1. (phosphatase of regenerating liver-1), PRL-2, and PRL-3 represent an intriguing subgroup of the intracellular protein tyrosine phosphatases (PTP). Individual PRLs are overexpressed in a variety of cancer cell lines and cancer tissues when compared with their normal counterparts⁹. A recent important review well describes these PRL-PTPs in cancer progression¹⁰. PRLs are intracellular C-terminally prenylated phosphatases. The localization of PRL-1 and PRL-3 to the inner leaflet of the plasma membrane and early endosomes was revealed by EM immunogold labeling^{11,12}. In contrast, the mutant forms of PRLs that lack the prenylation signal are localized in the nuclei¹³. PRL-3 was first discovered as a metastasis-associate phosphatase linked to colorectal cancer (CRC) metastasis with the finding that it was the only gene overexpressed in 100% of liver metastasis of CRC¹⁴. Overexpression of PRLs has subsequently shown to have a causative role in promoting cancer metastases and they become potential unique targets for diverse cancer treatment¹⁵. However, as these phosphatases are intracellularly localized, the conventional approach using therapeutic antibodies would seem a daunting task. In our earlier study, we reported an unexpected observation that mouse monoclonal antibodies (mAbs) against PRL-1 and PRL-3 were able to block experimental metastasis of cancer cells overexpressing intracellular EGFP-tagged PRL-1 and PRL-3¹⁶.

In this study, we further evaluate the reliability of such targeting strategy using a newly-generated chimeric PRL-3 antibody. Here we extend our earlier findings for potential future clinical therapeutics against intracellular oncoproteins in five important aspects. Firstly, we generated and utilized clinically-relevant chimeric antibodies instead of mouse antibodies. Secondly, we treated mice harboring naturally-occurring human cancer cells that express endogenous PRL-3 instead of exogenous PRL-3 in Chinese hamster cells (CHO). Thirdly, we showed that depletion of nature killer (NK) cells enhances tumor engraftment. Fourthly, using paired *nude* and *scid* mouse models, we discovered the crucial role of B-cells in determining the outcome of our antibody therapy. Finally, using fluorescent labeled antibodies to track antibody-tumor binding activities by IVIS live imaging system; we proposed two working models for the antibody therapy in treated and untreated mice. An evidence-based hitherto concept is proposed for a possible approach in targeting intracellular oncoproteins with antibody therapies. The results suggest that an evaluation of a wide spectrum of intracellular oncoproteins (such as phosphatases, kinases, transcription factors) as possible targets for anticancer therapy may be warranted.

### Example 21. Materials and Methods (Section E2): Generation of specific PRL-3 human/mouse chimeric mAb (clone #318)

For PRL-3 chimeric mAb generation, total RNA was extracted from 6x10⁶ hybridoma cells (clone#318)¹⁹ using the RNeasy Mini Kit (QIAGEN, cat#74104). The RNAs were then reverse-transcribed into cDNA using SuperScript II RNase H (Invitrogen, Cat 18064-014). The resulting total cDNAs were used as templates to generate the 'universal variable region' using Ig-Prime Kits (Novagen, cat#69831-3) for PCR (95⁰C-4⁰C-72⁰C, 30 cycles). The PCR fragment was cloned into the PCRII-TOPO-Vector with a TA cloning kit (Invitrogen, part#45-0640). The PCR fragment was cut with Mfe1 and Xho1, and then inserted into the respective sites of a human IgG1 constant region expression vector-pCMV-human IgG1¹⁷ to join the mouse variable region of heavy chain (clone #318)¹⁸ with the human IgG1 constant region. Similar PCR procedures were performed for the mouse variable region of the light chain (clone #318) with ends containing restriction sites for ApaL1 and Pst 1. The PCR fragment was cut with ApaLI and Pst I and then inserted into the respective sites of a human IgG1 constant region expression vector containing the variable region of the heavy chain of clone #318. The complete construct was transiently transfected into 293T cells cultured in ultra-low IgG FBS (Gibco, 16250-078). The chimeric mAb was subsequently harvested from the culture supernatant and concentrated up to 40 times with centrifugal filter devices (Millipore, cat#UFC900596). The chimeric mAb was tested for its specificity by indirect immunofluorescence (IF) and Western blot analysis.

### Example 22. Materials and Methods (Section E2): Cell lines and cell culture

HCT116 (CCL-247) human colorectal carcinoma cell line, H460 (NCI-H460) human non-small lung cancer cell line, A431 (CRL-1555) human epidermoid carcinoma cell line, B16F0 (CRL-6322), and B16F10 (CRL - 6475) mouse melanoma cell lines were purchased from the American Type Culture Collection (ATCC, Manassas, VA). A2780 (Cat#93112519) human ovarian cancer cell line was purchased from ECACC, UK. Cells were grown in appropriate media recommended by the suppliers.

### Example 23. Materials and Methods (Section E2): Western blot analysis

Generation of mouse PRL-3 monoclonal antibody and Western blot procedures have been described previously¹⁸. GAPDH antibody was from Cell Signaling Technology (Beverly, MA).

### Example 24. Materials and Methods (Section E2): Experimental Metastatic Assay in mice20

1x10⁶ cancer cells were injected into the circulation of eight-week old *nude* mice (Jackson Labs, USA) via the tail vein on day 1. Either chimeric PRL-3 mAb or mouse PRL-3 or PRL-1 mAbs was used to treat mice; the first antibody treatment was carried on day 3 post-cancer cell injection, followed by two administrations per week. For control untreated group, PBS was administrated via tail vein. All animal studies were approved by the Institutional Review Board of the IMCB, in strict compliance with rules and policies of the Animal Facility Center of The Agency for Science, Technology and Research (A* STAR), Singapore.

### Example 25. Materials and Methods (Section E2): Depletion of NK cells

Anti-asialo GM1 anti-serum (rabbit) was purchased from Wako Pure Chemical Industries, Ltd (Osaka 540-8605, Japan). The GM1 anti-serum (50 µl) was injected into the circulation of eight-week old *nude* mice (Jackson Labs, USA) via the tail vein 24 h before the experimental metastasis assay.

### Example 26. Materials and Methods (Section E2): Statistical analysis

The Kaplan-Meier method was used to estimate cumulative survival rates, and differences in survival rates. *p*-values less than 0.01 were considered to be significant.

### Example 27. Materials and Methods (Section E2): Antibody labeling and IVIS live imaging

Purified PRL-3 antibody was labeled with CF™750 Dye Antibody Labeling Kits (http://vww.biotium.com/product/product info/Newproduct/Mix-n-Stain Kits.asp). Labeled antibodies were injected via tail vein 1 hr before live imaging. Bioware Ultra Cell Line HCT 116-*luc2* (www.caliperLS.com) is a luciferase expressing cell line which was stably transfected with firefly luciferase gene *(luc2)* under the human ubiquitin C promoter. HCT116-Luc2 cell line was established by using HCT116 human adenocarcinoma (ATCC, CCL-247™) and transducing lentivirus containing luciferase 2 gene under the control of human ubiquitin C promoter (pGL4 luc2). 1x10⁶ HCT 116*-luc2* cancer cells were injected into tail veins of 8-week old nude mice (Jackson Labs, USA). Antibody was injected into treated mice via tail veins on day 3, follow by two antibody injections per week. After 7-week treatment, mice are injected by an intraperitoneal route with a luciferin solution (15 mg/ml or 30 mg/kg, in PBS, dose of 150 mg/kg) that is allowed to distribute in awaked animals for about 5-15 minutes. The mice are placed into a clear plexiglass anesthesia box (2.5-3.5 % isofluorane) that allows unimpeded visual monitoring of the animals using IVIS® Spectrum Imaging System 3D Series to track and monitor tumor development in vivo. The results between treated verse untreated mice were determined.

### Example 28. Materials and Methods (Section E2): FACS analysis

The human epidermoid carcinoma cell line A431 was grown in DMEM with high glucose (4.5g/L), supplemented with 10 % FBS and 5 % antibiotic. B16F0 and B16F10 cells were grown in RPMI, supplemented with 10 %PBS and 5 % antibiotics. Cells (5X10⁶) were dislodged from the dishes with non-enzymatic pre-warmed cell dissociation solution (Sigma, Cat# c-5914) and transferred to 5 ml polystyrene tubes and washed once with complete medium. The cells were then incubated with 1 µl EGFR (Genetech, USA) or 5 µl PRL-3 primary mouse antibodies¹⁸ in 100 µl of complete medium for 1 hr at room temperature (RT). Cells were agitated every 15 mins to prevent clumping. Cells were then washed 2x with complete medium and incubated for 1 hr at RT with goat anti-mouse AlexaFluor 546 antibody (Invitrogen, USA), washed, and re-suspended in 1 ml complete medium prior to analysis using BD FACS Caliber. Raw data was processed using WinMDI ver2.8 software.

### Example 29. Materials and Methods (Section E2): Histopathologic Analyses Using Immunohistochemistry (IHC)

Human lung tissue arrays 009-01-004; and CC00-01-006; CC04-01-CC04 were purchased from Cybrdi, Inc. (Rockville, Maryland 20850 USA: http://cybrdi.com/index.php). Human AML bone marrow samples were obtained from the National University Hospital-National University of Singapore (NUH-NUS) Tissue Repository with approval of the Institutional Review Board (IRB) of NUH-NUS for research uses. The use of all human tissue samples including commercial samples were approved by Institutional Review Board (IRB) of the Institute of Molecular and Cell Biology. We used Dako EnVision™ Systems K 1395 (Dako, Carpinteria, CA) to perform IHC analysis^{18,19}.

### Example 30. Results (Section E2): Generation of PRL-3 mouse/human chimeric antibodies (clone #318)

We previously reported that PRL-3 or PRL-1 mouse mAbs could specifically target their respective intracellular PRL-3 or PRL-1 phosphatase to inhibit cancer metastases in *nude* mice¹⁶. In an attempt to translate our laboratory findings to clinical setting, we have engineered a mouse/human chimeric mAb against PRL-3 to minimize the potential antigenicity of the mouse mAb in human. Using recombinant DNA technology, we separately fused the constant domains of heavy or light chains of the human IgG1 molecule¹⁷ with the mouse variable regions of PRL-3 mAb(clone#318)¹⁸ by transgenic fusion of the immunoglobulin genes (Figure 7A). The expression construct was transfected into Human Embryonic Kidney 293T cells to produce recombinant PRL-3 chimeric mAb that was then harvested from the culture medium and further concentrated. The antigen-binding specificity of the PRL-3 chimeric mAb was well conserved as confirmed by performing indirect immuofluorescence on DLD-1 cells that overexpress exogenous EGFP-PRL-3 (Figure 7B) and western blot analyses (Figure 7C). The PRL-3 chimeric mAb specifically recognized EGFP-PRL-3 (∼48 kDa) and myc-PRL-3 (∼21 kDa) (Figure 7C, lane 1-2) but react with neither myc-PRL-1 nor myc-PRL-2 proteins (Figure 7C, lane 3-4). A 50% cell Inhibitory Cytotoxic concentration (IC50) was carried out in a mouse melanoma B16F0 cells for the chimeric antibody and we observed no cellular toxicity in viability even at high concentrations (40 µg/ml) when cells were cultured in 10% FBS medium under normal culture conditions (data not shown). Since PRL-1, -2, and -3 are overexpressed in a broad range of human cancers¹⁹, we anticipate that the PRL-antibodies are likely to have broad applications to block different types of PRLs-positive cancer spreading, especially in some lethal malignancies such as lung cancers and acute myeloid leukemia_(AML) that often relapse within short timeframes. Amongst lung cancers, we found PRL-3 is overexpressed in 31% of squamous cell carcinoma and 26% of adenocarcinoma (Figure 1A-C), two main subtypes of highly recurrent non-small cell lung carcinoma comprising 80% of human lung cancer (http://en.wikipedia.org/wiki/Lung cancer#Non-small cell lung carcinoma .28NSCLC.29). We also found that PRL-3 is overexpressed in 35% (24 out of 69 cases) of AML (Figure 12D). A few injections of PRL-3 chimeric antibody therapies will clean up the leftover of micro-metastasis and circulating cancer cells after the surgery of PRL-3-positive cancers to prevent the recurrence.

### Example 31. Results (Section E2): PRL-3 chimeric antibody effectively inhibits the metastatic tumors formed by B16F0 cancer cells that express endogenous PRL-3 but not by B16F10 cancer cells that do not express endogenous PRL-3

To find a clinically relevant animal model for PRL-3-associated cancers, we screened dozens of cancer cell lines for the expression of endogenous PRL-3 protein levels by Western blot analysis. Ideal cell line pairs for our animal models should present contrasting levels of endogenous PRL-3 and should have the ability to induce metastatic tumors in mice within short timeframes. We found two mouse melanoma cell lines B16F0 and B16F10 (F0 and F10) that could fulfill the criteria for the desired experiment. Although F10 cells are naturally more metastatic than F0 cells, we found that parental F0 cells express higher levels of endogenous PRL-3 protein than F10 cells (Figure 8A), suggesting that F10 cell metastatic activity might be no longer PRL-3 dependent. When we employed an experimental metastatic assay²⁰ in which cultured cancer cells were introduced into the circulation of nude mice by lateral tail vein injection, both F0 and F10 cell lines can rapidly form multiple metastatic tumors in mice within 17 days. Such aggressive *in vivo* metastasis models allow us to see the differences in efficacy shortly after antibody therapy between treated and untreated groups. On day 3 (the latest time we can delay in treatment) post cancer cell injections, chimeric-PRL-3 antibodies were administrated similarly via tail veins into the 'treated mice', followed by two subsequent administrations of the antibody per week (Figure 8B). At the end of the experiment, we found that the PRL-3 chimeric antibody: could eradicate tumors formed by PRL-3 expressing F0 cancer cells; the metastatic tumors in multiple tissues were dramatically reduced in 'treated mice' at the end of the experiments (Figure 8C). The Kaplan-Meier survival analysis for F0 recipients will discuss later (Figure 10A, c). In a parallel experiment, the PRL-3 antibody had no effect in blocking metastatic tumors formed by F10 cancer cells that do not express PRL-3 protein. Hundreds of metastatic tumors were found in the lungs of both treated and untreated F10 cell recipients, and no obvious difference was seen between 'untreated mice' (Figure 8D, upper panel) and 'treated mice' (Figure 8D, lower panel). Furthermore, Kaplan-Meier survival analysis demonstrated that the PRL-3 antibody could not extend the survival time for 'treated' F10 recipients (Figure 8E).

### Example 32. Results (Section E2): PRL-3 chimeric antibodies effectively inhibit the formation of metastatic tumors formed by human cancer cells that express endogenous PRL-3, but not by cancer cells that do not express endogenous PRL-3

In addition to mouse F0 melanoma cells, we further found that HCT116-luc2, HCT-116 human colorectal cancer cell line, and A2780 human ovarian cancer cell line express endogenous PRL-3 protein (Figure 9A lanes: 1-3). A2780 has also been reported as a PRL-3 positive cell line previously²¹. As a control, we found a human non-small lung cancer cell line (NCI-H460) which does not express endogenous levels of PRL-3 (Figure 9A lane 4). Regardless PRL-3 positive or negative, the four human cancer cell lines can rapidly form metastatic tumors in *nude* mice within 1-2 months respectively. HCT-116-luc2 is a luciferase expressing cell line which was stably transfected with firefly luciferase gene (luc2) in HCT-116 cells. The cell line was established by transducing lentivirus containing luciferase 2 gene under the control of human ubiquitin C promoter. This cell line can be used in vivo by Xenogen's IVIS® Spectrum Series imaging to monitor tumor formation. Using this system, we demonstrated that the PRL-3 antibody could inhibit metastasis of HCT116-luc2 cancer cells as a clear reduction of metastatic lung tumors in live imaging at 7 week of antibody therapy was observed (Figure 9B). Furthermore, significant differences were found between PRL-3 antibody treated and untreated mice at 2-month post-inoculation with HCT-116 cells (Figure 9C, a) and at 1-month post-inoculation with A2780 cells (Figure 9C, b). The PRL-3 mAb-treated animals appeared vibrant and healthy (up to 4-month), whereas the untreated mice had all lost weight and were moribund. In paralleled, NCI-H460 (PRL-3 negative cells) recipients did not respond to PRL-3 antibody treatment (Figure 9C, c). Taken together, these results further support the conclusion that the efficiency of the PRL-3 antibody treatment is tightly correlated with PRL-3 expression status of the cancer cells. We showed that the efficiency of both mouse- and chimeric-PRL-3 antibody therapies is comparable (Figure 9D).

### Example 33. Results (Section E2): NK cells are important in anticancer therapy

We then investigated if nature killer (NK) cells play a role in the PRL-3 antibody therapy since NK cells are a type of cytotoxic lymphocyte that constitute a major component of the innate immune system. To deplete the *nude* mice's NK cells, we pre-injected *nude* mice with anti-asialo GM-1 antibody²². Our above-mentioned procedures of the antibody therapy were performed in these GM1-injected *nude* mice. We found that the efficacy of therapies was essentially lost in GM-1 injected mice (Figure 13). Even worse, such GM1 injected *nude* mice showed more severe tumors (in black)-bearing burden in lung, liver, adrenal, testis, and bone than un-injected ones, indicating that innate immune system is important in our antibody therapy. NK cells have been demonstrated to have a role in human hematopoietic stem cell graft rejection²², removal of NK cells may result in abolishment of graft rejection of NK cell activities, leading tumor engraftment more successfully, therefore, GM1 injected 'PRL-3-treated' mice were worse than GM1-un-injected 'PRL-3-untreated' mice in terms of tumor growth.

### Example 34. Results (Section E2): B-cells may be important in mediating the therapeutic effects of PRL-3 antibodies

Thus far, we performed the PRL-3 antibody therapies in T-cell deficient *nude* mice. To address if B lymphocytes are critical in our antibody therapy model, we next performed antibody therapies in Severe Combined Immunodeficiency *(scid)* mice, which lack functional lymphocytes because they carry a deficiency that impairs rearrangement of separate gene elements of the immunoglobulin and T-cell antigen receptor genes, thus, disrupting the differentiation and maturation of both B- and T-lymphocyte progenitor cells. They thus show a severe combined immunodeficiency affecting both B and T lymphocytes, although they have normal NK cells, macrophages, and granulocytes. For HCT-116-induced metastatic tumors, Kaplan-Meier analysis of survival curves of PRL-3 antibody treated versus untreated mice showed a statistically significant (P <0.001) increase in life span of *nude* mice but not *scid* mice (Figure 10A, a-b). Intriguingly, we found a 45% increase in the median survival time for the treated HCT-116-injected *nude* mice (16 weeks) compared to untreated mice (11 weeks). In contrast, in untreated or treated groups of HCT-116-injected *scid* mice, we observed no difference in the effect of PRL-3 antibody therapy, which showed similar median survival duration (11 weeks). Furthermore, we employed F0 PRL-3 positive cancer cell line to confirm this finding using similar strategies. In F0-injected *nude* mice (Figure 10A, c-d), PRL-3 antibody treatment increased median survival duration by 47% (24 days for treated, versus 17 days for untreated). Consistently, the antibody had no effect in untreated or treated F0-injected *scid* mice, with both groups showing similar median life-span (∼17 days). The mechanism is independent of antibody species, as similar results were obtained using mouse or chimeric PRL-3 antibodies (Figure 10B, a-d). Collectively, the results from these genetic mouse models suggest that PRL-3 antibody efficacy against metastatic tumor formation is dependent on host B-cell but not T cells. We suggest that other than antibody production, B-cell may have additional function to secrete unknown factor(s) that could facilitate the actions of antibodies. Indeed, our preliminary data shows that B-cells may secrete factors that could facilitate antibody uptake by the cancer cells. We observed a substantial increase in the internalization of antibody in PRL-3 positive F0 cells when the assay was performed in presence of culture supernatant from human B cells. Parallel, PRL-3 null F10 cells were unable to harbor any antibody either in presence of the B cell conditioned media or in presence of live B cells (data not shown).

### Example 35. Results (Section E2): PRL-3 antigen is insignificant at the cell surface

A possible mechanism of PRL-3 antibody action could be the binding of a cell surface antigen, triggering a B-cell dependent elimination of the PRL-3 expressing cell. However, to date, no reports describe a cell surface localization of PRL-3. To address the possibility of PRL-3 antibody binding its antigen on the cell surface, we used a FACS assay routinely used in cell surface labeling. As a positive control, anti-EGFR antibody binding of the EGFR-overexpressing human epidermoid carcinoma A431 cell line was used for this assay. We found that incubation of A431 cells with anti-EGFR antibodies caused a distinct peak-shift in the FACS assay (Figure 11A, a). However, no peak shift was observed for either F0 (PRL-3 positive) or F10 (PRL-3 negative) cells incubated with or without anti-PRL-3 antibody (Figure 11A, b-c). These results imply that cell surface PRL-3 antigen, if any, was unlikely to be the major cause of antibody binding and uptake. However, since immune system constantly battles invaders, such as bacteria and viruses, and cancer cells *in vivo,* the cancer cells can be destructed and somehow expose their intracellular protein to immune system. If this is so, the same possibility may apply to other intracellular oncoproteins as well.

### Example 36. Results (Section E2): IVIS live imaging-based working models

In our animal model systems, we found that antibody therapies were ineffective after 3-days post cancer cells injection, suggesting that the first 3-day may be sufficient for cancer cell engraftment in new tissues. Using IVIS system to track sites of metastatic tumors through labeled antibody-antigen binding, we herein proposed two working models (Figure 11B). a. Treated mice. 1. at early stages, cancer cells were in clusters, timely introduction of the antibodies could somehow capture antigen-specific cancer cells in the circulatory, or in the lymphatic system together with the help of lymphocytes (such as B, and NK cells) via innate immune system to destroy and remove these cancer cells. 2. Escaped cancer cells will be able to arrive at new organs for implantations; therefore, the constant delivery of the antibody to the circulation is important to unplug these incipient tumors and prevent them from further development, destructing and clearing them from the host immune system. 3. The incipient tumors in 'treated mice' have no chance to develop tumors fully and therefore were in open-stages exposed to immune system. In addition, we found higher blood vessel density in small tumors (H&E lung sections) than in large tumors. As such, fluorescent labeled antibodies could easily access via blood circulation to metastatic lung tumors in 'treated mice'. We therefore observed the 'treated' metastatic lungs with strong florescent labeled. b. Untreated mice. The above-mentioned early steps had been missing; the unchecked cancer cells were rapidly multiplied in the host. The massive cancer cells were uncontrolled implanted and had sufficient time to develop micro- to macro-metastases, building up their territory with tumor boundaries 'fences' as defense system with close-stages away from the immune system. Furthermore, we found lower blood vessel density in large areas of tumor, insufficient blood supply results in necrosis (not shown). As consequences, fluorescent labeled antibodies were less accessible via blood supply to reach metastatic lung tumors in 'untreated mice'. We therefore found the 'untreated' metastatic lungs with weak florescent labeled.

### Example 30. Discussion (Section E2)

PRL-3 is up-regulated in numerous types of human cancer. It is foreseeable that the PRL-3 chimeric antibody will be a promising therapeutic agent in blocking diverse PRL-3-associated cancer metastases. We hope to start with our effort on a few lethal malignancies (such as lung cancer and AML) that often relapse within short timeframes. Since these cancers are aggressive, therapeutic effects are easily observed and the outcomes between treated and untreated patients can be clearly defined. Especially, leukemic cells are easily accessible and are in direct contact with antibodies in the circulating system. The terminal patients suffering from these aggressive cancers are more likely to be recruited into clinical trials. Hopefully, success in these examples may make an impact on targeting other intracellular oncoproteins with antibody therapies, which is an important area of translational research and will provide a viable alternative treatment to cancer patients.

The molecular mechanism(s) behind these unconventional findings presented here remains elusive and is actively being investigated. Currently, understanding how antibody destroys tumors *in vivo* is still a 'dark box' to us. Similarly, it has been more than two decades to aim at understanding the mechanism(s) of the well-know anti-HER2/neu antibody therapy, although the anti-HER2/neu antibody has been in clinical uses for years in treating breast cancers²³. Anticipating unraveling the 'dark box' using *in vitro* system may be unrealistic because a major consideration of traditional *in vitro* cell culture system is far simplified due to the limitation of single cell type grown in an incubator, typically using medium supplemented with 10% fetal bovine serum, lack of immune system involved, which was unlikely to be able to actually mimic *in vivo* complexities of multiple types of cells and organs, in 100% native blood circulation and lymphatic system. Whatever mechanism is, the central part of this study is that antibodies to intracellular protein can exert therapeutic effects, and more importantly, the effect is reproducible. If we can see the same therapeutic effect in humans that could be of enormous value in cancer treatment to support for the under-recognized notion that antibody therapy can be used for targeting intracellular antigens.

Nevertheless, our hard attempts in understanding the possible events within the 'dark box' enable us to achieve several conclusions from this study: **1. The PRL-3 antibody treatment is tightly correlated with PRL-3 expression status of the cancer cells.** We demonstrate that the chimeric antibody could indeed successfully block the formation of metastatic tumors derived from several cancer cell lines (B16F0, HCT-116, and A2780) that express endogenous intracellular PRL-3 phosphatase. The inhibition is specific as the antibody had no effect in blocking the formation of metastatic tumors derived from other cancer cell lines (B16F10, H460) that do not express PRL-3. Previously, we had demonstrated that mouse PRL-3 antibody had no effect in inhibiting metastatic lung tumors formed by CT26 mouse colon cells, another PRL-3 negative cancer cell line¹⁶. Taken together, the data support the notion that the efficiency of either mouse or chimeric PRL-3 antibody treatment is tightly correlated with PRL-3 expression status of the cancer cells. If the metastatic property of cancer cells was not due to PRL-3 overexpression (such as B16F10, H460, CT26 cells), the administration of PRL-3 mAb has no effect in blocking tumors formed by these PRL-3 negative cells. Furthermore, we previously¹⁶ had shown that PRL-1 mAb specifically blocks PRL-1 (but not PRL-3) metastatic tumors; while PRL-3 mAb specifically blocks PRL-3 (but not PRL-1) metastatic tumors though PRL-1 and PRL-3 share very high homology in protein sequence. These results imply that PRL antibody therapy is highly specific to its antigen and does not involve cross-reactivity with other non-specific cell surface proteins. **2. NK cells in 'innate immune system' are involved in the therapy.** To understand if innate immune system is involved in the antibody therapy, we intravenously injected GM1 antibody via tail vein into *nude* mice to deplete NK cells, which are a type of cytotoxic lymphocytes that constitute a major component of the innate immune system. In the absence of NK cells, we found, anti-PRL-3 antibody did not exhibit therapeutic effects. Furthermore, tumor-engraftment was dramatically enhanced (Figure 13), indicating that NK cells normally play a critical role in graft-rejection for implantation of foreign cells. Previously, mounting evidence suggests that NK cells play an important part in the destruction of incipient tumours²⁴. **3. ADCC may also be involved in the therapy.** The best characterized mechanism of antibody therapy is antibody-dependent cellular cytotoxicity (ADCC). In ADCC, antibodies bind to specific cell surface antigens and trigger an Fc-mediated immune response involving cytotoxic CD8 T cells, complement activation, and/or NK cell activity. Although we did not observe any peak-shift in our FACS analysis of PRL-3 cell surface antigens in both PRL-3 positive F0 cells and PRL-3 negative F10 cells, we could not rule out a possibility that these cancer cells were under abnormal inflammatory pressure, which may cause the destruction of cancer cells to release and expose their intracellular proteins to be attacked by the antibody and somehow trigger specific immune response, leading lymphocyte to remove these cancer cells. Alternatively, *in vivo,* cancer cells are under hypoxic stress and serum deprivation, conditions that arrest cells at G₁ and Go phrases²⁵. It is possible that these conditions may cause release of intracellular antigens for the antibody to recognize. If so, other intracellular oncoproteins may also encounter the similar situations, they therefore can be similarly targeted with antibody therapies. **4. 'Adaptive immune system' (IgM and/or B Cells) are important in the mAb therapeutic effect.** We employed athymic *nude* mice and immunodefficient *scid* mice for the tumour reduction experiments and achieved the therapeutic effect only in *nude* mice but not in *scid* mice. The major differences between *nude* and *scid* mice are that *nudes* are T-cell deficient, but have functional IgM antibodies and B cells, while *scids* have no functional adaptive immune system including B-, T-cells, and IgM and other antibodies. Both *nudes* and *scids* have intact innate immune system including NK cell and complement activity, but with positive responses seen only in the *nude* (but not in *scid*) mice, indicate that mature B cells (but not T cell), and possibly IgM/serum antibodies, are important. As the antibodies were exogenously introduced into mice, the requirement of B-cells for anti-metastatic PRL-3 antibody activity is hypothesized that an alternative role for B-cell in the antibody response, possibly via secretion of unidentified factor(s) that modulates any given antibodies for the host response. Collectively, we emphasize that intricate interplay of innate and acquired immune system is crucial for the anticancer efficacy of a chimeric antibody targeting intracellular PRL-3 oncoprotein. **5. NOT all intracellular oncoproteins can be targeted with antibody therapy** Desirable anti-cancer therapeutic agents should specifically target cancer cells while leaving normal tissues unharmed. It should be emphasized that the PRL-3 chimeric antibody therapy has little detectable side effect in *nude* mice since PRL-3 expression in normal tissues is not ubiquitous. We showed that PRL-3 protein was detected in only a few organs such as the spleen, brain, and pancreas (Figure 14A). In contrast, PRL-2 is ubiquitously expressed in most of mouse tissues (Figure 14B). As expected, PRL-2 antibody therapy to PRL-2 expressing cancers was unsuccessful (Figure 14C). The PRL-2 antibody-treated mice died 1-week earlier or showed worse outcomes than untreated mice, which might be due to PRL-2's ubiquitous expression in most of mouse tissues, implicating a possibility of PRL-2 antibody may attack normal tissues to cause this undesired side-effect. The results suggest that a good therapeutic target should be more specifically to tumor antigen without harming host normal tissues. 6. **Antibody therapy against intracellular oncoprotein is clinically relevant.** To generate a very aggressive cancer model for treatment, we directly injected 1 million cancer cells into the circulation of a *nude* mouse; the total blood volume in *nude* mouse is 8% of its body weight (8% of ∼19g = 1.5ml). The total blood volume in a man (50 kg) is around 4.7 liters. If 1 million cancer cells were injected into ∼1.5 ml blood circulation in nude mouse; which is comparable to 3133 (4.7 liter/1.5 ml) millions cancer cells in ∼ 4.7 liter blood in a man. The remarkable therapeutic effect by starting antibody (intravenously injected into tail vein) treatment even 3 days post-cancer cell injection indicates the treatment is intriguingly effective. If we can achieve similar life span extension in treated patients to that of mouse system, it will be comparable in years in humans in term of the total life span and the extended life expectancy. From these preclinical studies to reality, we anticipate that a few injections of chimeric antibody is very critical to reduce recurrence rate, as the PRL-3 antibodies will clean up circulating cancer cells²⁶ or unplugging invisible incipient tumors after surgical removal of visible PRL-3-associated solid tumors.

At this end, the lack of any observable side effect in *nude* mice upon PRL-3 antibody therapy further alludes to its potential clinical benefits. Our data prompts a reevaluation of a wide spectrum of tumor-specific intracellular oncoproteins as possible targets for anti-cancer mAb therapy, thus realizing the full potential of these 'magic bullet'.

### Example 38. References (Section E2)

1. D. Hanahan and R. A. Weinberg, The Hallmarks of Cancer. Cell. 100(1), 57-70 (2000).
2. J. M. Reichert, C. J. Rosensweig, L. B. Faden and M. C. Dewitz, Monoclonal Antibody Successes in the Clinic. Nat Biotechnol. 23(9), 1073-1078 (2005).
3. K. Imai and A. Takaoka, Comparing Antibody and Small-Molecule Therapies for Cancer. Nat Rev Cancer. 6(9), 714-727 (2006).
4. M. Baker, Upping the Ante on Antibodies. Nat Biotechnol. 23(9), 1065-1072 (2005).
5. Ruiz-Arguelles and D. Alarcon-Segovia, Penetration of Autoantibodies into Living Cells. Isr Med Assoc J. 3(2), 121-126 (2001).
6. D. AlarcoÂ n- Segovia, L. Llorente, A. Ruiz-Arguelles, Y. Richaud-Patin and B. Perez-Romano, Penetration of Anti-DNA Antibodies into Mononuclear Cells Causes Apoptosis. ArthritisRheum. 38, S179-182 (1995).
7. D. Portales-PeÂrez, D. AlarcoÂ n-Segovia, L. Llorente, R.-A. e. A, C. Abud-Mendoza, L. Baranda, H. De-la-Fuente, T. Ternyck, R. Gonzalez-Amaro and J., Penetrating Anti-DNA Monoclonal Antibodies Induce Activation of Human Peripheral Blood Mononuclear Cells. J Autoimmun. 11(5), 563-571 (1998).
8. T. D. Golan, A. E. Gharavi and K. B. Elkon, Penetration of Autoantibodies into Living Epithelial Cells. J Invest Dermatol. 100(3), 316-322 (1993).
9. B. J. Stephens, H. Han, V. Gokhale and D. D. Von Hoff, Prl Phosphatases as Potential Molecular Targets in Cancer. Mol Cancer Ther. 4(11), 1653-1661 (2005).
10. D. C. Bessette, D. Qiu and C. J. Pallen, Prl Ptps: Mediators and Markers of Cancer Progression. Cancer Metastasis Rev. 27(2), 231-252 (2008).
11. J. Wang, C. E. Kirby and R. Herbst, The Tyrosine Phosphatase Pr1-1 Localizes to the Endoplasmic Reticulum and the Mitotic Spindle and Is Required for Normal Mitosis. J Biol Chem. 277(48), 46659-46668 (2002).
12. Q. Zeng, X. Si, H. Horstmann, Y. Xu, W. Hong and C. J. Pallen, Prenylation-Dependent Association of Protein-Tyrosine Phosphatases Prl-1, -2, and -3 with the Plasma Membrane and the Early Endosome. J Biol Chem. 275(28), 21444-21452 (2000).
13. X. Si, Q. Zeng, C. H. Ng, W. Hong and C. J. Pallen, Interaction of Farnesylated Prl-2, a Protein-Tyrosine Phosphatase, with the Beta-Subunit of Geranylgeranyltransferase Ii. J Biol Chem. 276(35), 32875-32882 (2001).
14. S. Saha, A. Bardelli, P. Buckhaults, V. E. Velculescu, C. Rago, B. St Croix, K. E. Romans, M. A. Choti, C. Lengauer, K. W. Kinzler and B. Vogelstein, A Phosphatase Associated with Metastasis of Colorectal Cancer. Science. 294, 1343-1346 (2001).
15. Q. Zeng, J.-M. Dong, K. Guo, J. Li, H.-X. Tan, V. Koh, C. J. Pallen, E. Manser and W. Hong, Prl-3 and Prl-1 Promote Cell Migration, Invasion, and Metastasis. Cancer Res. 63(11), 2716-2722 (2003).
16. K. Guo, J. P. Tang, C. P. B. Tan, H. Wang and Q. Zeng, Monoclonal Antibodies Target Intracellular Prl Phosphatases to Inhibit Cancer Metastases in Mice. Cancer Biol Ther. 7(5), 750-757 (2008).
17. J. H. Brendon, C. M. Adrianus and P. C. Angeline, Passive Immunoprophylaxis and Therapy with Humanized Monoclonal Antibody Specific for Influenza a H5 Hemagglutinin in Mice. Respir Res. 7, 126-136 (2006).
18. J. Li, K. Guo, V. W. Koh, J. P. Tang, B. Q. Gan, H. Shi, H. X. Li and Q. Zeng, Generation of Prl-3- and Prl-1-Specific Monoclonal Antibodies as Potential Diagnostic Markers for Cancer Metastases. Clin Cancer Res 11(6), 2195-2204 (2005).
19. H. Wang, L. A. Vardy, C. P. Tan, J. M. Loo, K. Guo, J. Li, S. G. Lim, J. Zhou, W. J. Chng, S. B. Ng, H. X. Li and Q. Zeng, Pcbpl Suppresses the Translation of Metastasis-Associated Prl-3 Phosphatase. Cancer cell. 18(1), 52-62 (2010).
20. B. Weigelt, L. P. Johannes and L. J. v. t. Veer, Breast Cancer Metastasis Markers and Models. Nat Rev Cancer. 5(8), 591-602 (2005).
21. F. Polato, A. Codegoni, R. Fruscio, P. Perego, C. Mangioni, S. Saha, A. Bardelli and M. Broggini, Prl-3 Phosphatase Is Implicated in Ovarian Cancer Growth. Clin Cancer Res. 11(19 Pt 1), 6835-6839 (2005).
22. H. Yoshino, T. Ueda, M. Kawahata, K. Kobayashi, Y. Ebihara, A. Manabe, R. Tanaka, M. Ito, S. Asano, T. Nakahata and K. Tsuji, Natural Killer Cell Depletion by Anti-Asialo Gm1 Antiserum Treatment Enhances Human Hematopoietic Stem Cell Engraftment in Nod/Shi-Scid Mice. Bone Marrow Transplant. 26(11), 1211-1216 (2000).
23. M. Kasai, T. Yoneda, S. Habu, Y. Maruyama, K. Okumura and T. Tokunaga, In Vivo Effect of Anti-Asialo Gm1 Antibody on Natural Killer Activity. Nature. 291(5813), 334-335 (1981).
24. S. Park, Z. Jiang, E. D. Mortenson, L. Deng, O. Radkevich-Brown, X. Yang, H. Sattar, Y. Wang, N. K. Brown, M. Greene, Y. Liu, J. Tang, S. Wang and Y. X. Fu, The Therapeutic Effect of Anti-Her2/Neu Antibody Depends on Both Innate and Adaptive Immunity. Cancer Cell. 18(2), 160-170 (2010).
25. S. Cooper, Reappraisal of Serum Starvation, the Restriction Point, G0 and G1 Phase Arrest Points. FASEB J. 17(3), 333-340 (2003).
26. Y. Hüsemann, J. B. Geigl, F. Schubert, P. Musiani, M. Meyer, E. Burghart, G. Forni, R. Eils, T. Fehm, G. Riethmüller and C. A. Klein, Systemic Spread Is an Early Step in Breast Cancer. Cancer cell. 13(1), 58-68 (2008).

### Examples Section E3 (Example 39). Immunization of C57BL6 Mice with VHZ and Monitoring Tumor Development

Section E3 comprises Example 39.

### Example 39: Section E3: Immunization of C57BL6 Mice with VHZ and Monitoring Tumor Development

8-week old C57BL6 mice were immunized by intraperitoneal injection with a total volume of 200 ml Freund's Adjuvant: 20 mg of VHZ antigen in 100 ml saline mixed with 100 ml of complete adjuvant (Cat#77140, Pierce).

The next two immunizations were injected with a total volume of 200 ml adjuvant: 20 mg of VHZ antigen in 100 ml saline mixed with 100 ml of incomplete adjuvant (Cat#77145, Pierce).

The second and third injections were administrated every 2 weeks, 100-200 ml of tail bleed was collected in a heparin-coated capillary tube.

Plasma was prepared from blood sample and antibody titer was measured by ELISA.

The detailed steps of ELISA were described previously.

Mice with high titers of VHZ antibodies in their sera were selected and lateral tail vein injected with 1x106 VHZ-expressing cancer cells.

The results are shown in Figure 15. Figure 15 shows that mice vaccinated with VHZ antigen can prevent VHZ-expressing tumors. Unvaccinated mice serve as a negative control in this study.

## Claims

1. PRL3 for use in a method of prevention of metastatic cancer in a human individual, the method comprising administering an effective amount of PRL3 protein to the individual to stimulate the individual to produce its own antibodies to prevent tumor formation.

2. PRL3 for use according claim 1, in which the cancer is associated with or caused by overexpression PRL3.

3. PRL3 for use according to any one of the preceding claims wherein the cancer is selected from the group consisting of: acute lymphocytic leukemia (ALL), acute myeloid leukemia (AML), adrenocortical cancer, anal cancer, bladder cancer, blood cancer, bone cancer, brain tumor, breast cancer, cancer of the female genital system, cancer of the male genital system, central nervous system lymphoma, cervical cancer, childhood rhabdomyosarcoma, childhood sarcoma, chronic lymphocytic leukemia (CLL), chronic myeloid leukemia (CML), colon and rectal cancer, colon cancer, endometrial cancer, endometrial sarcoma, esophageal cancer, eye cancer, gallbladder cancer, gastric cancer, gastrointestinal tract cancer, hairy cell leukemia, head and neck cancer, hepatocellular cancer, Hodgkin's disease, hypopharyngeal cancer, Kaposi's sarcoma, kidney cancer, laryngeal cancer, leukemia, leukemia, liver cancer, lung cancer, malignant fibrous histiocytoma, malignant thymoma, melanoma, mesothelioma, multiple myeloma, myeloma, nasal cavity and paranasal sinus cancer, nasopharyngeal cancer, nervous system cancer, neuroblastoma, non-Hodgkin's lymphoma, oral cavity cancer, oropharyngeal cancer, osteosarcoma, ovarian cancer, pancreatic cancer, parathyroid cancer, penile cancer, pharyngeal cancer, pituitary tumor, plasma cell neoplasm, primary CNS lymphoma, prostate cancer, rectal cancer, respiratory system, retinoblastoma, salivary gland cancer, skin cancer, small intestine cancer, soft tissue sarcoma, stomach cancer, stomach cancer, testicular cancer, thyroid cancer, urinary system cancer, uterine sarcoma, vaginal cancer, vascular system, Waldenstrom's macroglobulinemia and Wilms' tumor.

4. A vaccine composition comprising PRL3, together with a pharmaceutically acceptable excipient, diluent or carrier.

## Patentansprüche

1. PRL3 zur Verwendung in einem Verfahren zur Prävention von metastatischem Krebs in einem menschlichen Individuum, wobei das Verfahren das Verabreichen einer wirksamen Menge an PRL3-Protein an das Individuum umfasst, um das Individuum anzuregen, seine eigenen Antikörper zu produzieren, um die Tumorbildung zu verhindern.

2. PRL3 zur Verwendung nach Anspruch 1, wobei der Krebs mit einer Überex-pression von PRL3 einhergeht oder dadurch verursacht wurde.

3. PRL3 zur Verwendung nach einem der vorangegangenen Ansprüche, worin der Krebs aus der Gruppe bestehend aus: akuter lymphozytischer Leukämie (ALL), akuter myeloischer Leukämie (AML), Nebennierenrindenkrebs, Analkrebs, Blasenkrebs, Blutkrebs, Knochenkrebs, Hirnkrebs, Brustkrebs, Krebs des weiblichen Genitalsystems, Krebs des männlichen Genitalsystems, Lymphom des Zentralnervensystems, Gebärmutterhalskrebs, Rhabdomyosarkom im Kindesalter, Sarkom im Kindesalter, chronischer lymphozytischer Leukämie (CLL), chronischer myeloischer Leukämie (CML), Dickdarm- und Rektumkrebs, Dickdarmkrebs, Krebs des Endometriums, endometrialem Sarkom, Speiseröhrenkrebs, Augenkrebs, Gallenblasenkrebs, Magenkrebs, Krebs des Verdauungstrakts, Haarzellenleukämie, Kopf- und Nackenkrebs, Leberzellenkrebs, Morbus Hodgkin, Hypopharynxkrebs, Kaposi-Sarkom, Nierenkrebs, Kehlkopfkrebs, Leukämie, Leberkrebs, Lungenkrebs, bösartigem fibrösem Histiozytom, bösartigem Thymom, Melanom, Mesotheliom, multiplem Myelom, Myelom, Nasenhöhlen- und Paranasalsinus-Krebs, nasopharyngealem Krebs, Krebs des Nervensystems, Neuroblastom, Non-Hodgkin-Lymphom, Mundhöhlenkrebs, oropharyngealem Krebs, Osteosarkom, Eierstockkrebs, Pankreaskrebs, Nebenschilddrüsenkrebs, Peniskrebs, Rachenkrebs, Hypophysenkrebs, Plasmazellenneoplasma, primärem ZNS-Lymphom, Prostatakrebs, Rektalkrebs, Krebs der Atemwege, Retinoblastom, Speicheldrüsenkrebs, Hautkrebs, Dünndarmkrebs, Weichteilsarkom, Magenkrebs, Hodenkrebs, Schilddrüsenkrebs, Krebs der Harnwege, Gebärmuttersarkom, Scheidenkrebs, Krebs des Gefäßsystems, Waldenström-Makroglobulinämie und Wilms-Tumor ausgewählt ist.

4. Vakzinenzusammensetzung, die PRL3 sowie einen pharmazeutisch annehmbaren Exzipienten, Verdünner oder Träger umfasst.

## Revendications

1. PRL3 pour utilisation dans un procédé de prévention d'un cancer métastatique chez un individu humain, le procédé comprenant l'administration d'une quantité efficace de protéine PRL3 à l'individu pour stimuler l'individu afin qu'il produise ses propres anticorps pour prévenir la formation de tumeurs.

2. PRL3 pour utilisation selon la revendication 1, dans laquelle le cancer est associé à une surexpression de la PRL3 ou qui est causé par celle-ci.

3. PRL3 pour utilisation selon l'une quelconque des revendications précédentes dans laquelle le cancer est choisi dans le groupe constitué de : la leucémie lymphoïde aiguë (ALL), la leucémie myéloïde aiguë (AML), le cancer corticosurrénal, le cancer anal, le cancer de la vessie, le cancer du sang, le cancer des os, la tumeur cérébrale, le cancer du sein, le cancer de l'appareil génital féminin, le cancer de l'appareil génital masculin, le lymphome du système nerveux central, le cancer du col de l'utérus, le rhabdomyosarcome de l'enfant, le sarcome de l'enfant, la leucémie lymphoïde chronique (CLL), la leucémie myéloïde chronique (CML), le cancer du côlon et rectal, le cancer du côlon, le cancer de l'endomètre, le sarcome de l'endomètre, le cancer de l'oesophage, le cancer de l'oeil, le cancer de la vésicule biliaire, le cancer gastrique, le cancer du tractus gastro-intestinal, la leucémie à tricholeucocytes, le cancer de la tête et du cou, le cancer hépatocellulaire, la maladie de Hodgkin, le cancer de l'hypopharynx, le sarcome de Kaposi, le cancer du rein, le cancer du larynx, la leucémie, la leucémie, le cancer du foie, le cancer du poumon, l'histiocytome fibreux malin, le thymome malin, le mélanome, le mésothéliome, le myélome multiple, le myélome, le cancer de la cavité nasale et des sinus paranasaux, le cancer du rhinopharynx, le cancer du système nerveux, le neuroblastome, le lymphome non hodgkinien, le cancer de la cavité buccale, le cancer de l'oropharynx, l'ostéosarcome, le cancer ovarien, le cancer du pancréas, le cancer des glandes parathyroïdes, le cancer du pénis, le cancer du pharynx, la tumeur de l'hypophyse, le néoplasme des plasmocytes, le lymphome du CNS primaire, le cancer de la prostate, le cancer rectal, le cancer du système respiratoire, le rétinoblastome, le cancer des glandes salivaires, le cancer de la peau, le cancer de l'intestin grêle, le sarcome des tissus mous, le cancer de l'estomac, le cancer de l'estomac, le cancer des testicules, le cancer de la thyroïde, le cancer du système urinaire, le sarcome utérin, le cancer du vagin, le cancer du système vasculaire, la macroglobulinémie de Waldenstrom et la tumeur de Wilms.

4. Composition vaccinale comprenant la PRL3, conjointement avec un excipient, diluant ou vecteur pharmaceutiquement acceptable.
